# EUROPEAN PATENT APPLICATION

(11) **EP 4 818 324 A1**
(43) Date of publication of application: **07.10.2026**
(21) Application number: 24895849.8
(22) Date of filing: 03.09.2024
(51) Int. Cl.: C12P 7/18

(54) **BIOLOGICAL METHOD FOR PREPARING 1,2-PROPANEDIOL**

(30) Priority: 29.11.2023 CN 202311615909
(71) Applicant: Tianjin University, Tianjin 300072 (CN)
(72) Inventor: ZHANG, Yan, Tianjin 300072 (CN); MA, Kailiang, Tianjin 300072 (CN)
(74) Representative: Huang, Liwei
(86) International application number: PCT/CN2024/116570
(87) International publication number: WO 2025/112750

(57) **Abstract**

Provided is a method for producing 1,2-propanediol, including: a) reacting a mixture I including an α-1,4-glucan lyase, a 1,5-anhydro-D-fructose reductase, and reduced nicotinamide adenine dinucleotide (NADH) or reduced nicotinamide adenine dinucleotide phosphate (NADPH) with a substrate containing α-1,4-glycosidic bonds; or reacting a culture broth of cells capable of expressing and secreting an α-1,4-glucan lyase and/or a 1,5-anhydro-D-fructose reductase with NADH or NADPH and a substrate containing α-1,4-glycosidic bonds; and b) reacting a reaction product obtained in step a) with a mixture II including a sugar kinase, a 1,5-anhydroalditol-6-phosphate isomerase, a radical enzyme activator, an aldolase, and a hydroxyacetone reductase, or co-culturing a reaction product obtained in step a) with cells capable of expressing a 1,5-anhydroalditol-6-phosphate isomerase, a radical enzyme activator, an aldolase, a hydroxyacetone reductase, and a transport complex, to obtain 1,2-propanediol.

## Description

### Cross-Reference to Related Application

This application claims priority and related rights of Chinese Patent Application No. 202311615909.3 filed on November 29, 2023, and the contents of which are incorporated herein in its entirety by reference.

### Technical Field

The present disclosure relates generally to the fields of genetic engineering, enzyme engineering, and bioinformatics. Specifically, the present disclosure relates to a biological method for producing 1,2-propanediol.

### Background Art

1,2-Propanediol is mainly used in the production of coatings and unsaturated polyester resins, and also in the production of plasticizers and hydraulic brake fluids. In addition, 1,2-propanediol can be used as a good solvent in the preparation of inks and epoxy resins; be used in a non-ionic detergent serving as an enzyme stabilizer and solvent; be used as an antifreeze agent; and be used as a humectant in the pharmaceutical, cosmetic, animal feed, and tobacco industries.

Many bacteria possess a natural 1,2-propanediol synthesis pathway that utilizes deoxysugars as raw materials. One advantage of microbial production is that the resulting 1,2-propanediol is of a single enantiomeric form, which can serve as a precursor for chiral pharmaceutical compounds.

The reported strategies for microbial production of 1,2-propanediol include the 6-deoxyhexose pathway (producing S-form 1,2-propanediol), the methylglyoxal pathway (producing R-form 1,2-propanediol), and the lactate pathway (producing racemic 1,2-propanediol). The most significant limitation of the 6-deoxyhexose pathway is its requirement for the expensive sugars such as fucose and rhamnose as raw materials. All three pathways involve the toxic intermediates methylglyoxal and/or lactaldehyde.

Given that 1,2-propanediol is an important industrial raw material with significant value and uses, research on its synthetic pathways holds considerable scientific and industrial disclosure value.

### Summary of the Invention

In a first aspect, the present disclosure provides a novel method for producing 1,2-propanediol, including:
a) reacting a mixture I including an α-1,4-glucan lyase, a 1,5-anhydro-D-fructose reductase, and reduced nicotinamide adenine dinucleotide (NADH) or reduced nicotinamide adenine dinucleotide phosphate (NADPH) with a substrate containing α-1,4-glycosidic bonds, such as starch or maltose; or reacting a culture broth of cells capable of expressing (e.g., heterologously expressing) and secreting an α-1,4-glucan lyase and/or a 1,5-anhydro-D-fructose reductase with NADH or NADPH and a substrate containing α-1,4-glycosidic bonds;
b) reacting a reaction product obtained in step a) with a mixture II including a sugar kinase, a 1,5-anhydroalditol-6-phosphate isomerase, a radical enzyme activator, an aldolase, and a hydroxyacetone reductase, or co-culturing a reaction product obtained in step a) with cells capable of expressing a 1,5-anhydroalditol-6-phosphate isomerase, a radical enzyme activator, an aldolase, a hydroxyacetone reductase, and a transport complex, to obtain 1,2-propanediol.

In a second aspect, the present disclosure provides a novel method for producing 1,2-propanediol, including: reacting 1,5-anhydroalditol with a mixture II including a sugar kinase, a 1,5-anhydroalditol-6-phosphate isomerase, a radical enzyme activator, an aldolase, and a hydroxyacetone reductase, or co-culturing 1,5-anhydroalditol with cells capable of expressing a 1,5-anhydroalditol-6-phosphate isomerase, a radical enzyme activator, an aldolase, a hydroxyacetone reductase, and a transport complex, to obtain 1,2-propanediol. In a preferred embodiment, the 1,5-anhydroalditol is 1,5-anhydroglucitol or 1,5-anhydromannitol.

In some embodiments of the first aspect, the mixture I further includes other enzymes that regenerate NAD(P)⁺ to NAD(P)H, such as formate dehydrogenase (FDH). In specific embodiments, formate dehydrogenase and formate reduce generated NAD(P)⁺, achieving regeneration of NAD(P)H, thereby increasing a yield of products such as 1,5-anhydroalditol.

In some embodiments of the first aspect, the cells in step a) are also capable of expressing other enzymes that regenerate NAD(P)⁺ to NAD(P)H, such as FDH. The cells are capable of secreting the proteins they express (e.g., heterologously express) into an extracellular space to participate in the reaction.

In some embodiments of the first aspect, cells such as E. coli or yeast are used to heterologously express an α-1,4-glucan lyase, a 1,5-anhydro-D-fructose reductase, and/or other enzymes that regenerate NAD(P)⁺ to NAD(P)H, such as formate dehydrogenase, and secrete these enzymes into an extracellular space, thereby achieving results of step a).

In specific embodiments of the first aspect, the transport complex is a bacterial phosphoenolpyruvate-phosphotransferase system (PTS).

In some embodiments of the first aspect, the 1,5-anhydro-D-fructose reductase is a 1,5-anhydro-D-fructose reductase from *Sus scrofa* (Gafr) or a 1,5-anhydro-D-fructose reductase from *Ensifer adhaerens* (Mafr).

In some embodiments of the first aspect, the α-1,4-glucan lyase is derived from *Gracilariopsis lemaneiformis*, and/or the FDH is derived from *Pseudomonas sp..*

In some embodiments of the first aspect, the reaction product obtained in step a) is 1,5-anhydroalditol. In specific embodiments, the 1,5-anhydroalditol is 1,5-anhydroglucitol or 1,5-anhydromannitol.

In some embodiments of the first aspect, the substrate containing α-1,4-glycosidic bonds is selected from starch, maltose, or glycogen. In some embodiments, the starch is soluble starch. In some specific embodiments, the reaction in step a) is carried out in a solution containing starch, for example, a culture medium.

In some embodiments of the first aspect, the sugar kinase or the transport complex phosphorylates the reaction product obtained in step a).

In some embodiments of the second aspect, the sugar kinase or the transport complex phosphorylates 1,5-anhydroalditol to 1,5-anhydroalditol-6-phosphate, e.g., 1,5-anhydroglucitol-6-phosphate or 1,5-anhydromannitol-6-phosphate.

In some specific embodiments, the sugar kinase is from Uniprot: Q7M537, belonging to EC:2.7.1.147.

In some embodiments of the first or second aspect, the cell is a eukaryotic cell or a prokaryotic cell. In some embodiments, the eukaryotic cell is a yeast cell. In other embodiments, the prokaryotic cell is selected from *Escherichia*, *Klebsiella*, *Streptococcus*, *Lactobacillus*, *Bifidobacterium*, *Bacteroidetes*, and *Firmicutes*, etc. In preferred embodiments, the cell is an *E. coli* cell. In specific embodiments, the cell is an E. coli cell that is induced to highly express YbiW and/or PflD gene clusters by 1,5-AG and/or 1,5-AM, or is genetically modified to highly express YbiW and/or PflD gene clusters under a strong promoter. In some embodiments, the cell is an E. coli MG1655 cell. In some embodiments of the first or second aspect, the 1,5-anhydroalditol-6-phosphate isomerase includes an amino acid sequence shown in SEQ ID NO:1 or a functional variant thereof, where the functional variant possesses 1,5-anhydroglucitol-6-phosphate isomerase activity. In some embodiments, the 1,5-anhydroalditol-6-phosphate isomerase has an active site in its three-dimensional conformation defined such that, with reference to SEQ ID NO: 1, the active site includes amino acid residues H165, H282, S283, H334, C441, E443, R453, T455, L562, S662, I664, and G786, which are spatially close to one another.

In some embodiments of the first or second aspect, the 1,5-anhydroalditol-6-phosphate isomerase includes an amino acid sequence shown in SEQ ID NO:97 or a functional variant thereof, wherein the functional variant possesses 1,5-anhydromannitol-6-phosphate isomerase activity. In some embodiments, the 1,5-anhydroalditol-6-phosphate isomerase has an active site in its three-dimensional conformation defined such that, with reference to SEQ ID NO: 97, the active site includes amino acid residues Q162, H169, S277, S278, R323, F331, P335, C431, E433, D445, Y628, V630, and G752, which are spatially close to one another.

In some embodiments of the first or second aspect, the functional variant is a natural isoenzyme of the amino acid sequence shown in SEQ ID NO:97.

In some embodiments of the first or second aspect, an substrate of the 1,5-anhydroalditol-6-phosphate isomerase is 1,5-anhydroalditol-6-phosphate.

In some embodiments of the first or second aspect, the functional variant is produced by insertion, substitution, and/or deletion of one or more amino acids in the amino acid sequence shown in SEQ ID NO:1 or 97, or a natural isoenzyme thereof.

In some embodiments of the first or second aspect, the transport complex includes at least one of SEQ ID NOs: 150-153 or functional variants thereof, and the transport complex possesses phosphoenolpyruvate-dependent phosphotransferase system transport activity.

In some embodiments of the first or second aspect, the radical enzyme activator is an S-adenosylmethionine radical enzyme family glycine radical enzyme activating enzyme.

In some embodiments of the first or second aspect, the aldolase is 1-deoxyfructose-6-phosphate aldolase.

In a third aspect, the present disclosure provides a composition including an α-1,4-glucan lyase, a 1,5-anhydro-D-fructose reductase, and reduced nicotinamide adenine dinucleotide (NADH) or reduced nicotinamide adenine dinucleotide phosphate (NADPH), and use thereof in catalyzing the formation of 1,5-anhydroalditol from a substrate containing α-1,4-glycosidic bonds. In some embodiments, the composition further includes other enzymes that regenerate NAD(P)⁺ to NAD(P)H, such as formate dehydrogenase (FDH). In some embodiments, the substrate containing α-1,4-glycosidic bonds is selected from starch, maltose, or glycogen, etc.

In a fourth aspect, the present disclosure provides a composition including a sugar kinase, a 1,5-anhydroalditol-6-phosphate isomerase, a radical enzyme activator, an aldolase, and a hydroxyacetone reductase, and use thereof in catalyzing the formation of 1,2-propanediol from 1,5-anhydroalditol.

In some embodiments of the third or fourth aspect, the 1,5-anhydroalditol is 1,5-anhydroglucitol or 1,5-anhydromannitol. In some embodiments, the 1,5-anhydroglucitol or 1,5-anhydromannitol is phosphorylated by a sugar kinase to 1,5-anhydroglucitol-6-phosphate or 1,5-anhydromannitol-6-phosphate, respectively.

### Brief Description of the Drawings

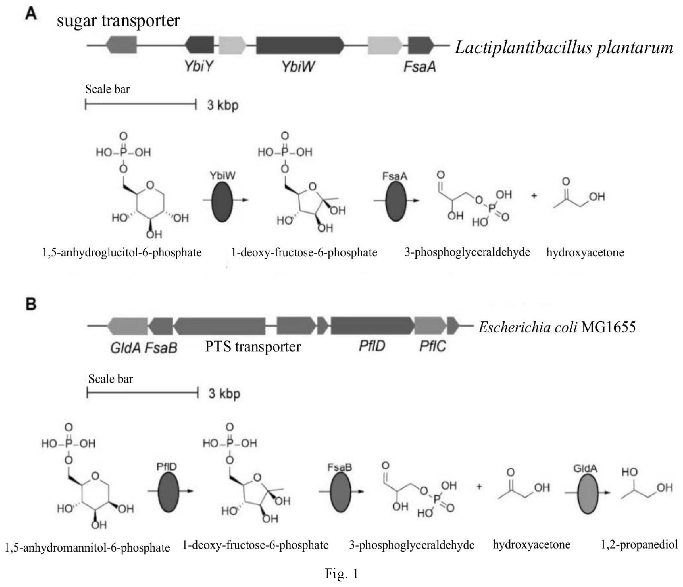

Fig. 1 is a schematic diagram of radical-dependent anhydroglycolysis. A. Gene cluster in *Lactiplantibacillus plantarum* (including the gene encoding the 1,5-anhydroglucitol-6-phosphate isomerase shown in SEQ ID NO: 2) and a YbiW-dependent anhydroglycolysis pathway. B. Gene cluster in *Escherichia coli* (including the gene encoding the 1,5-anhydromannitol-6-phosphate isomerase shown in SEQ ID NO: 98) anda PflD-dependent anhydroglycolysis pathway.

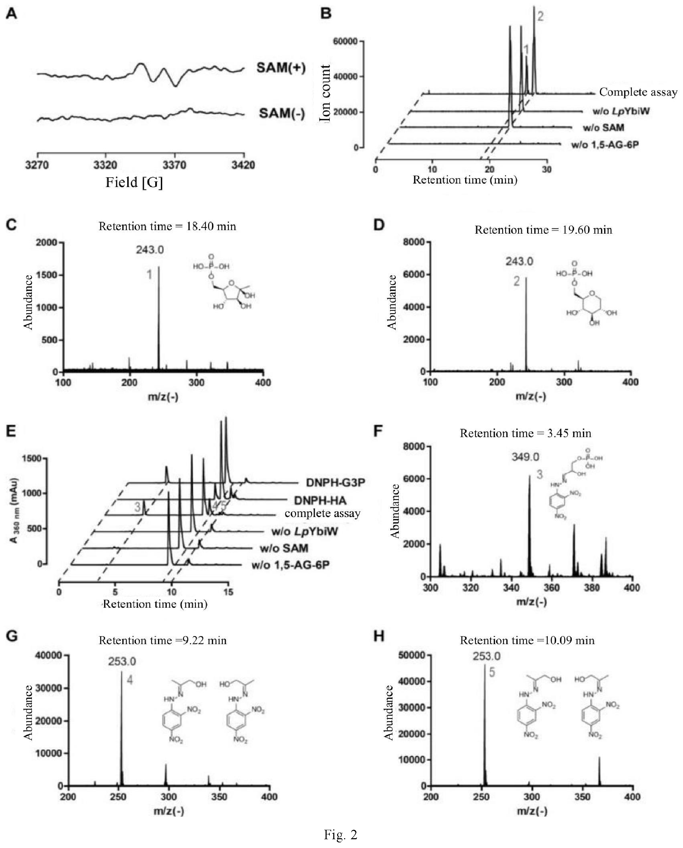

Fig. 2 shows electron paramagnetic resonance (EPR) spectra and LC-MS enzyme reaction analysis of YbiW. A. EPR spectra of LpYbiW and LpYbiY with titanium(III) citrate in the presence or absence of S-adenosylmethionine (SAM). B. LC-MS detection of 1-deoxyfructose-6-phosphate (1-deoxy-F6P) formation in the isomerization of 1,5-anhydroglucitol-6-phosphate (1,5-AG-6P) catalyzed by LpYbiW. Extracted ion chromatogram (EIC) monitoring for m/z=243 (in a negative ion mode) of 1-deoxy-F6P formation (t_{R}=18.40 min). C-D. Mass spectra (in a negative ion mode) of compound 1 (1-deoxy-F6P) and compound 2 (1,5-AG-6P) corresponding to the EIC peaks in B. E. HPLC elution profiles of DNPH derivatives of reaction products and standards in the LpFsaA-coupled LpYbiW reaction analysis. F-H. ESI(-) m/z mass spectra of DNPH-3-phosphoglyceraldehyde (peak 3) and DNPH-hydroxyacetone (peaks 4 and 5) in E. "w/o" indicates "not containing".

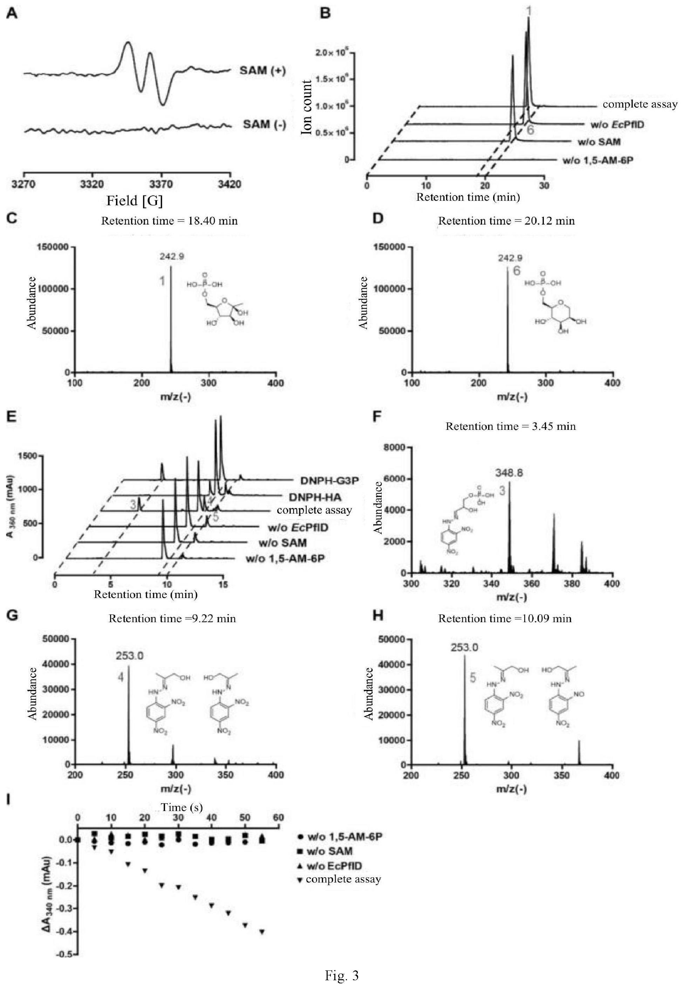

Fig. 3 shows EPR spectra and LC-MS enzyme reaction analysis of PflD. A. EPR spectra of EcPflD and EcPflC with titanium(III) citrate in the presence or absence of SAM. B. LC-MS detection of 1-deoxy-F6P formation in the isomerization of 1,5-anhydromannitol-6-phosphate (1,5-AM-6P) catalyzed by EcPflD. EIC monitoring for m/z=243 (in a negative ion mode) of 1-deoxy-F6P formation (t_{R}=18.40 min). C-D. Mass spectra (in a negative ion mode) of compound 1 (1-deoxy-F6P) and compound 6 (1,5-AM-6P) corresponding to the EIC peaks in B. E. HPLC elution profiles of DNPH derivatives of reaction products and standards in the EcFsaB-coupled EcPflD reaction analysis. F-H. Mass spectra of DNPH-3-phosphoglyceraldehyde (peak 3) and DNPH-hydroxyacetone (peaks 4 and 5) in E. I. Spectrophotometric determination of EcGldA-EcFsaB coupled with EcPflD, monitoring NADH consumption as hydroxyacetone is reduced. "w/o" indicates "not containing".

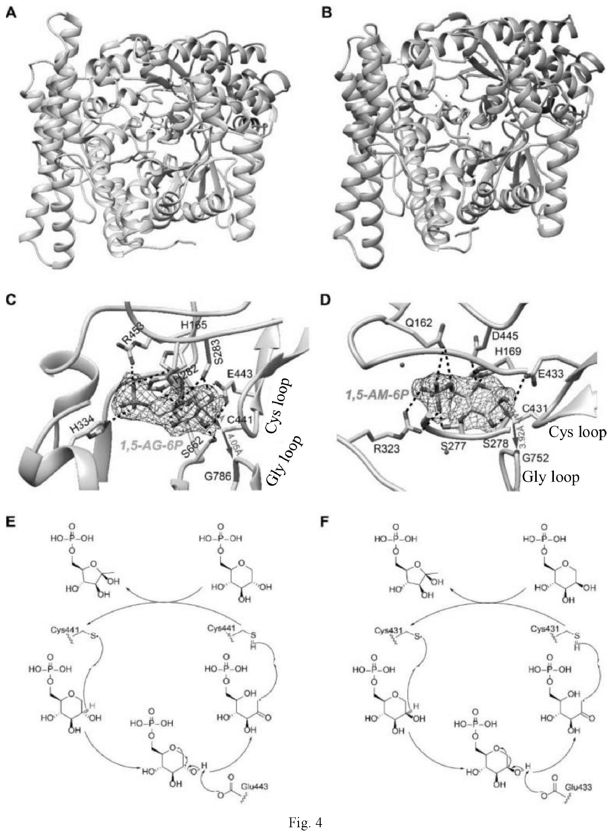

Fig. 4 shows X-ray diffraction crystal structures of EcYbiW and SdPflD. A-B. Structures of monomers of EcYbiW and SdPflD in each asymmetric unit. C-D. Active site structures of complexes of EcYbiW with 1,5-AG-6P and SdPflD with 1,5-AM-6P, respectively. Hydrogen atom transfer pathways are indicated by arrows, hydrogen bonds are represented by black dashed lines, and distances between key atoms are shown. The 2Fo-Fc electron density of the substrate is at 1.0σ. E-F. Catalytic mechanisms of EcYbiW and SdPflD.

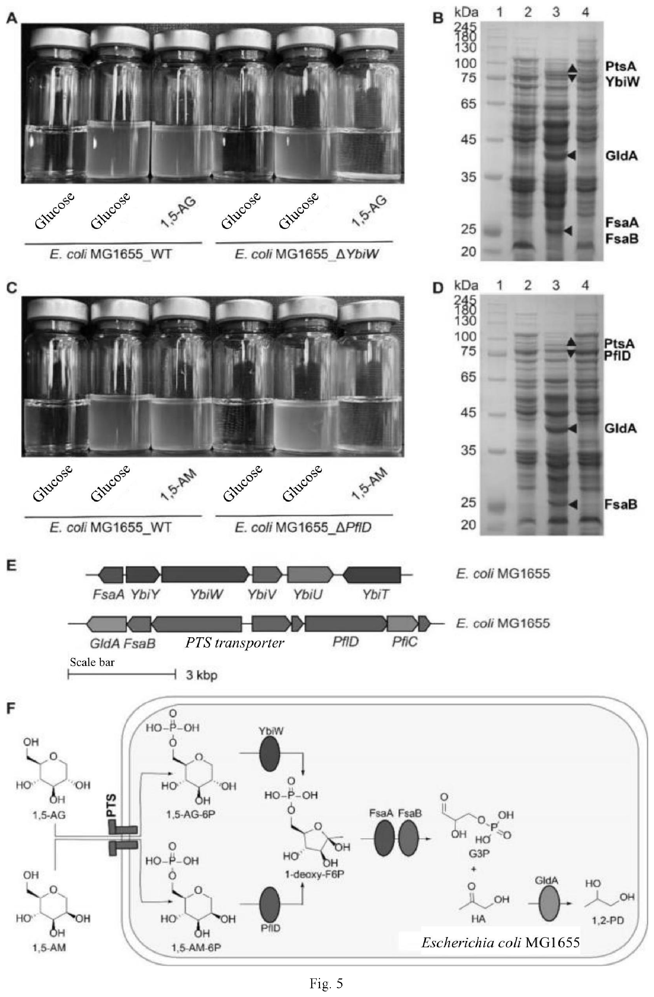

Fig. 5 shows YbiW-dependent glycolytic pathway induced by 1,5-anhydroglucitol (1,5-AG) and PflD-dependent glycolytic pathway induced by 1,5-anhydromannitol (1,5-AM) in *E. coli*. A. Growth of *E. coli* MG1655 wild-type and ΔybiW strains using 1,5-AG as the sole carbon source. Positive control: glucose is used as the sole carbon source. Negative control (w/o glucose): no carbon source is added. B. SDS-PAGE analysis of *E. coli* MG1655 wild-type using glucose (lane 2) or 1,5-AG (lane 3) as a sole carbon source, and of ΔybiW using glucose (lane 4) as a sole carbon source. Arrows indicate two about 95 kDa bands containing PtsA and YbiW, a about 42 kDa band identified as GldA, and a about 27 kDa band identified containing FsaB and FsaA. C. Growth of *E. coli* MG1655 wild-type and ΔpflD strains using 1,5-AM as a sole carbon source. Positive control: glucose is used as a sole carbon source. Negative control: no carbon source is added. D. SDS-PAGE analysis of *E. coli* MG1655 wild-type using glucose (lane 2) or 1,5-AM (lane 3) as a sole carbon source, and ΔpflD using glucose (lane 4) as a sole carbon source. Arrows indicate a about 95 kDa band identified as PtsA, a about 90 kDa band identified as PflD, a about 42 kDa band identified as GldA, and a about 27 kDa band identified as FsaB. E. Adjacent genome of YbiW (SEQ ID NO: 1) and PflD (SEQ ID NO: 98) in *E. coli* MG1655. F. 1,5-AG anhydroglycolysis pathway and 1,5-AM anhydroglycolysis pathway in *E. coli*. "w/o" indicates "not containing".

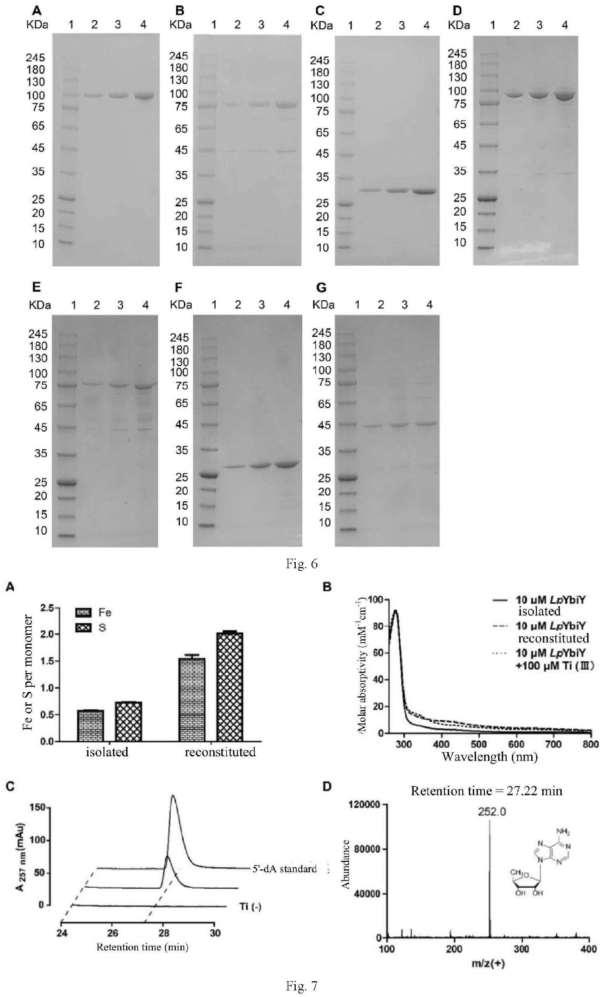

Fig. 6 shows SDS-PAGE analysis of purified proteins used for enzyme assays and biochemical characterization. A. LpYbiW; B. MBP-LpYbiY; C. LpFsaA; D. EcPflD; E. MBP-EcPflC; F. EcFsaB; G. EcGldA. Lanes 1 to 4 in each 4-20% gradient gel (Bis-Tris) contain protein molecular weight marker and 1 µg, 2 µg, and 4 µg of recombinant protein, respectively.
Fig. 7 shows characterization of recombinant LpYbiY. A. [Fe-S] cluster quantification in LpYbiY, assays performed in triplicate and shown with standard deviation. B. UV-Vis absorption spectra of LpYbiY is isolated and reconstituted, corresponding that the characteristic absorbance at 410 nm for the [4Fe-4S]²⁺ cluster in reconstituted LpYbiY disappears upon reduction with the strong reducing agent titanium(III) citrate. C. LC-MS elution profiles of SAM cleavage reaction mixtures catalyzed by LpYbiY in the presence and absence of Ti(III); commercially available 5'-deoxyadenosine (5'-dA) used as a standard. D. Positive ionization mass spectrum of the 5'-dA peak eluted at 27.22 minutes.

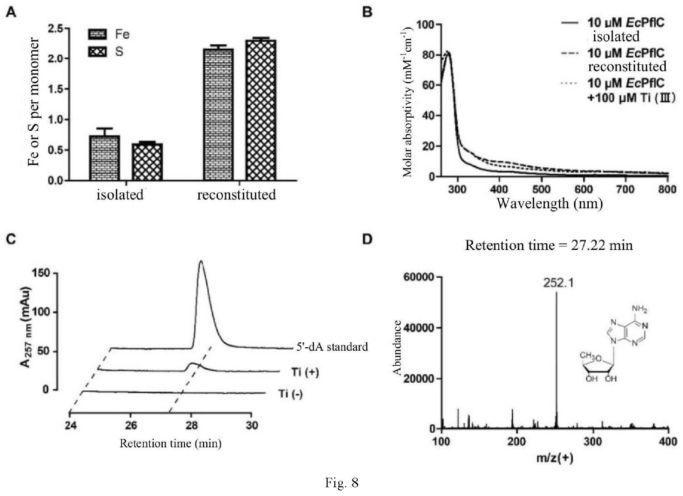

Fig. 8 shows characterization of recombinant EcPflC. A. [Fe-S] cluster quantification in EcPflC, assays performed in triplicate and shown with standard deviation. B. UV-Vis absorption spectra of isolated and reconstituted EcPflC, the characteristic absorbance at 410 nm corresponding to the [4Fe-4S]²⁺ cluster in reconstituted EcPflC disappears upon reduction with the strong reducing agent titanium(III) citrate. C. LC-MS elution profiles of SAM cleavage reaction mixtures catalyzed by EcPflC in the presence and absence of Ti(III); commercially available 5'-dA used as a standard. D. Positive ionization mass spectrum of the 5'-dA peak eluted at 27.22 minutes.

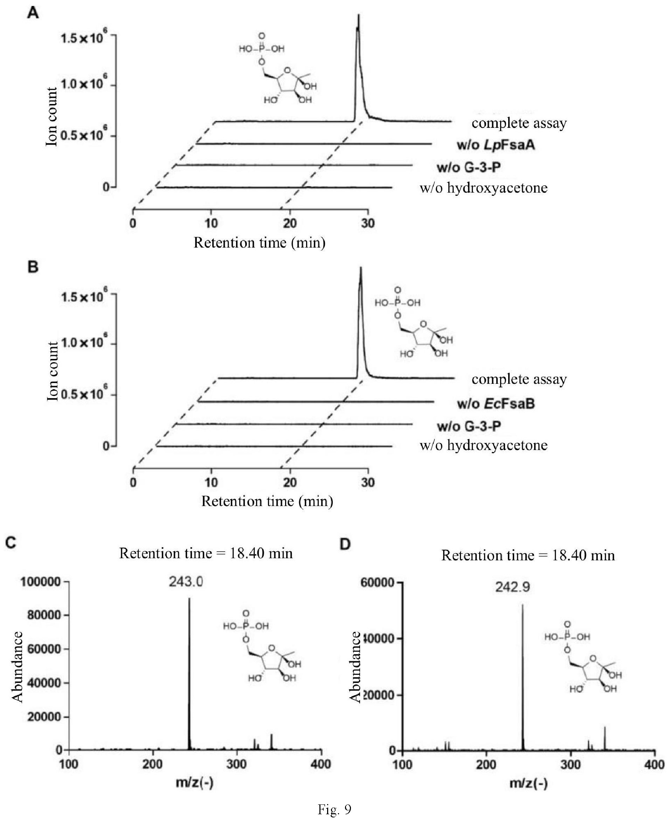

Fig. 9 shows LC-MS enzyme activity assays of LpFsaA and EcFsaB catalyzing the aldol addition of hydroxyacetone and 3-phosphoglyceraldehyde. A-B. Extracted ion chromatograms (m/z=243, in a negative ion mode) of LpFsaA- and EcFsaB-catalyzed reaction mixtures, monitoring the formation of 1-deoxy-F6P. C-D. Negative ionization mass spectra of 1-deoxy-F6P formed by LpFsaA and EcFsaB. "w/o" indicates "not containing".

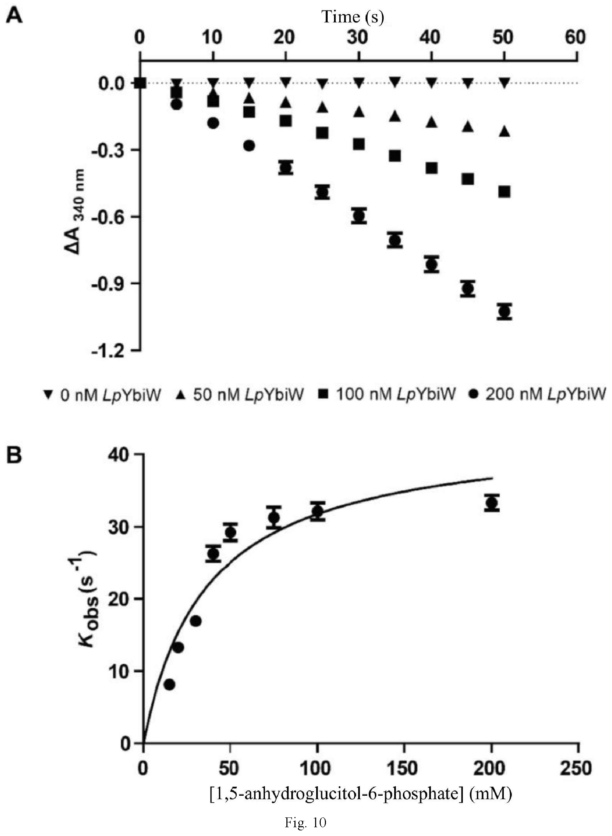

Fig. 10 shows enzyme kinetics data for LpYbiW. A. Dose-dependent LpFsaA-EcGldA coupled enzyme activity assay for LpYbiW; the amount of LpYbiW used in the assay is indicated. B. Michaelis-Menten kinetics of LpYbiW. Error bars represent the standard deviation of three independent experiments.

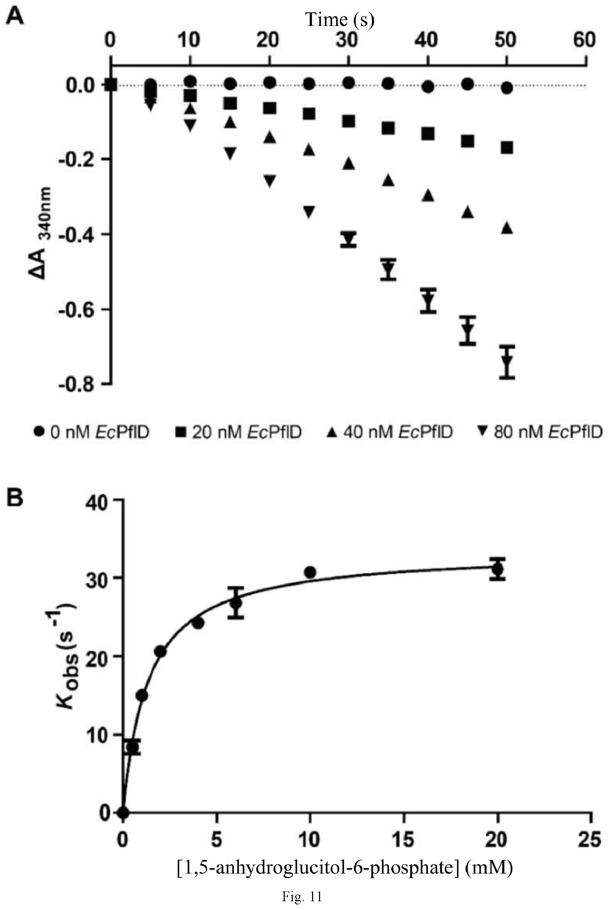

Fig. 11 shows enzyme kinetics data for EcPflD. A. Dose-dependent EcFsaB-EcGldA coupled enzyme activity assay for EcPflD; the amount of EcPflD used in the assay is indicated. B. Michaelis-Menten kinetics of EcPflD. Error bars represent the standard deviation of three independent experiments.

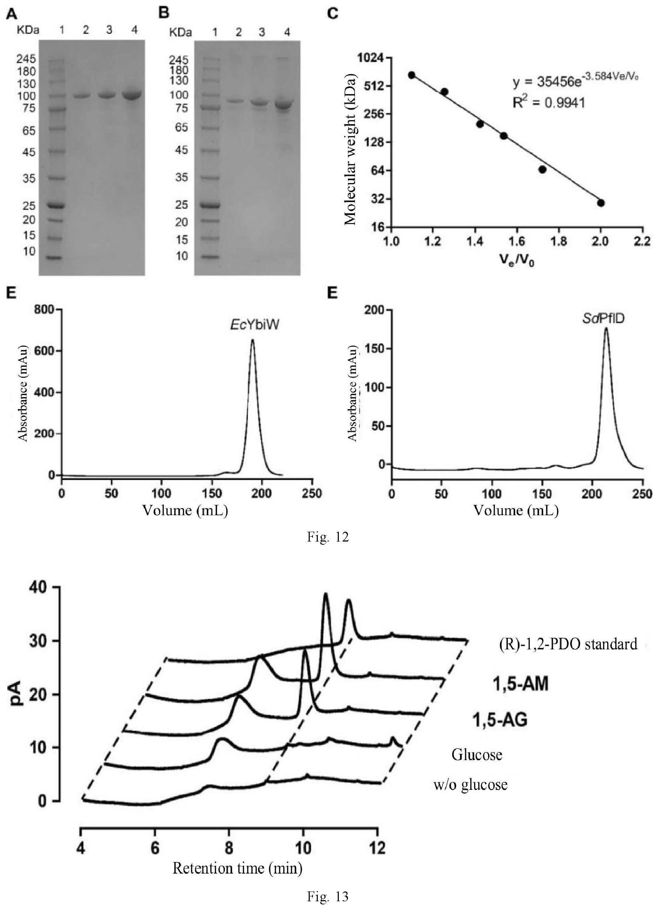

Fig. 12 shows SDS-PAGE and SEC analysis of purified EcYbiW (E114A, E115A, and K117A) and SdPflD used for protein crystallization. A. 4-20% gradient gel (Bis-Tris): lane 1, protein molecular weight marker; lanes 2-4, 1 µg, 2 µg, and 4 µg of purified EcYbiW (E114A, E115A, and K117A). B. 4-20% gradient gel (Bis-Tris): lane 1, protein molecular weight marker; lanes 2-4, 1 µg, 2 µg, and 4 µg of purified SdPflD. C. SEC standard curve established using standard proteins: bovine thyroglobulin (669 kDa), horse spleen ferritin (443 kDa), sweet potato β-amylase (200 kDa), yeast alcohol dehydrogenase (150 kDa), BSA (66 kDa), and bovine carbonic anhydrase (29 kDa) (Sigma MWGF 1000-1KT). D-E. Elution profiles of EcYbiW (E114A, E115A, and K117A) and SdPflD using Superdex 200 gel filtration chromatography to determine molecular weights of EcYbiW and SdPflD; estimated molecular weights are 154.9 kDa and 77.9 kDa, respectively.
Fig. 13 shows gas chromatography (GC) analysis of fermentation broth of wild-type *E. coli* MG1655 grown on glucose, 1,5-AG, or 1,5-AM. "w/o glucose" indicates not containing glucose (negative control); glucose is the positive control.

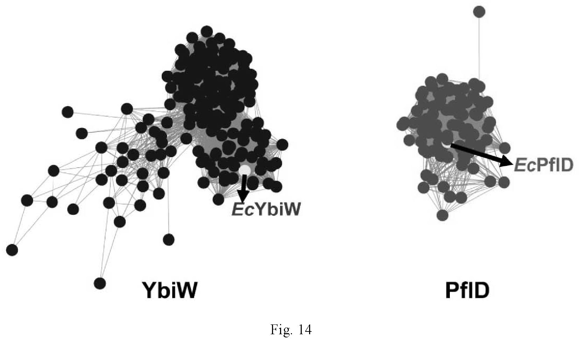

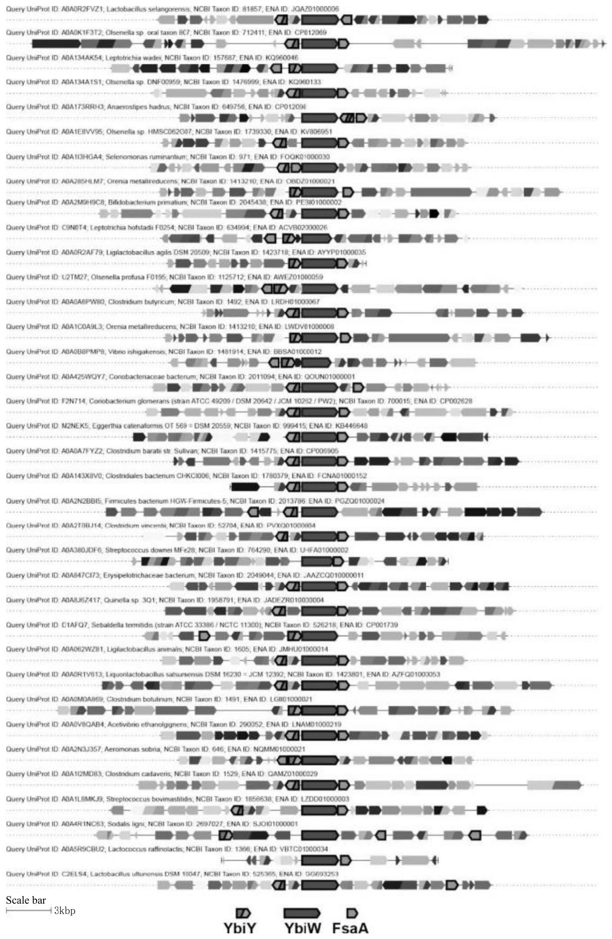

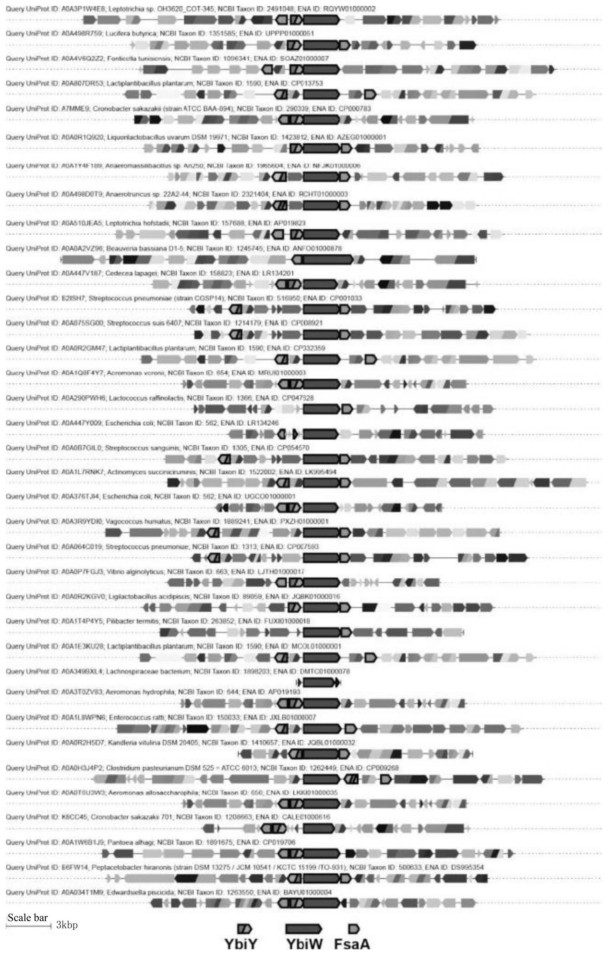

Fig. 14 shows the YbiW cluster and PflD cluster constructed using the Sequence Similarity Network (SSN) tool. Each node represents sequences with 80% or more similarity. The nodes containing EcYbiW and EcPflD are indicated with arrows in the two clusters.

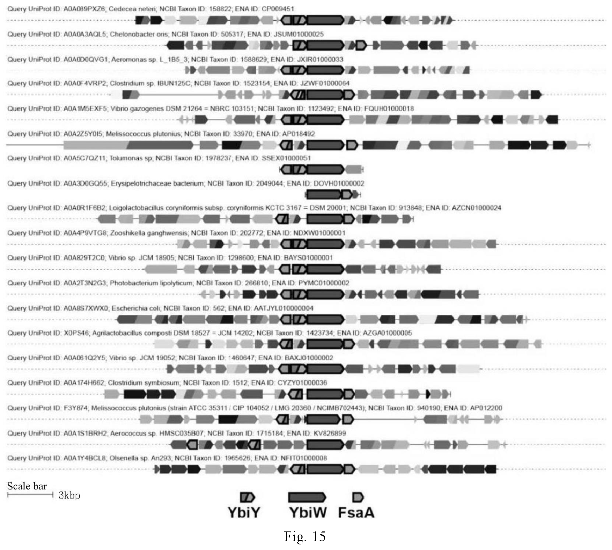

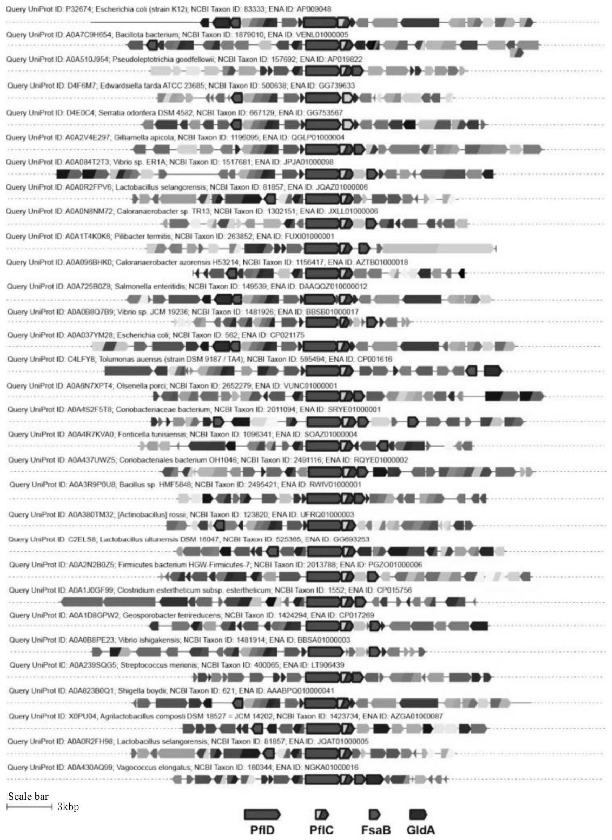

Fig. 15 shows exemplary gene cluster distributions including the natural isozyme of 1,5-anhydroglucitol-6-phosphate isomerase shown in SEQ ID NO: 1, an S-adenosylmethionine radical enzyme family glycine radical enzyme activating enzyme, and a 1-deoxyfructose-6-phosphate aldolase (gene directions indicate the coding strand direction, 5' to 3').

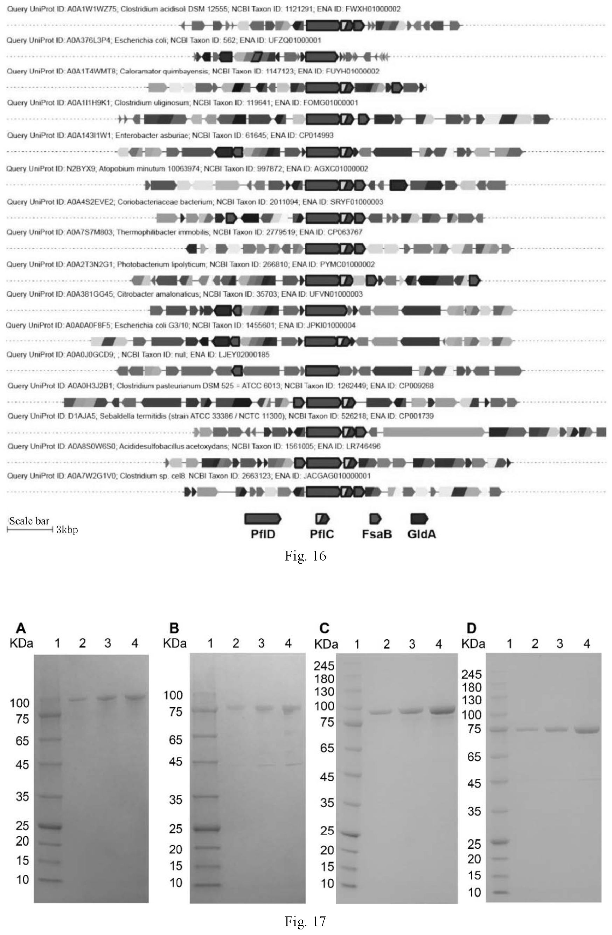

Fig. 16 shows exemplary gene cluster distributions including the natural isozyme of 1,5-anhydromannitol-6-phosphate isomerase shown in SEQ ID NO: 97, an S-adenosylmethionine radical enzyme family glycine radical enzyme activating enzyme, a 1-deoxyfructose-6-phosphate aldolase, and a hydroxyacetone reductase.
Fig. 17 shows SDS-PAGE analysis of purified proteins used for biochemical characterization. A. EcYbiW; B. MBP-EcYbiY; C. SdPflD; D. MBP-SdPflC. Lanes 1 to 4 in each 4-20% gradient gel (Bis-Tris) contain a protein molecular weight marker and 1 µg, 2 µg, and 4 µg of recombinant protein, respectively.

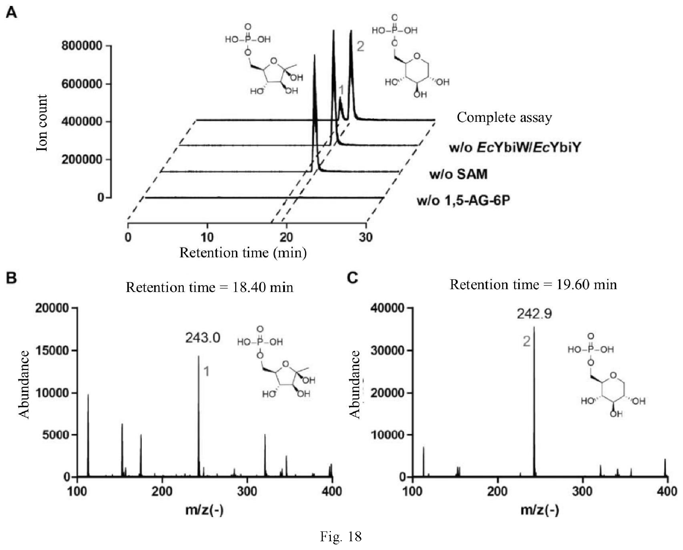

Fig. 18 shows LC-MS enzyme reaction analysis of *E. coli* YbiW. A. LC-MS detection of 1-deoxy-F6P formation in the isomerization of 1,5-AG-6P catalyzed by EcYbiW. EIC monitoring for m/z=243 (in a negative ion mode) of 1-deoxy-F6P formation (t_{R}=18.40 min). B-C. Mass spectra (in a negative ion mode) of compound 1 (1-deoxy-F6P) and compound 2 (1,5-AG-6P) corresponding to the EIC peaks in A. "w/o" indicates "not containing".

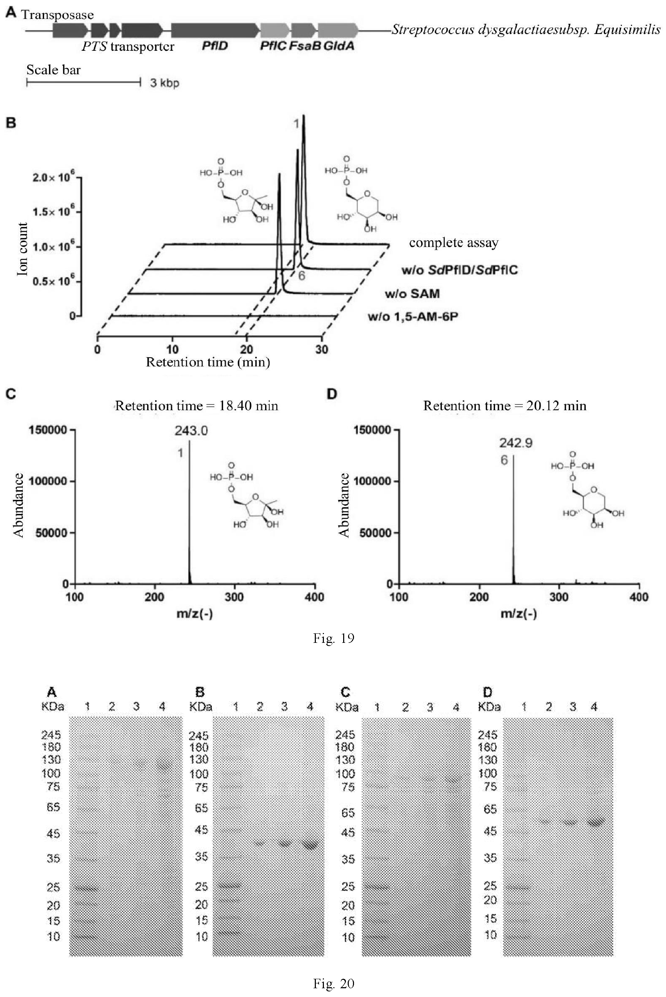

Fig. 19 shows the gene cluster of PflD from *Streptococcus dysgalactiae subsp. Equisimilis* and LC-MS enzyme reaction analysis. A. Gene cluster in *Streptococcus dysgalactiae subsp. equisimilis* (including the gene encoding the 1,5-anhydromannitol-6-phosphate isomerase shown in SEQ ID NO: 97). B. LC-MS detection of 1-deoxy-F6P formation in the isomerization of 1,5-AM-6P catalyzed by SdPflD. EIC monitoring for m/z=243 (in a negative ion mode) of 1-deoxy-F6P formation (t_{R}=18.40 min). C-D. Mass spectra (in a negative ion mode) of compound 1 (1-deoxy-F6P) and compound 6 (1,5-AM-6P) corresponding to the EIC peaks in B. "w/o" indicates "not containing".
FIG. 20 shows SDS-PAGE analysis of purified proteins for biochemical characterization and co-culture with *E. coli* MG1655 cells. A. α-1,4-glucan lyase; B. 1,5-anhydro-D-fructose reductase (Gafr, Uniprot accession No. P82125, EC:1.1.1.263); C. Maltose-binding protein (MBP)-1,5-anhydro-D-fructose reductase (Mafr, Uniprot accession No. Q2I8V6, EC:1.1.1.292); D. FDH. Lanes 1 to 4 in each 4-20% gradient gel (Bis-Tris) contain a protein molecular weight marker and 1 µg, 2 µg, and 4 µg of recombinant protein, respectively.

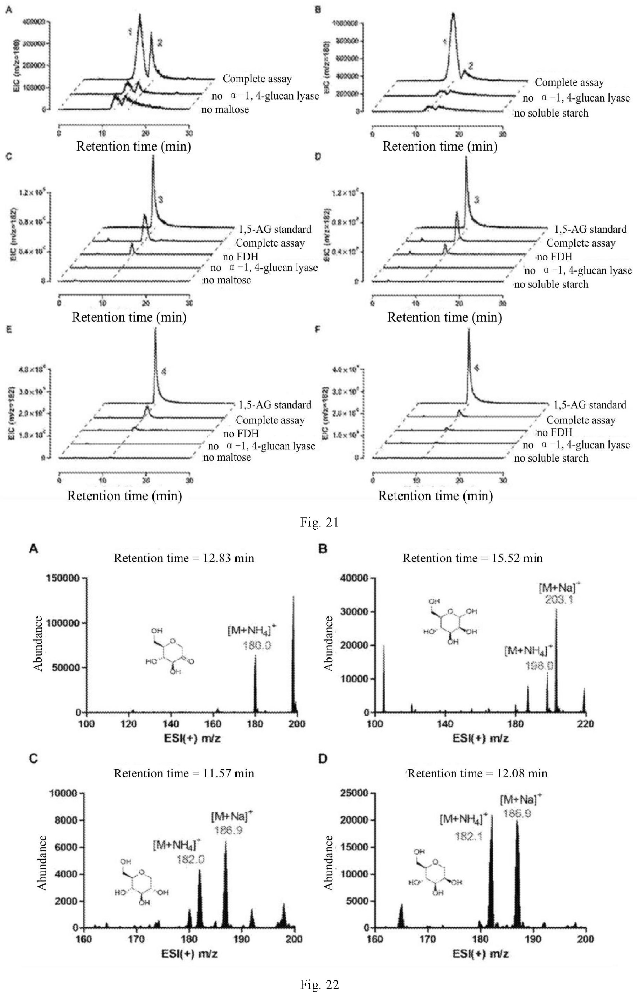

FIG. 21 shows LC-MS determination of enzyme activities of α-1,4-glucan lyase, Gafr, Mafr, and FDH. A, B. Formation of 1,5-anhydrofructose (1,5-AF) from maltose (A) and soluble starch (B) using α-1,4-glucan lyase, respectively, detected by LC-MS. Extracted ion chromatograms (EIC, m/z = 180) monitored formation of 1,5-AF (retention time 12.83 min, peak 1) in positive ion mode; peak 2 (retention time 15.52 min) was identified as glucose. C, D. Formation of 1,5-anhydroglucitol (1,5-AG) from maltose (C) and soluble starch (D) using α-1,4-glucan lyase, Gafr, and FDH, respectively, detected by LC-MS. Extracted ion chromatograms (EIC, m/z = 182) monitored formation of 1,5-AG (retention time 11.38 min, peak 3) in positive ion mode. E, F. Formation of 1,5-anhydromannitol (1,5-AM) from maltose (E) and soluble starch (F) using α-1,4-glucan lyase, Mafr, and FDH, respectively, detected by LC-MS. Extracted ion chromatograms (EIC, m/z = 182) in positive mode show formation of 1,5-AM (retention time 11.82 min, peak 4).
FIG. 22 shows mass spectra of the intermediates and products shown in FIG. 2. A-D. ESI(+) m/z spectra of 1,5-anhydrofructose (1,5-AF, peak 1), glucose (peak 2), 1,5-anhydroglucitol (1,5-AG, peak 3), and 1,5-anhydromannitol (1,5-AM, peak 4).

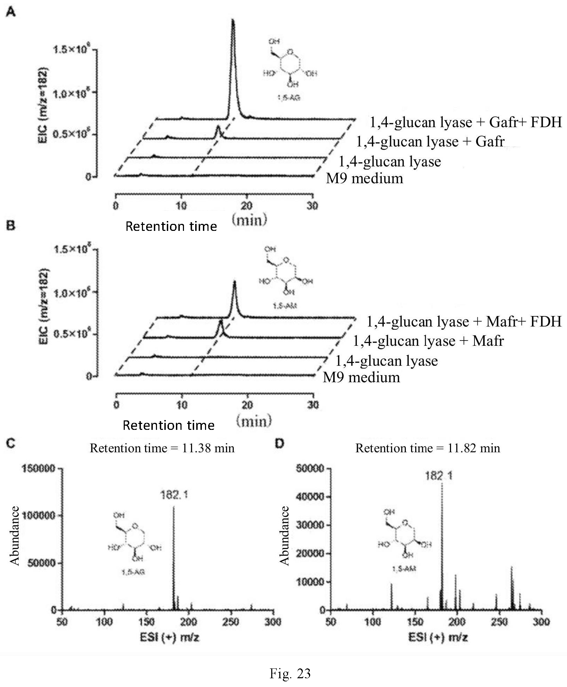

FIG. 23 shows LC-MS determination of enzyme activity in "starch M9 medium". A. Detection of 1,5-anhydroglucitol (1,5-AG) formed using α-1,4-glucan lyase, Gafr, and FDH by LC-MS. Extracted ion chromatogram (EIC) monitoring m/z = 182 in positive mode shows formation of 1,5-AG (retention time 11.38 min). B. Detection of 1,5-anhydromannitol (1,5-AM) formed from soluble starch in M9 medium using α-1,4-glucan lyase, Mafr, and FDH by LC-MS. Extracted ion chromatogram (EIC) at m/z = 182 in positive ion mode shows formation of 1,5-AM (retention time 11.82 min). C. Positive ion mass spectrum of the EIC peak ([M+NH₄]⁺) in A is 1,5-AG. D. Positive ion mass spectrum of the EIC peak ([M+NH₄]⁺) in B is 1,5-AM.

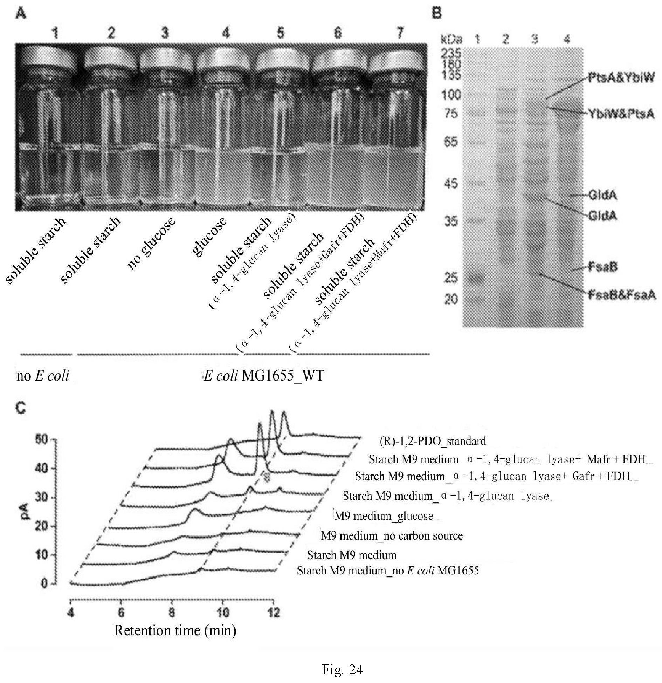

FIG. 24 shows anaerobic culture of wild-type *E. coli* MG1655 using starch. A. Growth of wild-type *E. coli* MG1655 co-cultured with α-1,4-glucan lyase, Gafr, MBP-Mafr, and FDH using soluble starch as the sole carbon source. In positive controls, glucose was used as the sole carbon source, in negative controls, M9 medium without carbon source, and "starch M9 medium" without *E. coli* MG1655 cells were used. Growth of strains and different carbon sources are also indicated in the figure. B. SDS-PAGE analysis of *E. coli* MG1655 grown on glucose (lane 2), "starch M9 medium" containing α-1,4-glucan lyase, Gafr, and FDH (lane 3), and "starch M9 medium" containing α-1,4-glucan lyase, MBP-Mafr, and FDH (lane 4). C. Gas chromatography analysis of 1,2-propanediol (t_{R} = 9.01 min) in fermentation extracts from co-culture of different enzyme combinations with wild-type *E. coli* MG1655 in starch M9 medium, including the authentic standard of (R)-1,2-propanediol.

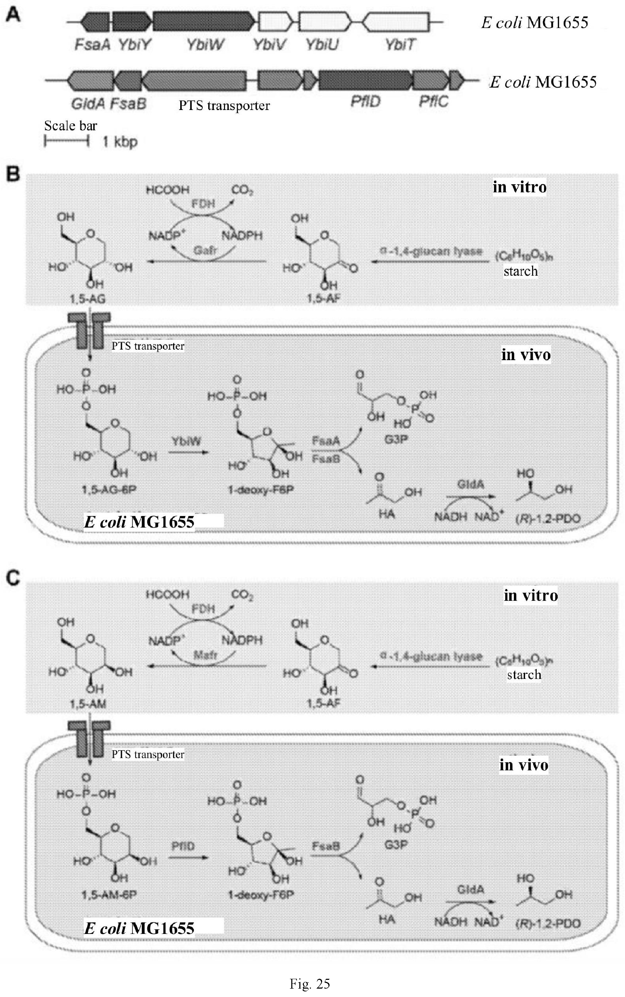

FIG. 25 shows in vitro enzyme reactions and the GRE-dependent anhydroglycolytic pathway. A. Gene clusters adjacent to YbiW and PflD in *E. coli* MG1655. B. Putative in vitro enzymatic reactions of α-1,4-glucan lyase, Gafr, FDH involved in starch hydrolysis, and the YbiW-dependent glycolytic process in *E. coli* MG1655 cells. C. Putative in vitro enzymatic reactions of α-1,4-glucan lyase, Gafr, FDH involved in starch hydrolysis, and the PflD-dependent glycolytic process in *E. coli* MG1655 cells.

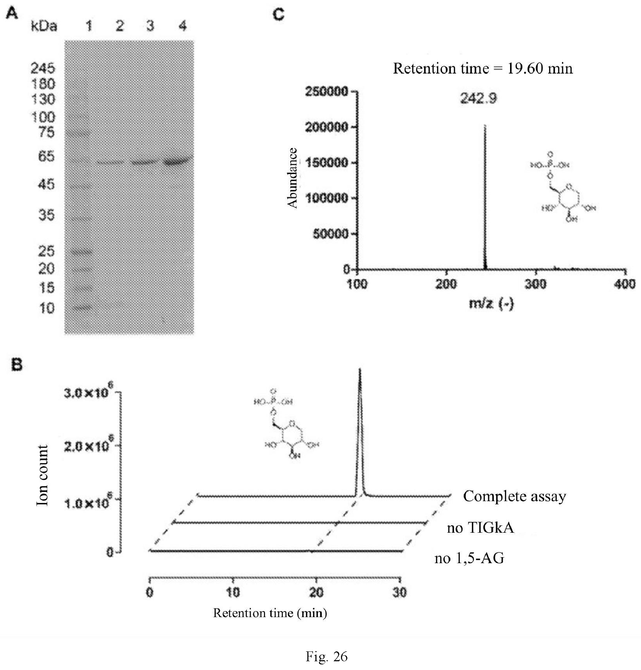

FIG. 26 shows SDS-PAGE analysis and enzyme activity assay of sugar kinase TlGlkA. A. 4-20% gradient gel (Bis-Tris): lane 1, protein molecular weight marker; lanes 2-4, 1 µg, 2 µg, and 4 µg of purified TlGlkA, respectively. B. Detection of 1,5-anhydroglucitol-6-phosphate (1,5-AG-6P) formation in the TlGlkA-catalyzed phosphorylation of 1,5-anhydroglucitol by LC-MS. Extracted ion chromatogram (EIC) at m/z = 243 in negative ion mode monitored formation of 1,5-AG-6P (t_{R} = 19.60 min). C. Mass spectrum of the compound 1,5-AG-6P corresponding to the EIC peak in B (in a negative ion mode).

### Sequence Descriptions

SEQ ID NO: 1 shows the amino acid sequence of 1,5-anhydroglucitol-6-phosphate isomerase with the Uniprot accession No. P75793, expressed by *Escherichia coli*.
SEQ ID NO: 2 shows the amino acid sequence of 1,5-anhydroglucitol-6-phosphate isomerase with the Uniprot accession No. A0A807DR53, expressed by *Lactiplantibacillus plantarum*.
SEQ ID NO: 3 shows the amino acid sequence of 1,5-anhydroglucitol-6-phosphate isomerase with the Uniprot accession No. A0A0R2FVZ1, expressed by *Lactobacillus selangorensis*.
SEQ ID NO: 4 shows the amino acid sequence of 1,5-anhydroglucitol-6-phosphate isomerase with the Uniprot accession No. A0A0A6S4M4, expressed by *Streptococcus uberis*.
SEQ ID NO: 5 shows the amino acid sequence of 1,5-anhydroglucitol-6-phosphate isomerase with the Uniprot accession No. A0A0K1F3T2, expressed by *Olsenella sp. oral taxon 807*.
SEQ ID NO: 6 shows the amino acid sequence of 1,5-anhydroglucitol-6-phosphate isomerase with the Uniprot accession No. A0A134AK54, expressed by *Leptotrichia wadei*.
SEQ ID NO: 7 shows the amino acid sequence of 1,5-anhydroglucitol-6-phosphate isomerase with the Uniprot accession No. A0A134A1S1, expressed by *Senella sp.* DNF00959.
SEQ ID NO: 8 shows the amino acid sequence of 1,5-anhydroglucitol-6-phosphate isomerase with the Uniprot accession No. A0A0E2UQR7, expressed by *Streptococcus parauberis*.
SEQ ID NO: 9 shows the amino acid sequence of 1,5-anhydroglucitol-6-phosphate isomerase with the Uniprot accession No. A0A173RRH3, expressed by *Anaerostipes hadrus*.
SEQ ID NO: 10 shows the amino acid sequence of 1,5-anhydroglucitol-6-phosphate isomerase with the Uniprot accession No. A0A1E8VV95, expressed by *Senella sp.* HMSC062G07.
SEQ ID NO: 11 shows the amino acid sequence of 1,5-anhydroglucitol-6-phosphate isomerase with the Uniprot accession No. A0A1I3HGA4, expressed by *Selenomonas ruminantium*.
SEQ ID NO: 12 shows the amino acid sequence of 1,5-anhydroglucitol-6-phosphate isomerase with the Uniprot accession No. A0A285HLM7, expressed by *Orenia metallireducens*.
SEQ ID NO: 13 shows the amino acid sequence of 1,5-anhydroglucitol-6-phosphate isomerase with the Uniprot accession No. A0A2M9H9C8, expressed by *Bifidobacterium primatium*.
SEQ ID NO: 14 shows the amino acid sequence of 1,5-anhydroglucitol-6-phosphate isomerase with the Uniprot accession No. C9N0T4, expressed by *Leptotrichia hofstadii* F0254.
SEQ ID NO: 15 shows the amino acid sequence of 1,5-anhydroglucitol-6-phosphate isomerase with the Uniprot accession No. A0A0R2AF79, expressed by *Ligilactobacillus agilis* DSM 20509.
SEQ ID NO: 16 shows the amino acid sequence of 1,5-anhydroglucitol-6-phosphate isomerase with the Uniprot accession No. U2TM27, expressed by *Senella profusa* F0195.
SEQ ID NO: 17 shows the amino acid sequence of 1,5-anhydroglucitol-6-phosphate isomerase with the Uniprot accession No. A0A0A6PW80, expressed by *Clostridium butyricum*.
SEQ ID NO: 18 shows the amino acid sequence of 1,5-anhydroglucitol-6-phosphate isomerase with the Uniprot accession No. A0A1C0A9L3, expressed by *Orenia metallireducens*.
SEQ ID NO: 19 shows the amino acid sequence of 1,5-anhydroglucitol-6-phosphate isomerase with the Uniprot accession No. A0A0B8PMP8, expressed by *Vibrio ishigakensis*.
SEQ ID NO: 20 shows the amino acid sequence of 1,5-anhydroglucitol-6-phosphate isomerase with the Uniprot accession No. A0A425WQY7, expressed by *Coriobacteriaceae*.
SEQ ID NO: 21 shows the amino acid sequence of 1,5-anhydroglucitol-6-phosphate isomerase with the Uniprot accession No. F2N714, expressed by *Coriobacterium glomerans* strain ATCC 49209/DSM 20642/JCM 10262/PW2.
SEQ ID NO: 22 shows the amino acid sequence of 1,5-anhydroglucitol-6-phosphate isomerase with the Uniprot accession No. M2NEK5, expressed by *Eggerthia cateniformis* OT 569.
SEQ ID NO: 23 shows the amino acid sequence of 1,5-anhydroglucitol-6-phosphate isomerase with the Uniprot accession No. A0A0A7FYZ2, expressed by *Clostridium baratii str. Sullivan*.
SEQ ID NO: 24 shows the amino acid sequence of 1,5-anhydroglucitol-6-phosphate isomerase with the Uniprot accession No. A0A143X8V0, expressed by *Clostridiales* bacterium CHKC1006.
SEQ ID NO: 25 shows the amino acid sequence of 1,5-anhydroglucitol-6-phosphate isomerase with the Uniprot accession No. A0A2N2BBI5, expressed by *Firmicutes* bacterium HGW-Firmicutes-5.
SEQ ID NO: 26 shows the amino acid sequence of 1,5-anhydroglucitol-6-phosphate isomerase with the Uniprot accession No. A0A2T0BJ14, expressed by *Clostridium vincentii*.
SEQ ID NO: 27 shows the amino acid sequence of 1,5-anhydroglucitol-6-phosphate isomerase with the Uniprot accession No. A0A380JDF6, expressed by *Streptococcus downei* MFe28.
SEQ ID NO: 28 shows the amino acid sequence of 1,5-anhydroglucitol-6-phosphate isomerase with the Uniprot accession No. A0A847CI73, expressed by *Erysipelotrichaceae* bacterium.
SEQ ID NO: 29 shows the amino acid sequence of 1,5-anhydroglucitol-6-phosphate isomerase with the Uniprot accession No. A0A8J6Z417, expressed by *Quinella sp. 3Q1*.
SEQ ID NO: 30 shows the amino acid sequence of 1,5-anhydroglucitol-6-phosphate isomerase with the Uniprot accession No. D1AFQ7, expressed by *Sebaldella termitidis* (strain ATCC 33386/NCTC 11300).
SEQ ID NO: 31 shows the amino acid sequence of 1,5-anhydroglucitol-6-phosphate isomerase with the Uniprot accession No. A0A062WZ81, expressed by *Ligilactobacillus animalis*.
SEQ ID NO: 32 shows the amino acid sequence of 1,5-anhydroglucitol-6-phosphate isomerase with the Uniprot accession No. A0A0R1V613, expressed by *Liquorilactobacillus satsumensis* DSM 16230.
SEQ ID NO: 33 shows the amino acid sequence of 1,5-anhydroglucitol-6-phosphate isomerase with the Uniprot accession No. A0A0M0A869, expressed by *Clostridium botulinum*.
SEQ ID NO: 34 shows the amino acid sequence of 1,5-anhydroglucitol-6-phosphate isomerase with the Uniprot accession No. A0A0V8QAB4, expressed by *Acetivibrio ethanolgignens*.
SEQ ID NO: 35 shows the amino acid sequence of 1,5-anhydroglucitol-6-phosphate isomerase with the Uniprot accession No. A0A2N3J357, expressed by *Aeromonas sobria*.
SEQ ID NO: 36 shows the amino acid sequence of 1,5-anhydroglucitol-6-phosphate isomerase with the Uniprot accession No. A0A1I2MD83, expressed by *Clostridium cadaveris*.
SEQ ID NO: 37 shows the amino acid sequence of 1,5-anhydroglucitol-6-phosphate isomerase with the Uniprot accession No. A0A1L8MKJ9, expressed by *Streptococcus bovimastitidis*.
SEQ ID NO: 38 shows the amino acid sequence of 1,5-anhydroglucitol-6-phosphate isomerase with the Uniprot accession No. A0A4R1NC63, expressed by *Sodalis ligni*.
SEQ ID NO: 39 shows the amino acid sequence of 1,5-anhydroglucitol-6-phosphate isomerase with the Uniprot accession No. A0A5R9CBU2, expressed by *Lactococcus raffinolactis*.
SEQ ID NO: 40 shows the amino acid sequence of 1,5-anhydroglucitol-6-phosphate isomerase with the Uniprot accession No. C2ELS4, expressed by *Lactobacillus ultunensis* DSM 16047.
SEQ ID NO: 41 shows the amino acid sequence of 1,5-anhydroglucitol-6-phosphate isomerase with the Uniprot accession No. A0A3P1W4E8, expressed by *Leptotrichia sp.* OH3620.
SEQ ID NO: 42 shows the amino acid sequence of 1,5-anhydroglucitol-6-phosphate isomerase with the Uniprot accession No. A0A498R759, expressed by *Lucifera butyrica*.
SEQ ID NO: 43 shows the amino acid sequence of 1,5-anhydroglucitol-6-phosphate isomerase with the Uniprot accession No. A0A4V6Q2Z2, expressed by *Fonticella tunisiensis*.
SEQ ID NO: 44 shows the amino acid sequence of 1,5-anhydroglucitol-6-phosphate isomerase with the Uniprot accession No. A7MME9, expressed by *Cronobacter sakazakii* strain ATCC BAA-894.
SEQ ID NO: 45 shows the amino acid sequence of 1,5-anhydroglucitol-6-phosphate isomerase with the Uniprot accession No. A0A0R1Q920, expressed by *Liquorilactobacillus uvarum* DSM 19971.
SEQ ID NO: 46 shows the amino acid sequence of 1,5-anhydroglucitol-6-phosphate isomerase with the Uniprot accession No. A0A1Y4F189, expressed by *Anaeromassilibacillus sp.* An250.
SEQ ID NO: 47 shows the amino acid sequence of 1,5-anhydroglucitol-6-phosphate isomerase with the Uniprot accession No. A0A498D0T9, expressed by *Anaerotruncus sp.* 22A2-44.
SEQ ID NO: 48 shows the amino acid sequence of 1,5-anhydroglucitol-6-phosphate isomerase with the Uniprot accession No. A0A510JEA5, expressed by *Leptotrichia hofstadii*.
SEQ ID NO: 49 shows the amino acid sequence of 1,5-anhydroglucitol-6-phosphate isomerase with the Uniprot accession No. A0A0A2VZ96, expressed by *Beauveria bassiana* D1-5.
SEQ ID NO: 50 shows the amino acid sequence of 1,5-anhydroglucitol-6-phosphate isomerase with the Uniprot accession No. A0A447V187, expressed by *Cedecea lapagei*.
SEQ ID NO: 51 shows the amino acid sequence of 1,5-anhydroglucitol-6-phosphate isomerase with the Uniprot accession No. B2ISH7, expressed by *Streptococcus pneumoniae* strain CGSP14.
SEQ ID NO: 52 shows the amino acid sequence of 1,5-anhydroglucitol-6-phosphate isomerase with the Uniprot accession No. A0A075SG00, expressed by *Streptococcus suis* 6407.
SEQ ID NO: 53 shows the amino acid sequence of 1,5-anhydroglucitol-6-phosphate isomerase with the Uniprot accession No. A0A0R2GM47, expressed by *Lactiplantibacillus plantarum*.
SEQ ID NO: 54 shows the amino acid sequence of 1,5-anhydroglucitol-6-phosphate isomerase with the Uniprot accession No. A0A1Q8F4Y7, expressed by *Aeromonas veronii*.
SEQ ID NO: 55 shows the amino acid sequence of 1,5-anhydroglucitol-6-phosphate isomerase with the Uniprot accession No. A0A290PWH6, expressed by *Lactococcus raffinolactis*.
SEQ ID NO: 56 shows the amino acid sequence of 1,5-anhydroglucitol-6-phosphate isomerase with the Uniprot accession No. A0A447Y009, expressed by *Escherichia coli*.
SEQ ID NO: 57 shows the amino acid sequence of 1,5-anhydroglucitol-6-phosphate isomerase with the Uniprot accession No. A0A0B7GIL0, expressed by *Streptococcus sanguinis*.
SEQ ID NO: 58 shows the amino acid sequence of 1,5-anhydroglucitol-6-phosphate isomerase with the Uniprot accession No. A0A1L7RNK7, expressed by *Actinomyces succiniciruminis*.
SEQ ID NO: 59 shows the amino acid sequence of 1,5-anhydroglucitol-6-phosphate isomerase with the Uniprot accession No. A0A376TJI4, expressed by *Escherichia coli*.
SEQ ID NO: 60 shows the amino acid sequence of 1,5-anhydroglucitol-6-phosphate isomerase with the Uniprot accession No. A0A3R9YDI0, expressed by *Vagococcus humatus*.
SEQ ID NO: 61 shows the amino acid sequence of 1,5-anhydroglucitol-6-phosphate isomerase with the Uniprot accession No. A0A064C019, expressed by *Streptococcus pneumoniae*.
SEQ ID NO: 62 shows the amino acid sequence of 1,5-anhydroglucitol-6-phosphate isomerase with the Uniprot accession No. A0A0P7FGJ3, expressed by *Vibrio alginolyticus*.
SEQ ID NO: 63 shows the amino acid sequence of 1,5-anhydroglucitol-6-phosphate isomerase with the Uniprot accession No. A0A0R2KGV0, expressed by *Ligilactobacillus acidipiscis*.
SEQ ID NO: 64 shows the amino acid sequence of 1,5-anhydroglucitol-6-phosphate isomerase with the Uniprot accession No. A0A1T4P4Y5, expressed by *Pilibacter termitis*.
SEQ ID NO: 65 shows the amino acid sequence of 1,5-anhydroglucitol-6-phosphate isomerase with the Uniprot accession No. A0A1E3KU28, expressed by *Lactiplantibacillus plantarum*.
SEQ ID NO: 66 shows the amino acid sequence of 1,5-anhydroglucitol-6-phosphate isomerase with the Uniprot accession No. A0A349BXL4, expressed by *Lachnospiraceae bacterium*.
SEQ ID NO: 67 shows the amino acid sequence of 1,5-anhydroglucitol-6-phosphate isomerase with the Uniprot accession No. A0A3T0ZV83, expressed by *Aeromonas hydrophila*.
SEQ ID NO: 68 shows the amino acid sequence of 1,5-anhydroglucitol-6-phosphate isomerase with the Uniprot accession No. A0A1L8WPN6, expressed by *Enterococcus ratti*.
SEQ ID NO: 69 shows the amino acid sequence of 1,5-anhydroglucitol-6-phosphate isomerase with the Uniprot accession No. A0A0R2H5D7, expressed by *Kandleria vitulina*.
SEQ ID NO: 70 shows the amino acid sequence of 1,5-anhydroglucitol-6-phosphate isomerase with the Uniprot accession No. A0A0H3J4P2, expressed by *Clostridium pasteurianum* DSM 525.
SEQ ID NO: 71 shows the amino acid sequence of 1,5-anhydroglucitol-6-phosphate isomerase with the Uniprot accession No. A0A0T6U3W3, expressed by *Aeromonas allosaccharophila*.
SEQ ID NO: 72 shows the amino acid sequence of 1,5-anhydroglucitol-6-phosphate isomerase with the Uniprot accession No. K8CC45, expressed by *Cronobacter sakazakii*.
SEQ ID NO: 73 shows the amino acid sequence of 1,5-anhydroglucitol-6-phosphate isomerase with the Uniprot accession No. A0A1W6B1J9, expressed by *Pantoea alhagi*.
SEQ ID NO: 74 shows the amino acid sequence of 1,5-anhydroglucitol-6-phosphate isomerase with the Uniprot accession No. B6FW14, expressed by *Peptacetobacter hiranonis* strain DSM 13275/JCM 10541/KCTC 15199/TO-931.
SEQ ID NO: 75 shows the amino acid sequence of 1,5-anhydroglucitol-6-phosphate isomerase with the Uniprot accession No. A0A034T1M9, expressed by *Edwardsiella piscicida*.
SEQ ID NO: 76 shows the amino acid sequence of 1,5-anhydroglucitol-6-phosphate isomerase with the Uniprot accession No. A0A089PXZ6, expressed by *Cedecea neteri*.
SEQ ID NO: 77 shows the amino acid sequence of 1,5-anhydroglucitol-6-phosphate isomerase with the Uniprot accession No. A0A0A3AQL5, expressed by *Chelonobacter oris*.
SEQ ID NO: 78 shows the amino acid sequence of 1,5-anhydroglucitol-6-phosphate isomerase with the Uniprot accession No. A0A0D0QVG1, expressed by *Aeromonas sp.* L_1B5_3.
SEQ ID NO: 79 shows the amino acid sequence of 1,5-anhydroglucitol-6-phosphate isomerase with the Uniprot accession No. A0A0F4VRP2, expressed by *Clostridium sp.* IBUN125C.
SEQ ID NO: 80 shows the amino acid sequence of 1,5-anhydroglucitol-6-phosphate isomerase with the Uniprot accession No. A0A1M5EXF5, expressed by *Vibrio gazogenes* DSM 21264.
SEQ ID NO: 81 shows the amino acid sequence of 1,5-anhydroglucitol-6-phosphate isomerase with the Uniprot accession No. A0A2Z5Y015, expressed by *Melissococcus plutonius*.
SEQ ID NO: 82 shows the amino acid sequence of 1,5-anhydroglucitol-6-phosphate isomerase with the Uniprot accession No. A0A5C7QZ11, expressed by *Tolumonas sp..*
SEQ ID NO: 83 shows the amino acid sequence of 1,5-anhydroglucitol-6-phosphate isomerase with the Uniprot accession No. A0A3D0GQ55, expressed by *Erysipelotrichaceae bacterium*.
SEQ ID NO: 84 shows the amino acid sequence of 1,5-anhydroglucitol-6-phosphate isomerase with the Uniprot accession No. A0A0R1F6B2, expressed by *Loigolactobacillus coryniformis subsp. coryniformis* KCTC 3167.
SEQ ID NO: 85 shows the amino acid sequence of 1,5-anhydroglucitol-6-phosphate isomerase with the Uniprot accession No. A0A4P9VTG8, expressed by *Zooshikella ganghwensis*.
SEQ ID NO: 86 shows the amino acid sequence of 1,5-anhydroglucitol-6-phosphate isomerase with the Uniprot accession No. A0A829T2C0, expressed by *Vibrio sp.*.
SEQ ID NO: 87 shows the amino acid sequence of 1,5-anhydroglucitol-6-phosphate isomerase with the Uniprot accession No. A0A2T3N2G3, expressed by *Photobacterium lipolyticum*.
SEQ ID NO: 88 shows the amino acid sequence of 1,5-anhydroglucitol-6-phosphate isomerase with the Uniprot accession No. A0A8S7XWX0, expressed by *Escherichia coli*.
SEQ ID NO: 89 shows the amino acid sequence of 1,5-anhydroglucitol-6-phosphate isomerase with the Uniprot accession No. X0PS46, expressed by *Agrilactobacillus composti* DSM 18527.
SEQ ID NO: 90 shows the amino acid sequence of 1,5-anhydroglucitol-6-phosphate isomerase with the Uniprot accession No. A0A061Q2Y5, expressed by *Vibrio sp.* JCM 19052.
SEQ ID NO: 91 shows the amino acid sequence of 1,5-anhydroglucitol-6-phosphate isomerase with the Uniprot accession No. A0A174H662, expressed by *Clostridium symbiosum*.
SEQ ID NO: 92 shows the amino acid sequence of 1,5-anhydroglucitol-6-phosphate isomerase with the Uniprot accession No. F3Y874, expressed by *Melissococcus plutonius* strain ATCC 35311/CIP 104052/LMG 20360/NCIMB 702443.
SEQ ID NO: 93 shows the amino acid sequence of 1,5-anhydroglucitol-6-phosphate isomerase with the Uniprot accession No. A0A1S1BRH2, expressed by *Aerococcus sp.* HMSC035B07.
SEQ ID NO: 94 shows the amino acid sequence of 1,5-anhydroglucitol-6-phosphate isomerase with the Uniprot accession No. A0A1Y4BCL8, expressed by *Senella sp.* An293.
SEQ ID NO: 95 shows the amino acid sequence of 1,5-anhydroglucitol-6-phosphate isomerase with the Uniprot accession No. A0A7Z0VH52.
SEQ ID NO: 96 shows the amino acid sequence of 1,5-anhydroglucitol-6-phosphate isomerase with the Uniprot accession No. A0A0I9WIZ4.
SEQ ID NO: 97 shows the amino acid sequence of 1,5-anhydromannitol-6-phosphate isomerase with the NCBI accession No. OCX05109.1, expressed by *Streptococcus dysgalactiae subsp. equisimilis*.
SEQ ID NO: 98 shows the amino acid sequence of 1,5-anhydromannitol-6-phosphate isomerase with the Uniprot accession No. P32674, expressed by *Escherichia coli* strain K12.
SEQ ID NO: 99 shows the amino acid sequence of 1,5-anhydromannitol-6-phosphate isomerase with the Uniprot accession No. A0A7C9H654, expressed by *Firmicutes bacterium*.
SEQ ID NO: 100 shows the amino acid sequence of 1,5-anhydromannitol-6-phosphate isomerase with the Uniprot accession No. A0A510J954, expressed by *Pseudoleptotrichia goodfellowii*.
SEQ ID NO: 101 shows the amino acid sequence of 1,5-anhydromannitol-6-phosphate isomerase with the Uniprot accession No. D4F6M7, expressed by *Edwardsiella tarda*.
SEQ ID NO: 102 shows the amino acid sequence of 1,5-anhydromannitol-6-phosphate isomerase with the Uniprot accession No. D4E0C4, expressed by *Serratia odorifera* DSM 4582.
SEQ ID NO: 103 shows the amino acid sequence of 1,5-anhydromannitol-6-phosphate isomerase with the Uniprot accession No. A0A2V4E297, expressed by *Gilliamella apicola*.
SEQ ID NO: 104 shows the amino acid sequence of 1,5-anhydromannitol-6-phosphate isomerase with the Uniprot accession No. A0A084T2T3, expressed by *Vibrio sp.* ER1A.
SEQ ID NO: 105 shows the amino acid sequence of 1,5-anhydromannitol-6-phosphate isomerase with the Uniprot accession No. A0A0R2FPV6, expressed by *Lactobacillus selangorensis*.
SEQ ID NO: 106 shows the amino acid sequence of 1,5-anhydromannitol-6-phosphate isomerase with the Uniprot accession No. A0A0N8NM72, expressed by *Caloranaerobacter sp.*.
SEQ ID NO: 107 shows the amino acid sequence of 1,5-anhydromannitol-6-phosphate isomerase with the Uniprot accession No. A0A1T4K0K6, expressed by *Pilibacter termitis*.
SEQ ID NO: 108 shows the amino acid sequence of 1,5-anhydromannitol-6-phosphate isomerase with the Uniprot accession No. A0A096BHK0, expressed by *Caloranaerobacter azorensis* H53214.
SEQ ID NO: 109 shows the amino acid sequence of 1,5-anhydromannitol-6-phosphate isomerase with the Uniprot accession No. A0A725B0Z8, expressed by *Salmonella enteritidis*.
SEQ ID NO: 110 shows the amino acid sequence of 1,5-anhydromannitol-6-phosphate isomerase with the Uniprot accession No. A0A0B8Q7B9, expressed by *Vibrio sp.* JCM 19236.
SEQ ID NO: 111 shows the amino acid sequence of 1,5-anhydromannitol-6-phosphate isomerase with the Uniprot accession No. A0A037YM28, expressed by *Escherichia coli*.
SEQ ID NO: 112 shows the amino acid sequence of 1,5-anhydromannitol-6-phosphate isomerase with the Uniprot accession No. C4LFY8, expressed by *Tolumonas auensis* strain DSM 9187/TA4.
SEQ ID NO: 113 shows the amino acid sequence of 1,5-anhydromannitol-6-phosphate isomerase with the Uniprot accession No. A0A6N7XPT4, expressed by *Senella porci*.
SEQ ID NO: 114 shows the amino acid sequence of 1,5-anhydromannitol-6-phosphate isomerase with the Uniprot accession No. A0A4S2F5T8, expressed by *Coriobacteriaceae* bacterium.
SEQ ID NO: 115 shows the amino acid sequence of 1,5-anhydromannitol-6-phosphate isomerase with the Uniprot accession No. A0A4R7KVA0, expressed by *Fonticella tunisiensis*.
SEQ ID NO: 116 shows the amino acid sequence of 1,5-anhydromannitol-6-phosphate isomerase with the Uniprot accession No. A0A437UWZ5, expressed by *Coriobacteriales* bacterium OH1046.
SEQ ID NO: 117 shows the amino acid sequence of 1,5-anhydromannitol-6-phosphate isomerase with the Uniprot accession No. A0A3R9P0U8, expressed by *Bacillus sp.* HMF5848.
SEQ ID NO: 118 shows the amino acid sequence of 1,5-anhydromannitol-6-phosphate isomerase with the Uniprot accession No. A0A380TM32, expressed by *Actinobacillus rossii*.
SEQ ID NO: 119 shows the amino acid sequence of 1,5-anhydromannitol-6-phosphate isomerase with the Uniprot accession No. C2ELS8, expressed by *Lactobacillus ultunensis* DSM 16047.
SEQ ID NO: 120 shows the amino acid sequence of 1,5-anhydromannitol-6-phosphate isomerase with the Uniprot accession No. A0A2N2B0Z5, expressed by *Firmicutes* bacterium HGW-Firmicutes-7.
SEQ ID NO: 121 shows the amino acid sequence of 1,5-anhydromannitol-6-phosphate isomerase with the Uniprot accession No. A0A1J0GF99, expressed by *Clostridium estertheticum subsp.* estertheticum.
SEQ ID NO: 122 shows the amino acid sequence of 1,5-anhydromannitol-6-phosphate isomerase with the Uniprot accession No. A0A1D8GPW2, expressed by *Geosporobacter ferrireducens*.
SEQ ID NO: 123 shows the amino acid sequence of 1,5-anhydromannitol-6-phosphate isomerase with the Uniprot accession No. A0A0B8PE23, expressed by *Vibrio ishigakensis*.
SEQ ID NO: 124 shows the amino acid sequence of 1,5-anhydromannitol-6-phosphate isomerase with the Uniprot accession No. A0A239SQG5, expressed by *Streptococcus merionis*.
SEQ ID NO: 125 shows the amino acid sequence of 1,5-anhydromannitol-6-phosphate isomerase with the Uniprot accession No. A0A823B0Q1, expressed by *Shigella boydii*.
SEQ ID NO: 126 shows the amino acid sequence of 1,5-anhydromannitol-6-phosphate isomerase with the Uniprot accession No. X0PU04, expressed by *Agrilactobacillus composti* DSM 18527.
SEQ ID NO: 127 shows the amino acid sequence of 1,5-anhydromannitol-6-phosphate isomerase with the Uniprot accession No. A0A0R2FH98, expressed by *Lactobacillus selangorensis*.
SEQ ID NO: 128 shows the amino acid sequence of 1,5-anhydromannitol-6-phosphate isomerase with the Uniprot accession No. A0A430AQ99, expressed by *Vagococcus elongatus*.
SEQ ID NO: 129 shows the amino acid sequence of 1,5-anhydromannitol-6-phosphate isomerase with the Uniprot accession No. A0A1W1WZ75, expressed by *Clostridium acidisoli* DSM 12555.
SEQ ID NO: 130 shows the amino acid sequence of 1,5-anhydromannitol-6-phosphate isomerase with the Uniprot accession No. A0A376L3P4, expressed by *Escherichia coli*.
SEQ ID NO: 131 shows the amino acid sequence of 1,5-anhydromannitol-6-phosphate isomerase with the Uniprot accession No. A0A1T4WMT8, expressed by *Caloramator quimbayensis*.
SEQ ID NO: 132 shows the amino acid sequence of 1,5-anhydromannitol-6-phosphate isomerase with the Uniprot accession No. A0A1I1H9K1, expressed by *Clostridium uliginosum*.
SEQ ID NO: 133 shows the amino acid sequence of 1,5-anhydromannitol-6-phosphate isomerase with the Uniprot accession No. A0A143I1W1, expressed by *Enterobacter asburiae*.
SEQ ID NO: 134 shows the amino acid sequence of 1,5-anhydromannitol-6-phosphate isomerase with the Uniprot accession No. N2BYX9, expressed by *Atopobium minutum*.
SEQ ID NO: 135 shows the amino acid sequence of 1,5-anhydromannitol-6-phosphate isomerase with the Uniprot accession No. A0A4S2EVE2, expressed by *Coriobacteriaceae* bacterium.
SEQ ID NO: 136 shows the amino acid sequence of 1,5-anhydromannitol-6-phosphate isomerase with the Uniprot accession No. A0A7S7M803, expressed by *Thermophilobacter immobilis*.
SEQ ID NO: 137 shows the amino acid sequence of 1,5-anhydromannitol-6-phosphate isomerase with the Uniprot accession No. A0A2T3N2G1, expressed by *Photobacterium lipolyticum*.
SEQ ID NO: 138 shows the amino acid sequence of 1,5-anhydromannitol-6-phosphate isomerase with the Uniprot accession No. A0A381GG45, expressed by *Citrobacter amalonaticus*.
SEQ ID NO: 139 shows the amino acid sequence of 1,5-anhydromannitol-6-phosphate isomerase with the Uniprot accession No. A0A0A0F8F5, expressed by *Escherichia coli* G3.
SEQ ID NO: 140 shows the amino acid sequence of 1,5-anhydromannitol-6-phosphate isomerase with the Uniprot accession No. A0A0J0GCD9, expressed by *Enterobacter asburiae*.
SEQ ID NO: 141 shows the amino acid sequence of 1,5-anhydromannitol-6-phosphate isomerase with the Uniprot accession No. A0A0H3J2B1, expressed by *Clostridium pasteurianum*.
SEQ ID NO: 142 shows the amino acid sequence of 1,5-anhydromannitol-6-phosphate isomerase with the Uniprot accession No. D1AJA5, expressed by *Sebaldella termitidis* strain ATCC 33386/NCTC 11300.
SEQ ID NO: 143 shows the amino acid sequence of 1,5-anhydromannitol-6-phosphate isomerase with the Uniprot accession No. A0A8S0W6S0, expressed by *Acididesulfobacillus acetoxydans*.
SEQ ID NO: 144 shows the amino acid sequence of 1,5-anhydromannitol-6-phosphate isomerase with the Uniprot accession No. A0A7W2G1V0, expressed by *Clostridium sp.*.
SEQ ID NO: 145 shows the amino acid sequence of LpYbiY with the Uniprot accession No. A0A162FGG2, expressed by *Lactiplantibacillus plantarum*.
SEQ ID NO: 146 shows the amino acid sequence of LpFsaA with the Uniprot accession No. A0A0M4CJJ6, expressed by *Lactiplantibacillus plantarum*.
SEQ ID NO: 147 shows the amino acid sequence of EcPflC with the Uniprot accession No. P32675, expressed by *Escherichia coli* strain K12.
SEQ ID NO: 148 shows the amino acid sequence of EcFsaB with the Uniprot accession No. P32669, expressed by *Escherichia coli* strain K12.
SEQ ID NO: 149 shows the amino acid sequence of EcGldA with the Uniprot accession No. P0A9S5, expressed by *Escherichia coli* strain K12.
SEQ ID NO: 150 shows the amino acid sequence of PtsA, a subunit of the PTS with the Uniprot accession No. P32670, expressed by *Escherichia coli* strain K12.
SEQ ID NO: 151 shows the amino acid sequence of FrwB, FrwC and FrwD, subunits of the PTS with the Uniprot accession No. P69816, expressed by *Escherichia coli* strain K12.
SEQ ID NO: 152 shows the amino acid sequence of FrwC, a subunit of the PTS with the Uniprot accession No. P32672, expressed by *Escherichia coli* strain K12.
SEQ ID NO: 153 shows the amino acid sequence of FrwD, a subunit of the PTS with the Uniprot accession No. P32676, expressed by *Escherichia coli* strain K12.
SEQ ID NO: 154 shows the nucleotide sequence of Primer 1F.
SEQ ID NO: 155 shows the nucleotide sequence of Primer 1R.
SEQ ID NO: 156 shows the nucleotide sequence of Primer 2F.
SEQ ID NO: 157 shows the nucleotide sequence of Primer 2R.
SEQ ID NO: 158 shows the nucleotide sequence of Primer 3F.
SEQ ID NO: 159 shows the nucleotide sequence of Primer 3R.
SEQ ID NO: 160 shows the nucleotide sequence of Primer 4F.
SEQ ID NO: 161 shows the nucleotide sequence of Primer 4R.
SEQ ID NO: 162 shows the nucleotide sequence of Primer 5F.
SEQ ID NO: 163 shows the nucleotide sequence of Primer 5R.
SEQ ID NO: 164 shows the nucleotide sequence of Primer 6F.
SEQ ID NO: 165 shows the nucleotide sequence of Primer 6R.
SEQ ID NO: 166 shows the nucleotide sequence of Primer 7F.
SEQ ID NO: 167 shows the nucleotide sequence of Primer 7R.
SEQ ID NO: 168 shows the nucleotide sequence of Primer 8F.
SEQ ID NO: 169 shows the nucleotide sequence of Primer 8R.
SEQ ID NO: 170 shows the nucleotide sequence of Primer 9F.
SEQ ID NO: 171 shows the nucleotide sequence of Primer 9R.
SEQ ID NO: 172 shows the nucleotide sequence of Primer 10F.
SEQ ID NO: 173 shows the nucleotide sequence of Primer 10R.
SEQ ID NO: 174 shows the nucleotide sequence of Primer 11F.
SEQ ID NO: 175 shows the nucleotide sequence of Primer 11R.
SEQ ID NO: 176 shows the nucleotide sequence of Primer 12F.
SEQ ID NO: 177 shows the nucleotide sequence of Primer 12R.
SEQ ID NO: 178 shows the nucleotide sequence of Primer 13F.
SEQ ID NO: 179 shows the nucleotide sequence of Primer 13R.
SEQ ID NO: 180 shows the nucleotide sequence of Primer 14F.
SEQ ID NO: 181 shows the nucleotide sequence of Primer 14R.
SEQ ID NO: 182 shows the nucleotide sequence of Primer 15F.
SEQ ID NO: 183 shows the nucleotide sequence of Primer 16F.
SEQ ID NO: 184 shows the nucleotide sequence of Primer 16R.
SEQ ID NO: 185 shows the nucleotide sequence of Primer 17F.
SEQ ID NO: 186 shows the nucleotide sequence of Primer 17R.
SEQ ID NO: 187 shows the nucleotide sequence of the guide RNA contained in the pRed_Cas9_recA_ΔybiW plasmid.
SEQ ID NO: 188 shows the nucleotide sequence of the guide RNA contained in the pRed_Cas9_recA_ΔpflD plasmid.
SEQ ID NO: 189 shows the nucleotide sequence of Primer 18F.
SEQ ID NO: 190 shows the nucleotide sequence of Primer 18R.
SEQ ID NO: 191 shows the nucleotide sequence of Primer 19F.
SEQ ID NO: 192 shows the nucleotide sequence of Primer 19R.
SEQ ID NO:193 shows the nucleotide sequence of primer 20F used to construct the α-1,4-glucan lyase.
SEQ ID NO:194 shows the nucleotide sequence of primer 20R used to construct the α-1,4-glucan lyase.
SEQ ID NO:195 shows the nucleotide sequence of primer 21F used to construct the α-1,4-glucan lyase.
SEQ ID NO:196 shows the nucleotide sequence of primer 21R used to construct the α-1,4-glucan lyase.
SEQ ID NO:197 shows the nucleotide sequence of primer 22F used to construct HMT-Mafr.
SEQ ID NO:198 shows the nucleotide sequence of primer 22R used to construct HMT-Mafr.

### Detailed Description of the Invention

Through bioinformatics analysis, the inventors of the present disclosure discovered two new radical-dependent glycolytic pathway for the first times, both of which can produce 1,2-propanediol. Through heterologous expression and purification of related enzymes in these two new pathways, enzyme activity was verified by LC-MS detection, and enzyme kinetic parameters were characterized, and the catalytic mechanism of key enzymes (e.g., 1,5-anhydroglucitol-6-phosphate isomerase or 1,5-anhydromannitol-6-phosphate isomerase) was explored by combining with protein crystallography analysis.

The inventors first discovered that, when using 1,5-anhydroglucitol and 1,5-anhydromannitol each as a sole carbon source, related pathway proteins were highly expressed in *E. coli* cells. Knockout of key genes (e.g., genes encoding 1,5-anhydroglucitol-6-phosphate isomerase or 1,5-anhydromannitol-6-phosphate isomerase) prevented *E. coli* from growing when using 1,5-anhydroglucitol or 1,5-anhydromannitol as the sole carbon source, indicating that *E. coli* can utilize 1,5-anhydroglucitol and 1,5-anhydromannitol for energy production and growth.

The inventors found that using each of starch and maltose as a substrate, α-1,4-glucan lyase could catalyze the production of 1,5-anhydrofructose (1,5-AF) from the substrate; adding Gafr and NADPH to this reaction system produced 1,5-anhydroglucitol (1,5-AG); further adding FDH and sodium formate significantly increased the yield of 1,5-AG. This indicated that the production of 1,5-AG from starch could be achieved through catalysis by these enzymes. The inventors also found that using each of starch and maltose as a substrate, adding Mafr and NADPH to the reaction system of α-1,4-glucan lyase produced 1,5-anhydromannitol (1,5-AM); further adding FDH and sodium formate significantly increased the yield of 1,5-AM. This indicated that the production of 1,5-AM from starch could be achieved through catalysis by these enzymes.

Then, the inventors tested the activities of enzymes such as α-1,4-glucan lyase, 1,5-anhydro-D-fructose reductase, and formate dehydrogenase (FDH) in a medium using soluble starch as the sole carbon source ("starch medium"), and found that the corresponding products could still be produced in the medium containing these enzymes. Through co-culture experiments of enzymes and cells, it was found that when α-1,4-glucan lyase, Gafr, FDH, NADPH, and sodium formate were added to the "starch medium", *E. coli* MG1655 showed significant growth; when α-1,4-glucan lyase, MBP-Mafr, FDH, NADPH, and sodium formate were added to the "starch medium", *E. coli* MG1655 also showed significant growth. SDS-PAGE results indicated that pathway proteins were induced to be highly expressed, and GC analysis showed the production of 1,2-propanediol in the fermentation broth, indicating that the production of 1,2-propanediol from starch could be achieved through co-culture of in vitro enzyme reactions and bacterial strains.

The two pathways discovered by the inventors, namely starch → 1,5-AG → 1,2-propanediol and starch → 1,5-AM → 1,2-propanediol, are new glycolytic pathways discovered since the 1920s to 1950s when EMP, ED and the pentose phosphate pathway were identified. Both of these new pathways can produce 1,2-propanediol.

Unless otherwise indicated, terms used in this disclosure have the meanings commonly understood by those skilled in the art.

As used herein, the term "NAD(P)⁺" means NAD⁺ or NADP⁺, and the term "NAD(P)H" means NADH or NADPH. As used herein, the term "active centor of an enzyme" refers to a region of an enzyme molecule that directly binds to a substrate molecule and catalyzes a chemical reaction of the substrate, and this region constitutes the active centor of the enzyme.

As used herein, the term "amino acid" refers to a compound in which a hydrogen atom on the carboxyl carbon atom is replaced by an amino group, and an amino acid molecule contains both an amino group and a carboxyl group. The amino acid includes natural and unnatural amino acids, as well as amino acid analogs and mimetics. natural amino acids include 20 types of (*L*)-amino acids used in protein biosynthesis, as well as other amino acids, such as 4-hydroxyproline, hydroxylysine, carboxylated lysine, desmosine, isodesmosine, homocysteine, citrulline, and ornithine. Unnatural amino acids include, for example, (*D*)-amino acids, norleucine, norvaline, p-fluorophenylalanine, ethionine, etc., as are known to those skilled in the art. Amino acid analogs include modified forms of natural and unnatural amino acids. Such modifications may include, for example, the substitution of chemical groups or moieties on the amino acid, or derivatization of the amino acid. Amino acid mimetics include, for example, organic structures that exhibit functionally similar properties, such as the charge and charge spatial characteristics of an amino acid. For example, an organic structure that mimics arginine (Arg or R) has a positively charged moiety located in a similar molecular space and having a degree of mobility similar to the ε-amino group of the side chain of a natural Arg amino acid. Mimetics also include constrained structures to maintain optimal spatial and charge interactions of amino acids or amino acid functional groups. A person skilled in the art is able to determining what structure constitutes a functionally equivalent amino acid analog or amino acid mimetic.

As used herein, the term "isozyme" refers to an enzyme that catalyzes the same reaction in an organism but has a different molecular structure.

As used herein, the term "full-length sequence" with respect to a particular polynucleotide or the protein it encodes means the entire nucleic acid sequence or the entire amino acid sequence of the natural (non-synthetic) endogenous sequence. A full-length polynucleotide encodes the full-length, catalytically active form of the particular protein. As used herein, the term "sugar kinase" refers to an enzyme that catalyzes the transfer of a phosphate group from a high-energy donor molecule to a specific substrate (sugar). Sugar kinases mentioned herein include 1,5-anhydroglucitol kinase and/or 1,5-anhydromannitol kinase.

In specific embodiments, the sugar kinase used herein is from *Thermococcus litoralis*, with Uniprot number: Q7M537, belonging to EC:2.7.1.147, which catalyzes the production of 1,5-anhydroglucitol-6-phosphate from 1,5-anhydroglucitol.

As used herein, the term "bacterial phosphoenolpyruvate-phosphotransferase system (PTS)" refers to an enzyme complex widely present in bacteria, fungi, and some archaea. PTS consists of cytoplasmic enzyme I (EI) or histidine phosphocarrier protein (HPr or NPr) and phosphotransferases, such as sugar-specific enzyme II complexes, and has both catalytic transport functions and a wide range of regulatory functions. The bacterial phosphoenolpyruvate-phosphotransferase system primarily phosphorylates various sugars and their derivatives through a phosphorylation cascade reaction, followed by transport them into a cell. All PTSs depend on cytoplasmic enzyme I (EI) and the histidine phosphocarrier protein (HPr), HPr phosphorylates the sugar via a sugar-specific EII complex, which is composed of two cytoplasmic domains (EIIA and EIIB) and one or two membrane domains (EIIC or EIID). The EIIC or EIID domain is associated with the membrane and transfers the sugar into the cytoplasm, where the sugar undergoes a multi-stage phosphorylation process involving the EIIA and EIIB domains. The phosphate group of phosphoenolpyruvate is transferred from EI to HPr, then from HPr to EIIA, then from EIIA to EIIB, and then to the sugar transported by EIIC or EIID. Most bacteria use the phosphoenolpyruvate-phosphotransferase system (PTS) to transport sugars such as glucose. EI catalyzes the phosphotransfer reaction from the PEP to HPr. HPr then transfers the phosphate group to various EIIA proteins, and then to EIIB. Finally, the carbohydrate is transported across the membrane through EIIC and EIID while being phosphorylated by EIIB.

As used herein, the term "1,5-anhydroalditol-6-phosphate isomerase" includes 1,5-anhydroglucitol-6-phosphate isomerase, which catalyzes the conversion of 1,5-anhydroglucitol-6-phosphate to 1-deoxyfructose-6-phosphate; and 1,5-anhydromannitol-6-phosphate isomerase, which catalyzes the conversion of 1,5-anhydromannitol-6-phosphate to 1-deoxyfructose-6-phosphate.

In some embodiments, the 1,5-anhydroalditol-6-phosphate isomerase is 1,5-anhydroglucitol-6-phosphate isomerase YbiW and 1,5-anhydromannitol-6-phosphate isomerase PflD.

In some embodiments, the 1,5-anhydroalditol-6-phosphate isomerase includes the amino acid sequence shown in SEQ ID NO:1 or a functional variant thereof. In some embodiments, the functional variant is a natural isoenzyme of the amino acid sequence shown in SEQ ID NO:1.

In some specific embodiments, the natural isozyme of the amino acid sequence shown in SEQ ID NO: 1 is derived from: *Lactiplantibacillus plantarum*, *Escherichia coli*, *Lactobacillus selangorensis*, *Streptococcus uberis*, *Olsenella sp.*, *Leptotrichia wadei*, *Streptococcus parauberis*, *Anaerostipes hadrus*, *Senella sp.*, *Selenomonas ruminantium*, *Orenia metallireducens*, *Bifidobacterium primatium*, *Leptotrichia hofstadii*, *Ligilactobacillus agilis*, *Senella profusa*, *Clostridium butyricum*, *Vibrio ishigakensis*, *Coriobacteriaceae*, *Coriobacterium glomerans*, *Eggerthia cateniformis*, *Clostridium baratii str. Sullivan*, *Clostridiales, Firmicutes, Clostridium vincentii, Streptococcus downei, Erysipelotrichaceae, Quinella sp., Sebaldella termitidis, Ligilactobacillus animalis, Liquorilactobacillus satsumensis, Clostridium botulinum, Acetivibrio ethanolgignens, Aeromonas sobria, Clostridium cadaveris, Streptococcus bovimastitidis, Sodalis ligni, Lactococcus raffinolactis, Lactobacillus ultunensis, Leptotrichia sp., Lucifera butyrica, Fonticella tunisiensis, Cronobacter sakazakii, Liquorilactobacillus uvarum, Anaeromassilibacillus sp., Anaerotruncus sp., Beauveria bassiana, Cedecea lapagei, Streptococcus pneumoniae, Streptococcus suis, Aeromonas veronii, Lactococcus raffinolactis, Streptococcus sanguinis, Actinomyces succiniciruminis, Vagococcus humatus, Vibrio alginolyticus, Ligilactobacillus acidipiscis, Pilibacter termitis, Lachnospiraceae, Aeromonas hydrophila, Enterococcus ratti, Kandleria vitulina, Clostridium pasteurianum, Aeromonas allosaccharophila, Pantoea alhagi, Peptacetobacter hiranonis, Edwardsiella piscicida, Cedecea neteri, Chelonobacter oris, Aeromonas sp., Clostridium sp., Vibrio gazogenes, Melissococcus plutonius, Tolumonas sp., Loigolactobacillus coryniformis, Zooshikella ganghwensis, Vibrio sp., Photobacterium lipolyticum, Agrilactobacillus composti, Clostridium symbiosum*, or *Aerococcus sp..*

In some specific embodiments, the natural isoenzymes of the amino acid sequence shown in SEQ ID NO:1 include the amino acid sequence shown in any one of SEQ ID NOs:2-96.

In some embodiments, a polypeptide as shown in any one of SEQ ID NOs:1-96 possesses 1,5-anhydroglucitol-6-phosphate isomerase activity, and the substrate is 1,5-anhydroglucitol-6-phosphate.

In some specific embodiments, when the polypeptide possesses 1,5-anhydroglucitol-6-phosphate isomerase activity, it isomerizes 1,5-anhydroglucitol-6-phosphate, and the product formed is 1-deoxyfructose-6-phosphate.

In some embodiments, the 1,5-anhydroalditol-6-phosphate isomerase includes the amino acid sequence shown in SEQ ID NO:97 or a functional variant thereof, wherein the functional variant is a natural isoenzyme of the amino acid sequence shown in SEQ ID NO:97.

In some embodiments, the natural isozyme of the amino acid sequence shown in SEQ ID NO: 97 is derived from: *Escherichia coli, Streptococcus dysgalactiae subsp. equisimilis, Firmicutes, Pseudoleptotrichia goodfellowii, Edwardsiella tarda, Serratia odorifera, Gilliamella apicola, Vibrio sp., Lactobacillus selangorensis, Caloranaerobacter sp., Pilibacter termitis, Caloranaerobacter azorensis, Salmonella enteritidis, Tolumonas auensis, Senella porci, Coriobacteriaceae, Fonticella tunisiensis, Coriobacteriales, Bacillus sp., Actinobacillus rossii, Lactobacillus ultunensis, Clostridium estertheticum, Geosporobacter ferrireducens, Vibrio ishigakensis, Streptococcus merionis, Shigella boydii, Agrilactobacillus composti, Lactobacillus selangorensis, Vagococcus elongatus, Clostridium acidisoli, Caloramator quimbayensis, Clostridium uliginosum, Enterobacter asburiae, Atopobium minutum, Thermophilobacter immobilis, Photobacterium lipolyticum, Citrobacter amalonaticus, Clostridium pasteurianum, Sebaldella termitidis, Acididesulfobacillus acetoxydans,* or *Clostridium sp..*

In some specific embodiments, the natural isoenzymes of the amino acid sequence shown in SEQ ID NO:97 include the amino acid sequence shown in any one of SEQ ID NOs:98-144.

In some embodiments, a polypeptide as shown in any one of SEQ ID NOs:97-144 possesses 1,5-anhydromannitol-6-phosphate isomerase activity, and the substrate is 1,5-anhydromannitol-6-phosphate.

In some specific embodiments, when the polypeptide possesses 1,5-anhydromannitol-6-phosphate isomerase activity, it isomerizes 1,5-anhydromannitol-6-phosphate, and the product formed is 1-deoxyfructose-6-phosphate.

In some embodiments, the functional variant is generated by one or more amino acid insertions, substitutions, and/or deletions in the amino acid sequence shown in SEQ ID NO: 1 or 97, or a natural isozyme thereof, where the insertions, substitutions, and/or deletions do not occur in the active site.

In some embodiments, the number of amino acid insertions, substitutions and/or deletions is 1-30, preferably 1-20, more preferably 1-10, where an obtained functional variant is basically remaining the 1,5-anhydroalditol-6-phosphate isomerase (e.g., 1,5-anhydroglucitol-6-phosphate isomerase or 1,5-anhydromannitol-6-phosphate isomerase) activity unchanged.

In some embodiments , the functional variant differs from the amino acid sequence shown in SEQ ID NO: 1 or 97 by about 1, 2, 3, 4, 5, 6, 7, 8, 9, or 10 amino acid insertions, substitutions, and/or deletions.

In some embodiments, the polypeptide is an isolated polypeptide.

In some embodiments, the polypeptide belongs to the glycine radical enzyme (GRE) family. When the polypeptide possesses 1,5-anhydroglucitol-6-phosphate isomerase or 1,5-anhydromannitol-6-phosphate isomerase activity, its catalysis involves glycine and cysteine radicals.

The nucleic acid molecules of the present disclosure can be combined with other DNA sequences, such as promoters, polyadenylation signals, other restriction sites, multiple cloning sites, other coding segments, etc., so that their total length can vary significantly. It is therefore considered that polynucleotide fragments of almost any length can be utilized; the total length is preferably limited by the ease of preparation and use in the intended recombinant DNA protocol.

Any one of a variety of mature technologies known and available in the art can be used to prepare, manipulate, and/or express polynucleotides and their fusions. Methods known in the art can be used to modify the nucleic acid molecules of the present disclosure, including but not limited to the cloning, processing, expression and/or activity modification of gene products.

In some embodiments, the nucleic acid molecule is produced by artificial synthesis, such as direct chemical synthesis or enzymatic synthesis.

In some embodiments, the nucleic acid molecule is produced by recombinant technology.

In some embodiments, the nucleic acid molecule is an isolated nucleic acid molecule.

The present disclosure provides an expression cassette, including the nucleic acid molecule.

In some specific embodiments, the expression cassette may additionally include a 5'leader sequence, which can play a role in enhancing translation.

When preparing the expression cassette, various DNA fragments can be manipulated to provide the DNA sequence in the proper orientation and in the proper reading frame when appropriate. To achieve this goal, adaptors or linkers can be used to connect DNA fragments, or other operations can be involved to provide convenient restriction sites, remove excess DNA, remove restriction sites, and so on. For this purpose, it may involve in vitro mutagenesis, primer repair, restriction, annealing, and replacement, such as transition and transversion.

The present disclosure provides an expression vector, including the above nucleic acid molecule.

Any suitable expression vector can be used in the present disclosure. For example, the expression vector can be a vector suitable for a system of *Escherichia coli.* in the present disclosure. In some embodiments, the expression vector can be any one of the vectors such as HT or pACYC.

In some embodiments, the nucleic acid molecule encoding the polypeptide shown in any one of SEQ ID NOs: 1-96 or the nucleic acid molecule encoding the polypeptide shown in any one of SEQ ID NOs: 97-144 is cloned into a vector to form a recombinant vector containing the nucleic acid molecules of the present disclosure.

In some embodiments, the expression vector used to clone the polynucleotide is a plasmid vector.

In some embodiments, the above-mentioned expression vector further includes a control sequence for regulating the expression of the nucleic acid molecule, where the nucleic acid molecule is operably linked to the control sequence. In some embodiments, methods well known to those skilled in the art are used to construct a nucleotide sequence that encodes the polypeptide shown in any one of SEQ ID NOs: 1-96 or a nucleotide sequence that encodes the polypeptide shown in any one of SEQ ID NOs: 97-144 and suitable expression vector for transcription/translation regulatory elements.

The present disclosure also provides a cell, including the above-described nucleic acid molecule, the expression cassette, or the expression vector. In specific embodiments, the cell is an *E. coli* cell, such as *E. coli* MG1655 cell. In some embodiments, the cell is capable of expressing and producing a 1,5-anhydroalditol-6-phosphate isomerase, including an amino acid sequence as shown in any one of SEQ ID NOs:1-144.

In some embodiments, the cell is capable of expressing and producing a 1,5-anhydroglucitol-6-phosphate isomerase, including an amino acid sequence as shown in any one of SEQ ID NOs:1-96.

In some embodiments, the cell is capable of expressing and producing a 1,5-anhydromannitol-6-phosphate isomerase, including an amino acid sequence as shown in any one of SEQ ID NOs:97-144.

In some embodiments, the cell further expresses an activator, an aldolase, a hydroxyacetone reductase, and/or a transport complex.

In specific embodiments, the activator is an S-adenosylmethionine radical enzyme family glycine radical enzyme activating enzyme (Enzyme classification number EC:1.97.1.4). In specific embodiments, the aldolase is 1-deoxyfructose-6-phosphate aldolase (also known as fructose-6-phosphate aldolase, enzyme classification number EC:4.1.2). In specific embodiments, the hydroxyacetone reductase is also known as glycerol dehydrogenase, enzyme classification number EC:1.1.1.6).

In some specific embodiments, the S-adenosylmethionine radical enzyme family glycine radical enzyme activating enzyme is an activating enzyme for the 1,5-anhydroalditol-6-phosphate isomerase. In some embodiments, the S-adenosylmethionine radical enzyme family glycine radical enzyme activating enzyme includes an [Fe-S] cluster. In some embodiments, the S-adenosylmethionine radical enzyme family glycine radical enzyme activating enzyme includes a [4Fe-4S] cluster, i.e., includes 4 Fe and 4 S. In some embodiments, the S-adenosylmethionine radical enzyme family glycine radical enzyme activating enzyme catalyzes the cleavage of S-adenosylmethionine (SAM). In some specific embodiments, the transport complex includes SEQ ID NOs:150-153 or functional variants thereof, and the transport complex possesses phosphoenolpyruvate-dependent phosphotransferase system transport activity. In some embodiments, SEQ ID NOs:150-153 represent amino acid sequences of polyphosphate transfer protein (PtsA), PTS system fructose-like EIIB component 2 (FrwB), PTS system fructose-like EIIC component 2 (FrwC), and PTS system fructose-like EIIB component 3 (FrwD), respectively.

In some embodiments, the four sequences shown in SEQ ID NOs:150-153 constitute the phosphoenolpyruvate-sugar phosphotransferase system, where PtsA contains EI and EIIA domains, FrwC is EIIC, and FrwB and FrwD are EIIB. In some specific embodiments, PtsA, FrwB, FrwC, and FrwD interact to form a transport complex that exerts phosphoenolpyruvate-dependent phosphotransferase system transport activity. In some embodiments, the transport complex transports 1,5-anhydroalditol (e.g., 1,5-anhydroglucitol, 1,5-anhydromannitol) into the host cell and phosphorylates it (e.g., to yield 1,5-anhydroglucitol-6-phosphate, 1,5-anhydromannitol-6-phosphate) for subsequent glycolysis.

In some embodiments, the functional variant of any one of SEQ ID NOs:150-153 differs from the amino acid sequence shown in the corresponding SEQ ID NO:150-153 by about 1, 2, 3, 4, 5, 6, 7, 8, 9, or 10 amino acid insertions, substitutions, and/or deletions.

In some embodiments, usable host cells are cells containing the above-described expression vector, which may be eukaryotic cells, e.g., yeast cell culture systems can be used for expression of the polypeptides of the present disclosure. The host cells may also be prokaryotic cells containing the above-described expression vector, for example, selected from *Escherichia* (e.g., *E. coli*), *Klebsiella*, *Streptococcus*, *Lactobacillus*, *Bifidobacterium*, *Bacteroidetes*, and *Firmicutes*, etc.

In some specific embodiments, the host cell is a yeast cell or *E. coli*.

In some specific embodiments, the nucleic acid molecule encoding one or more enzymes of the present disclosure may exist in the host cell in the form of a free vector, or may be integrated into the genome of the host cell.

In some embodiments, the isolated nucleic acid is operably linked to regulatory sequences that can be recognized by the host cell transformed with the expression vector.

The expression vector can be introduced into the host cell using any technique known in the art, including transformation, transduction, transfection, viral infection, gene gun, or Ti-mediated gene transfer. As an example, when the host is a prokaryote such as *E. coli*, competent cells can be harvested after exponential growth phase and transformed using the CaCl₂ method well known in the art.

In some specific embodiments, 1,5-anhydroglucitol is transported into the host cell by a transport complex (e.g., a transport complex having phosphoenolpyruvate-phosphotransferase system transport activity) and phosphorylated (e.g., at C-6 position) to yield 1,5-anhydroglucitol-6-phosphate, 1,5-anhydroglucitol-6-phosphate is catalyzed by 1,5-anhydroglucitol-6-phosphate isomerase to yield 1-deoxyfructose-6-phosphate, 1-deoxyfructose-6-phosphate is cleaved by an aldolase (e.g., 1-deoxyfructose-6-phosphate aldolase) to yield hydroxyacetone and 3-phosphoglyceraldehyde, and hydroxyacetone is reduced by hydroxyacetone reductase to yield 1,2-propanediol.

In some specific embodiments, 1,5-anhydromannitol is transported into the host cell by a transport complex (e.g., a transport complex having phosphoenolpyruvate-phosphotransferase system transport activity) and phosphorylated (e.g., at C-6 position) to yield 1,5-anhydromannitol-6-phosphate, 1,5-anhydromannitol-6-phosphate is catalyzed by 1,5-anhydromannitol-6-phosphate isomerase to yield 1-deoxyfructose-6-phosphate, 1-deoxyfructose-6-phosphate is cleaved by an aldolase (e.g., 1-deoxyfructose-6-phosphate aldolase) to yield hydroxyacetone and 3-phosphoglyceraldehyde, and hydroxyacetone is reduced by hydroxyacetone reductase to yield 1,2-propanediol.

In some embodiments, the method for producing 1,2-propanediol described herein further includes steps of collecting a culture and purifying 1,2-propanediol.

### Examples

The following examples are illustrative only and are not intended to limit the scope of the embodiments of the present disclosure or the scope of the claims.

### Example 1. Identification, characterization, and testing of the YbiW-related pathway

### Materials and Methods

### Experimental Materials

Tryptone and yeast extract used to prepare LB medium were purchased from Oxoid Limited (Hampshire, UK). Ultrapure deionized water from a Millipore Direct-Q system was used. TALON resin was purchased from Clontech Laboratories Inc. (California, USA). 1,5-Anhydroglucitol (1,5-AG) was purchased from Shanghai Yuanye Bio-Technology Co., Ltd., and 1,5-anhydroglucitol-6-phosphate (1,5-AG-6P) was chemically synthesized by Tianjin Heowns Biochemical Technology Co., Ltd. Oligonucleotide primers were synthesized by Beijing Tsingke Biotech Co., Ltd. All protein purification chromatography experiments were performed on an ÄKTA FPLC system (GE Healthcare, USA). Anaerobic experiments were carried out in a Lab2000 glove box (Etelux) protected by N₂ (with an oxygen concentration of less than 5 ppm).

### Gene synthesis and cloning

Gene fragments of LpYbiW (Uniprot accession No.: A0A807DR53), LpYbiY (Uniprot accession No.: A0A162FGG2), and *E. coli* codon-optimized LpFsaA (Uniprot accession No.: A0A0M4CJJ6) were synthesized by Beijing Tsingke Biotech Co., Ltd. LpYbiW and LpFsaA were inserted into the SspI site of the HT plasmid (an optimized pET28 vector) to express proteins with an N-terminal His₆ tag. LpYbiY was inserted into the NdeI site of the pACYC-MBP vector to express a protein with an N-terminal His₆ tag and maltose-binding protein (MBP).

For biochemical characterization purposes, EcYbiW (Uniprot accession No.: P75793) and EcYbiY (Uniprot accession No.: P75794) were obtained by amplifying the *E. coli* MG1655 genome using primers 18F/18R and 19F/19R (Table 6). EcYbiW was inserted into the SspI site of the HT plasmid (an optimized pET28 vector) to express a protein with an N-terminal His₆ tag. EcYbiY was inserted into the NdeI site of the pACYC-MBP vector to express a protein with an N-terminal His₆ tag and maltose-binding protein (MBP).

### Expression and Purification of LpYbiW, LpYbiY, and LpFsaA

HT-LpYbiW, pACYC-MBP-LpYbiY, and HT-LpFsaA plasmids were transformed into *E. coli* BL21(DE3) cells for expression of the corresponding proteins. LB agar plates containing 50 µg/mL kanamycin were used to screen for LpYbiW and LpFsaA, while LB agar plates containing 25 µg/mL chloramphenicol were used to screen for LpYbiY positive clones. Cells were cultured overnight in 4 mL of LB medium, then transferred to fresh LB medium (typically 1 L medium in a 2.6 L flask) and grown in a shaking incubator at 37°C, 220 rpm. When OD₆₀₀ reached approximately 0.8, the temperature was reduced to 18°C, and isopropyl β-D-1-thiogalactopyranoside (IPTG) was added to a final concentration of 0.3 mM to induce production of the target protein. After 16-20 hours, cells were collected by centrifugation (8000 ×g, 10 minutes at 4°C). The harvested cells were resuspended in 40 mL of lysis buffer (50 mM Tris/HCl, pH 8.0, 100 mM KCl, 1 mM phenylmethylsulfonyl fluoride (PMSF), 0.2 mg/mL lysozyme, 0.03% Triton X-100, and 0.02 mg/mL DNase I) and frozen in a refrigerator at -80°C.

The frozen cells were thawed and incubated at room temperature (RT, 25°C) for 20 minutes, during which cell lysis occurred. 5 mM β-mercaptoethanol (BME) was added, and nucleic acids were precipitated by adding 1% streptomycin sulfate. Cell debris was removed by centrifugation at 10,000×g for 10 minutes at 4°C. For LpYbiW and LpFsaA, the supernatant was filtered through a 0.22 µm filter and loaded onto a 5 mL TALON Co²⁺ column (Takara Bio USA, Inc.) pre-equilibrated with buffer A (20 mM Tris/HCl, pH 7.5, 200 mM KCl, and 5 mM BME). The column was washed with 10 times column volumes of buffer A to remove contaminating proteins, and the protein was eluted with 5 times column volumes of buffer A containing 150 mM imidazole. The eluted protein (about 20 mL) was dialyzed against 2 L of buffer A for 3 hours at 4°C, concentrated, aliquoted, frozen in liquid nitrogen, and stored at -80°C. For LpYbiY, the supernatant was filtered and loaded onto a column packed with 10 mL of amylose resin (New England Biolabs, Massachusetts, U.S.A.). The column was washed with 10 times column volumes of buffer A to remove contaminating proteins, and the target protein was eluted with buffer A containing 10 mM maltose.

Purified proteins were detected by SDS-PAGE using commercial gels (SurePAGE, Bis-Tris, 4-20%). Protein concentrations were calculated by measuring absorbance at 280 nm using an ultra-micro UV-visible spectrophotometer (Hangzhou Miou Instruments Co., Ltd.) [LpYbiW (*ε*₂₈₀ = 104,630 M⁻¹ cm⁻¹), MBP-LpYbiY (*ε*₂₈₀ = 100,730 M⁻¹ cm⁻¹), LpFsaA (*ε*₂₈₀ = 20,400 M⁻¹ cm⁻¹)].

### Expression and purification of EcYbiW and EcYbiY for biochemical characterization

The protein expression and purification methods and procedures, SDS-PAGE detection method, and concentration determination method were the same as those described above for LpYbiW and LpYbiY. [EcYbiW (ε₂₈₀ = 99,700 M⁻¹ cm⁻¹), MBP-EcYbiY (ε₂₈₀ = 97,750 M⁻¹ cm⁻¹)].

### Reconstitution and characterization of the LpYbiY prosthetic [Fe-S] cluster

Sequence alignment indicated that LpYbiY contains only one [4Fe-4S] cluster in the radical SAM domain, with a theoretical maximum of 4 Fe and 4 S per monomer. The LpYbiY protein solution was degassed with argon to remove oxygen and then transferred into a glove box. To the degassed protein solution, final concentrations of 100 mM Tris/HCl, (pH 7.5), 10 mM DTT, 4 equivalents of ferrous ammonium sulfate and sodium sulfide were added, and a resulting mixture was incubated overnight in a metal bath at 4°C (Dry Bath H2O3-100C; Coyote Bioscience, Beijing, China). Then, 4 equivalents of EDTA solution were added, followed by repeated concentration using centrifugal filter units (1.5 mL Ym-30 Amicon; Millipore) and buffer exchange by dilution with a buffer (20 mM Tris-HCl, pH 7.5, and 100 mM KCl).

Fe contents of unreconstituted and reconstituted LpYbiY were determined using ferrozine [3-(2-pyridyl)-5,6-diphenyl-1,2,4-triazine-p,p'-disulfonic acid monosodium salt]. An AAS iron standard was used to establish a standard curve for Fe in a range of 0-600 µM. The sulfur contents of unreconstituted and reconstituted LpYbiY were determined by measuring the absorbance of methylene blue formed by reaction with N,N-dimethyl-p-phenylenediamine dihydrochloride (DPD). A standard curve for S in the range of 0-600 µM was established using Na₂S.

UV-Vis absorption spectra of LpYbiY in a range of 200-800 nm were measured using a Nanophotometer NP80 Mobile (Germany). Unreconstituted and reconstituted LpYbiY were diluted to 10 µM with a buffer containing 20 mM Tris/HCl, (pH 7.5), 100 mM KCl, and transferred to a septum-sealed anaerobic cuvette for measurement. To the reconstituted LpYbiY solution, 10 equivalents of titanium(III) citrate were added and incubated for 10 minutes to measure its reduced form.

### LC-MS analysis of S-adenosylmethionine (SAM) cleavage catalyzed by LpYbiY

A reaction system (500 µL) containing 20 mM Tris/HCl, (pH 7.5), 100 mM KCl, 200 µM titanium(III) citrate, 20 µM reconstituted LpYbiY, and 1 mM SAM was incubated overnight at room temperature in a glove box. Titanium(III) citrate was not added to the negative control group. The reaction was quenched by adding formic acid (5% v/v final concentration), and a resulting mixture was incubated in a boiling water bath for 1 minute to denature the protein. Precipitated protein was removed by centrifugation at 14,000×g for 15 minutes. The supernatant was filtered through a 0.22 µm PES membrane and subjected to LC-MS analysis. The method was as follows: 20 µL of sample was loaded onto an Agilent 6420 Triple Quadrupole LC/MS instrument (Agilent Technologies) equipped with a C18 reverse-phase column. UV absorption was detected at 257 nm. The mobile phase system consisted of water (A) and acetonitrile (B). Linear gradient elution was performed from 0 to 16% B over 30 minutes at a flow rate of 0.5 mL/min. Commercial 5'-dA was used as a standard, and the formation of the SAM cleavage product catalyzed by LpYbiY was verified by mass spectrometry.

### Detection of LpYbiW radical generation by electron paramagnetic resonance (EPR) spectroscopy

Continuous-wave X-band EPR spectroscopy was used to characterize the LpYbiW glycyl radical. A reaction system (200 µL) containing 20 mM Tris/HCl, (pH 7.5), 100 mM KCl, 100 µM titanium(III) citrate, 1 mM SAM, 80 µM reconstituted LpYbiY, and 40 µM LpYbiW was incubated for 15 minutes at room temperature in a glove box. 10% glycerol was added, and a resulting mixture was loaded into an EPR tube (Wilmad Lab Glass, 734-LPV-7) with an outer diameter of 4 mm and a length of 8 inches. The tube was sealed with a rubber stopper, removed from the glove box, and frozen using liquid nitrogen before EPR analysis. The experimental spectrum of the glycyl radical was modeled using Bruker Xepr spin fitting to obtain g-values, hyperfine coupling constants, and line widths. Double integration of the simulated spectrum was used to measure the spin concentration. EPR spectra were obtained by accumulating 30 scans under the following conditions: temperature, 90 K; center field, 3370.00 G; sweep width, 200 G; microwave power, 10 µW; microwave frequency, 9.43 MHz; modulation amplitude, 0.5 mT; modulation frequency, 100 kHz; time constant, 20.48 ms; conversion time, 25 ms; sweep time, 20 seconds; receiver gain, 43 dB.

### LC-MS analysis for LpYbiW activity determination

LpYbiW was activated as described for the EPR experiment above, except that glycerol was not added. A 200 µL reaction mixture containing 10 µM activated LpYbiW, 0.1 mM titanium(III) citrate, and 10 mM 1,5-AG-6P was incubated at room temperature for 1 hour in a glove box. In negative controls, 1,5-AG-6P, SAM, or activated LpYbiW was omitted. 200 µL of acetonitrile was added to the reaction system to precipitate protein, the precipitate was removed by centrifugation, and the supernatant was filtered through a 0.22 µm PES membrane for LC-MS analysis.

LC-MS analysis was performed on an Agilent 6420 Triple Quadrupole LC/MS instrument (Agilent Technologies). The drying gas was maintained at a temperature of 300°C, with a flow rate of 9 L/min and a nebulizer pressure of 15 psi. LC-MS analysis was performed using a ZIC-HILIC column (5 µm, 150 × 4.6 mm; Merck). HPLC conditions were as follows: mobile phase A was 90% 20 mM ammonium acetate and 10% acetonitrile, mobile phase B was acetonitrile; gradient elution from 90% B to 70% B over 10 minutes, and from 70% B to 50% B over 20 minutes. The flow rate was set to 0.5 mL/min. The mass spectrometer was operated in ESI negative ion mode.

### LC-MS analysis for EcYbiW activity determination

The LC-MS analysis experiment for EcYbiW activity determination was performed using the same method as that for LpYbiW activity determination.

### LC-MS analysis of LpFsaA coupled with LpYbiW activity

A reaction mixture (200 µL) containing 10 µM activated LpYbiW, 0.1 mM titanium(III) citrate, and 10 mM 1,5-AG-6P was incubated for 1 hour at room temperature in a glove box, the LpFsaA coupled LpYbiW enzyme activity assay was performed. 10 µM LpFsaA was added to the above reaction system, and after an additional 1 hour of reaction in the glove box, 50 µL of the reaction sample was mixed with 550 µL of 0.73 M sodium acetate (pH 5.0), then mixed with 400 µL of freshly prepared 2,4-dinitrophenylhydrazine (DNPH) solution (20 mg DNPH dissolved in 50 mL methanol), followed by incubation at 50°C for 1 hour. A resulting mixture was centrifuged at 13,000×g for 5 minutes and filtered before LC-MS analysis. Product standards were prepared by treating 3-phosphoglyceraldehyde (5 mM, Sigma-Aldrich) and hydroxyacetone (2 mM, TCI) with sodium acetate (pH 5.0) and freshly prepared DNPH for 1 hour in the same manner.

LC-MS analysis was performed using 20 µL of sample on an Agilent ZORBAX SB-C18 reverse-phase column (4.6 × 250 mm) in ESI negative ion mode. The solvent system consisted of solvent C (deionized ultrapure water containing 0.1% formic acid) and D (chromatographic acetonitrile containing 0.1% formic acid). Liquid phase analysis was performed according to the following program: elution from 15% to 100% D over 15 minutes at a flow rate of 1.0 mL/min, and the detection wavelength was set to 360 nm.

### LC-MS analysis of LpFsaA activity

The activity of LpFsaA catalyzing the aldol condensation reaction of hydroxyacetone and 3-phosphoglyceraldehyde was also determined. A 200 µL reaction system containing 50 mM Tris/HCl, (pH 8.0), 100 mM KCl, 5 mM 3-phosphoglyceraldehyde, 10 mM hydroxyacetone, and 10 µM LpFsaA was incubated at room temperature for 1 hour. In negative controls, 3-phosphoglyceraldehyde, hydroxyacetone, or LpFsaA was omitted. LC-MS analysis was performed using the same sample preparation method and elution conditions as described for the LpYbiW activity determination, using a ZIC-HILIC column.

### Determination of Michaelis-Menten Kinetic Parameters of LpYbiW

The Michaelis-Menten kinetic parameters of LpYbiW were determined by coupling with LpFsaA and EcGldA. The reaction rate of LpYbiW was monitored by measuring NADH consumption at A₃₄₀ₙₘ. The absorbance change of the reaction system at 340 nm was measured every 5 seconds using the cuvette mode of an ultra-micro UV-visible spectrophotometer (Nanophotometer) in a glove box (1 cm light path). These reaction systems consisted of 100 nM activated LpYbiW, 20 mM Tris/HCl, (pH 7.5), 100 mM KCl, 1,5-anhydroglucitol-6-phosphate with varying concentrations, 5 µM LpFsaA, 5 µM EcGldA, and 0.4 mM NADH.

### Construction of expression plasmid for EcYbiW for crystallographic studies

The *E. coli* gene encoding YbiW (EcYbiW, Uniprot accession No.: P75793) was amplified by PCR using a primer pair 5F/5R and inserted into the NdeI site of the pACYC vector. Site-directed mutagenesis of plasmid pACYC-EcYbiW was then performed using a primer pair 6F/6R to obtain plasmid pACYC-EcYbiW (E114A, E115A, and K117A, indicating that amino acids at positions 114, 115, and 117 were mutated from glutamic acid (E) and lysine (K) to alanine (A), respectively) in order to express a surface entropy reduction mutant of EcYbiW and increase the success rate of protein crystallization screening.

### Expression and purification of EcYbiW for crystallographic studies

The plasmid pACYC-EcYbiW (E114A, E115A, and K117A) was transformed into *E. coli* BL21(DE3) cells, and positive clones were selected using LB agar plates containing 25 µg/mL chloramphenicol. A single colony was cultured overnight in 4 mL of LB medium, then transferred to 1 L of fresh LB medium and grown at 37°C, 220 rpm until OD₆₀₀ reached approximately 0.8. Expression was induced with 0.3 mM IPTG at 18°C for 16 hours. Cells from the 1 L culture were collected by centrifugation (8000×g, 10 minutes) and resuspended in 40 mL of lysis buffer (50 mM Tris/HCl, pH 8.0, 100 mM KCl, 0.03% Triton X-100). Cells were lysed by sonication, and insoluble cell debris was removed by centrifugation at 10,000×g for 10 minutes at 4°C. The supernatant was filtered through a 0.45 µm filter and loaded onto a 10 mL TALON Co²⁺ column, then washed with 10 times column volumes of buffer A (20 mM Tris/HCl, pH 7.5, 200 mM KCl, and 5 mM BME), and eluted with 5 times column volumes of buffer A containing 150 mM imidazole. The eluate was dialyzed against 2 L of buffer B (20 mM Tris/HCl, pH 7.5, 5 mM BME) for 3 hours, then loaded onto a 10 mL DEAE column and eluted with a linear gradient of 0 to 500 mM KCl in buffer B over 30 times column volumes. Fractions containing EcYbiW were collected and concentrated by ultrafiltration to approximately 5 mL. The protein solution was then injected into a Superdex 200 gel filtration column (300 mL) and eluted with buffer C (20 mM Tris/HCl, pH 7.5, 100 mM KCl, 1 mM DTT). The eluate from the gel filtration column was re-concentrated to 13.3 mg/mL for crystallographic studies.

### Crystallization, data collection, and structure determination of EcYbiW

Initial screening for EcYbiW crystals was performed using the sitting-drop vapor diffusion method in a 96-well format with an automated liquid handling robot system (Gryphon, Art Robbins). Screening was carried out at 291 K using various crystal screening kits from Hampton Research and Molecular Dimensions. After further optimization using the hanging-drop vapor diffusion method in 24-well plates, crystals suitable for single-crystal X-ray diffraction studies were obtained. The optimal conditions for producing block-like crystals of EcYbiW were 0.2 M NaCl, 0.1 M Tris, pH 8.0, 25% PEG3350 plus 10 mM 1,5-AG-6P, with 6.7 mg/mL EcYbiW protein solution. Crystals were cryoprotected using a crystallization solution containing 15% glycerol and flash-cooled in liquid nitrogen. Diffraction data were collected and processed at beamline BL10U2 of the Shanghai Synchrotron Radiation Facility (SSRF) to a resolution of 2.65 Å. A crystal structure model created with the website PHYRE2 was used for molecular replacement in the PHENIX software. The structure was manually built according to the electron density using Coot software, further refined in PHENIX, and then deposited in the RCSB Protein Data Bank (accession code: 8ID7). The appendix table contains data collection and final refinement statistics (Table 1). All structure Fig.s were generated using UCSF Chimera (Fig. 4A and Fig. 4B).

### Identification of the active sitecenter of 1,5-anhydroglucitol-6-phosphate isomerase

The obtained PDB data of the EcYbiW complex with 1,5-AG-6P (with a resolution of 2.65 Å) were displayed using UCSF Chimera software. Focusing on the substrate 1,5-AG-6P, amino acid residues within 10 Å of the substrate center were selected for display. Based on the hydrogen bond distances between the substrate and amino acid residues, key amino acid residues in the active center of 1,5-anhydroglucitol-6-phosphate isomerase that bind to the substrate were identified. In combination with the catalytic mechanism of glycine radical 1,2-lyases described in the literature, the key amino acid residues of the active center of 1,5-anhydroglucitol-6-phosphate isomerase were determined.

### Identification of isozymes of 1,5-anhydroglucitol-6-phosphate isomerase

To identify isozymes of 1,5-anhydroglucitol-6-phosphate isomerase in the glycine radical enzyme (GRE) family, a sequence similarity network (SSN) of 25,347 unique sequences from InterPro family IPR004184 was constructed using the web-based Enzyme Function Initiative-Enzyme Similarity Tool (EFI-EST). The alignment score threshold was set to 250, the minimum sequence length was set to 650, and an 80% similarity representative node network (RepNode network) was displayed using Cytoscape v3.519. Under these settings, previously characterized GREs with different catalytic activities were separated into distinct clusters.

Sequences with 80% or higher similarity were represented by a single node, and sequences were folded to reduce the total number of nodes, making the less complex network easier to load in Cytoscape. For the cluster formed by YbiW, one representative sequence was selected from each node based on different nodes. Using the crystal structure of the complex formed by EcYbiW and the substrate, amino acid residues interacting with the substrate were used as conserved sites for multiple sequence alignment. Sequences that simultaneously contained these conserved sites were retained. Further analysis of neighboring genes of these sequences was performed, and sequences whose gene cluster contained an aldolase were retained. Finally, representative sequences were selected.

### Results, Analysis, and Discussion

During bioinformatics analysis of glycine radical enzyme (GRE) sequences in the UniProt database, the inventors of the present disclosure noted an uncharacterized GRE (YbiW) present in a gene cluster involved in sugar metabolism. In addition to YbiW and its activase (YbiY), this gene cluster also contains an aldolase (FsaA). This aldolase catalyzes the formation of corresponding 2-keto-hexose-6-phosphates from hydroxyacetone, 1,3-dihydroxyacetone, and glyceraldehyde-3-phosphate. Accordingly, the inventors of the present disclosure speculated that this GRE is involved in sugar metabolism and that its substrate might be a hexose phosphate. Based on molecular docking results, the inventors of the present disclosure hypothesized that the substrate of YbiW is 1,5-anhydroglucitol-6-phosphate (1,5-AG-6P) and proposed a novel radical-dependent glycolytic pathway. In this pathway, 1,5-AG-6P is catalyzed by YbiW to undergo C-O bond cleavage, generating 1-deoxyfructose-6-phosphate. This product is further cleaved by the aldolase FsaA to produce hydroxyacetone and glyceraldehyde-3-phosphate (Fig. 1A).

To prove the inventors' hypothesis, the inventors selected the ybiW, ybiY, and fsaA genes from *Lactiplantibacillus plantarum*, subsequently heterologously expressed them in *E. coli* BL21(DE3) cells, and characterized the activity of these proteins.

The inventors reconstituted the [Fe-S]²⁺ cluster of the activase LpYbiY and characterized it. Anaerobic reconstitution of the [4Fe-4S] cluster resulted in 1.54 ± 0.12 Fe and 2.01 ± 0.08 S per monomer (a radical SAM domain, theoretical maximum of 4Fe and 4S for a [4Fe-4S] cluster) and a typical UV-visible spectrum for a [4Fe-4S] protein, with a molar extinction coefficient ε₄₁₀ₙₘ of 8.85 mM⁻¹ cm⁻¹ (Fig. 7B). Since the ε₄₁₀ₙₘ for each [4Fe-4S] cluster is about 15 mM⁻¹ cm⁻¹, the inventors estimated that each monomer contains approximately 0.59 [4Fe-4S], which is generally consistent with the measured Fe and S contents (Fig. 7A). LC-MS results showed that, like other radical SAM enzymes, LpYbiY catalyzed the cleavage of SAM in the presence of the reducing agent titanium(III) citrate to form 5'-deoxyadenosine (Fig. 7C to Fig. 7D). EPR spectroscopy showed that incubation of LpYbiW, LpYbiY, SAM, and Ti(III) generated a radical, which was quantified as 0.017 Gly•/dimer (Fig. 2A).

To determine the activity of LpYbiW, activated LpYbiW was incubated with 1,5-AG-6P and analyzed by LC-MS (Fig. 2B to Fig. 2D). A new peak with m/z (-) = 243.0 at t_{R} = 18.40 min was observed in the complete reaction mixture. In the activity assay of LpFsaA catalyzing the aldol condensation reaction of hydroxyacetone and glyceraldehyde-3-phosphate, a new peak (m/z (-) = 243.0, t_{R} = 18.40 min) was also generated in the complete reaction mixture (Fig. 9A, Fig. 9C). The Michaelis-Menten kinetic parameters of LpYbiW were also determined (Fig. 10, k_{cat} = 43.38 ± 2.96 s⁻¹/LpYbiW, K_{M} = 36.52 ± 6.66 mM). By coupling LpFsaA with LpYbiW for the reaction, LC-MS analysis detected the production of the corresponding products, hydroxyacetone and glyceraldehyde-3-phosphate (Fig. 2E to Fig. 2H). These data indicated that LpYbiW catalyzes the C-O bond cleavage of 1,5-AG-6P to generate 1-deoxyfructose-6-phosphate (1-deoxy-F6P).

According to LC-MS analysis results, a new peak with m/z (-) = 243.0 at t_{R} = 18.40 min (Fig. 18A) was generated in the complete reaction mixture. The mass spectrum corresponding to this new peak was shown in Fig. 18B, and the mass spectrum corresponding to the substrate was shown in Fig. 18C. The LC-MS results indicated that EcYbiW possesses 1,5-anhydroglucitol-6-phosphate isomerase activity.

To further investigate the catalytic mechanism of YbiW, the inventors determined the crystal structure of the EcYbiW complex with 1,5-AG-6P (with a resolution of 2.65 Å). YbiW contains one monomer per asymmetric unit. Each monomer exhibits the typical β/α barrel fold common to other GREs (Fig. 4A), as well as a radical-like conformation with Gly• and Cys• loops. From this crystal structure, the inventors could see that the sulfur atom of the Cys• residue Cys441 is adjacent to the hydrogen atom at the C2 position of the substrate, with a distance of 3.4 Å, which is consistent with a catalytic mechanism involving "abstraction of the C2 hydrogen atom by Cys•". The 2-OH group of substrate 1,5-AG-6P forms a hydrogen bond with Glu443; the phosphate group coordinates with His165, His334, and Arg453 of YbiW; and the 1-O atom forms a hydrogen bond with His334, and the 3-OH forms a hydrogen bond with Ser662 (Fig. 4C). Glu443 is involved in the deprotonation of the substrate 2-OH, which is consistent with the role of a base in the catalytic mechanism of GRE 1,2-elimination enzymes.

Based on the crystal structure of YbiW, the inventors proposed the following catalytic mechanism for YbiW. Similar to other GRE 1,2-elimination enzymes, Cys441• abstracts the H atom from the C2 position of the substrate 1,5-AG-6P, generating a substrate radical at the C2 position. Then, Glu443 deprotonates the 2-OH of the substrate, and the substrate radical translocates to the C1 position, followed by C-O bond cleavage to form a product radical. The product radical then abstracts the H atom that Cys441 abstracted from the substrate, thereby generating the product 1-deoxy-F6P and regenerating Cys441• (Fig. 4E).

Based on the crystal structure of the complex formed by EcYbiW and the substrate 1,5-anhydroglucitol-6-phosphate, and referring to the hydrogen bond distances formed between the substrate and amino acid residues, the key amino acid residues in the active center of 1,5-anhydroglucitol-6-phosphate isomerase that bind to the substrate were identified. In combination with the catalytic mechanism of glycine radical 1,2-lyases described in the literature, the key amino acid residues of the active center of 1,5-anhydroglucitol-6-phosphate isomerase were determined. In EcYbiW (SEQ ID NO:1), these amino acid residues are H165, H282, S283, H334, C441, E443, R453, T455, L562, S662, I664, and G786.

According to SSN (sequence similarity network) data analysis, the YbiW cluster showed a total of 207 nodes and 2,333 sequences. The inventors selected one representative sequence from each node. Using the crystal structure of EcYbiW (Uniprot accession No.: P75793), the amino acid residues interacting with the substrate (with reference to SEQ ID NO:1: H165, H282, S283, H334, C441, E443, R453, T455, L562, S662, I664, and G786) were used as conserved sites for multiple sequence alignment. Sequences that simultaneously contained these conserved sites were retained, resulting in the screening of 173 isozyme sequences. Further analysis of the neighboring genes of these 173 sequences was performed, and sequences whose gene cluster contained the aldolase FsaA were retained. Finally, a total of 95 representative sequences were listed (Fig. 14 and Fig. 15). Table 2 shows the accession numbers, strain sources, and amino acid sequence numbers of the 95 1,5-anhydroglucitol-6-phosphate isomerase isozymes found in the Uniprot database.

### Example 2: Identification, characterization, and testing of the PflD-related pathway

### Experimental Materials

Tryptone and yeast extract used to prepare LB medium were purchased from Oxoid Limited (Hampshire, UK). Ultrapure deionized water from a Millipore Direct-Q system was used. TALON resin was purchased from Clontech Laboratories Inc. (California, USA). 1,5-Anhydromannitol (1,5-AM) and 1,5-anhydromannitol-6-phosphate (1,5-AM-6P) were chemically synthesized by Tianjin Heowns Biochemical Technology Co., Ltd. Direct-Q oligonucleotide primers were synthesized by Beijing Tsingke Biotech Co., Ltd. All protein purification chromatography experiments were performed on ÄKTA pure FPLC system (GE Healthcare, USA). Anaerobic experiments were performed in a Lab2000 glove box (Etelux) purged with N₂ (with an oxygen concentration <5 ppm).

### Gene synthesis and cloning

EcPflD (Uniprot accession No. P32674), EcPflC (Uniprot accession No. P32675), EcFsaB (Uniprot accession No. P32669), and EcGldA (Uniprot accession No. P0A9S5) were amplified from the *E. coli* MG1655 genome using primer pairs 1F/1R, 2F/2R, 3F/3R, and 4F/4R, respectively (Table 3). EcPflD, EcFsaB, and EcGldA were inserted into the SspI site of the HT plasmid (an optimized pET28 vector) to express proteins with an N-terminal His₆ tag. EcPflC was inserted into the SspI site of the HMT vector to express a protein with an N-terminal His₆ tag and maltose-binding protein.

For biochemical characterization purposes, gene fragments of SdPflD (NCBI accession No. OCX05109.1) and SdPflC (NCBI accession No. OCX05103.1) were synthesized by Beijing Tsingke Biotech Co., Ltd. SdPflD was inserted into the SspI site of the HT plasmid (an optimized pET28 vector) to express a protein with an N-terminal His₆ tag. SdPflC was inserted into the NdeI site of the pACYC-MBP vector to express a protein with an N-terminal His₆ tag and MBP.

### Expression and purification of EcPflD, EcPflC, EcFsaB, and EcGldA

The EcPflD, EcPflC, EcFsaB, and EcGldA were plasmidized and transformed into *E. coli* BL21(DE3) cells for expression of corresponding proteins. LB agar plates containing 50 µg/mL kanamycin were used to select positive clones. Cells were cultured overnight in 4 mL of LB medium, then transferred to fresh LB medium (typically 1 L medium in a 2.6 L flask) and grown at 37°C in a shaking incubator at 220 rpm. When the OD₆₀₀ reached approximately 0.8, the temperature was lowered to 18°C, and isopropyl β-D-1-thiogalactopyranoside (IPTG) was added to a final concentration of 0.3 mM to induce production of the target protein. After 16-20 hours, cells were harvested by centrifugation (8000×g, 10 min, 4°C). The collected cells were resuspended in 40 mL of lysis buffer (50 mM Tris/HCl, pH 8.0, 100 mM KCl, 1 mM phenylmethylsulfonyl fluoride (PMSF), 0.2 mg/mL lysozyme, 0.03% Triton X-100, and 0.02 mg/mL DNase I) and frozen in a refrigerator at -80°C.

The frozen cells were thawed and incubated at room temperature (RT, 25°C) for 20 minutes, during which cell lysis occurred. 5 mM β-Mercaptoethanol (BME) was added, and nucleic acids were precipitated with 1% streptomycin sulfate. Cell debris was removed by centrifugation at 10,000×g for 10 minutes at 4°C. For EcPflD, EcFsaB, and EcGldA, the supernatant was filtered through a 0.22 µm filter and loaded onto a 5 mL TALON Co²⁺ column (Takara Bio USA, Inc.) pre-equilibrated with Buffer A (20 mM Tris/HCl, pH 7.5, 200 mM KCl, and 5 mM BME). After washing with 10 times column volumes of Buffer A to remove contaminating proteins, the target proteins were eluted with 5 times column volumes of Buffer A containing 150 mM imidazole. The eluted protein (about 20 mL) was dialyzed against 2 L of Buffer A for 3 hours at 4°C, concentrated, aliquoted, frozen in liquid nitrogen, and stored at -80°C. For EcPflC, the supernatant was filtered and loaded onto a column packed with 10 mL of amylose (New England Biolabs, Massachusetts, U.S.A.). After washing with 10 times column volumes of Buffer A to remove contaminating proteins, the target protein was eluted with Buffer A containing 10 mM maltose.

Purified proteins were detected by SDS-PAGE using commercial gels (SurePAGE, Bis-Tris, 4-20%). Protein concentrations were calculated by measuring absorbance at 280 nm using an ultra-micro UV-Vis spectrophotometer (Hangzhou Miu Instruments Co., Ltd.). [EcPflD (ε₂₈₀ = 74,720 M⁻¹cm⁻¹), MBP-EcPflC (ε₂₈₀ = 88,810 M⁻¹cm⁻¹), EcFsaB (ε₂₈₀ = 18,450 M⁻¹cm⁻¹)], EcGldA (ε₂₈₀ = 32,890 M⁻¹cm⁻¹)].

### Expression and purification of SdPflD and SdPflC for biochemical characterization

Protein expression and purification methods and procedures, SDS-PAGE detection methods, and concentration determination methods were the same as those for LpYbiW and LpYbiY described above. [SdPflD (ε₂₈₀ = 86,070 M⁻¹cm⁻¹), MBP-SdPflC (ε₂₈₀ = 107,260 M⁻¹cm⁻¹)].

### Reconstitution and characterization of the EcPflC prosthetic [Fe-S] cluster

Sequence alignment indicated that EcPflC contains only one [4Fe-4S] cluster in the radical SAM domain, with a theoretical maximum of 4Fe and 4S per monomer. The EcPflC protein solution was degassed with argon to remove oxygy, then transferred into a glove box. To the degassed protein solution, Tris/HCl with a final concentration of 100 mM, (pH 7.5), 10 mM DTT, and 4 equivalents of ferrous ammonium sulfate and sodium sulfide were added, and a resulting mixture was incubated overnight at 4°C in a metal bath (Dry Bath H2O3-100C; Coyote Bioscience, Beijing, China). Subsequently, 4 equivalents of EDTA solution were added, a resulting mixture was repeated concentration using centrifugal filter units (1.5 mL YM-30 Amicon; Millipore) and dilution with a buffer (20 mM Tris/HCl, pH 7.5, and 100 mM KCl) to exchange the buffer.

Fe contents of unconstituted and reconstituted EcPflC were quantified using ferrozine [3-(2-pyridyl)-5,6-diphenyl-1,2,4-triazine-p,p'-disulfonic acid monosodium salt]. A standard curve for Fe was established using AAS iron standards in a range of 0-600 µM. Sulfur contents of unconstituted and reconstituted EcPflC were determined by measuring the absorbance of methylene blue formed from the reaction with N,N-dimethyl-p-phenylenediamine dihydrochloride (DPD). A standard curve for sulfur was established using Na₂S in a range of 0-600 µM.

UV-Vis absorption spectra of EcPflC in a range of 200-800 nm were measured using a NanoPhotometer NP80 Mobile (Germany). Unconstituted and reconstituted EcPflC were diluted to 10 µM with a buffer containing 20 mM Tris-HCl, (pH 7.5), and 100 mM KCl, and transferred to a septum-sealed anaerobic cuvette for measurement. To a reconstituted EcPflC solution, 10 equivalents of titanium(III) citrate were added and a resulting mixure was incubated for 10 minutes to determine its reduced form.

### LC-MS analysis of SAM cleavage catalyzed by EcPflC

A reaction system (500 µL) containing 20 mM Tris/HCl, (pH 7.5), 100 mM KCl, 200 µM titanium(III) citrate, 20 µM reconstituted EcPflC, and 1 mM SAM was incubated overnight at room temperature in a glove box. The negative control lacked titanium(III) citrate. Formic acid (with a final concentration of 5% v/v) was added to quench the reaction, and a resulting mixture was incubated in a boiling water bath for 1 minute to denature the protein. Precipitated protein was removed by centrifugation at 14,000×g for 15 minutes. The supernatant was filtered using a 0.22 µm PES membrane and subjected to LC-MS analysis. The method was as follows: 20 µL of sample was injected onto an Agilent 6420 Triple Quadrupole LC/MS instrument (Agilent Technologies) equipped with a C18 reversed-phase column. UV absorbance was monitored at 257 nm. The mobile phase consisted of water (A) and acetonitrile (B), with a linear gradient elution from 0-16% B over 30 minutes at a flow rate of 0.5 mL/min. Commercial 5'-dA was used as a standard, and the formation of SAM cleavage product catalyzed by EcPflC was verified by mass spectrometry.

### Generation of the EcPflD radical detected by electron paramagnetic resonance (EPR) spectroscopy

The EcPflD glycine radical was characterized using continuous-wave X-band electron paramagnetic resonance (EPR) spectroscopy. A reaction system (200 µL) containing 20 mM Tris/HCl, (pH 7.5), 100 mM KCl, 100 µM titanium(III) citrate, 1 mM SAM, 80 µM reconstituted EcPflC, and 40 µM EcPflD was incubated for 15 minutes at room temperature in a glove box. Glycerol (10%) was added, and a resulting mixture was transferred to an EPR tube with an outer diameter of 4 mm and a length of 8 inches (Wilmad Lab Glass, 734-LPV-7). The tube was sealed with a rubber stopper, removed from the glove box, and frozen in liquid nitrogen prior to EPR analysis. Experimental spectra of the glycyl radical were modeled using Bruker Xepr spin fitting to obtain g-values, hyperfine coupling constants, and linewidths. Double integration of the simulated spectra was used to measure the spin concentration. EPR spectra were obtained by accumulating 30 scans under the following conditions: temperature, 90 K; center field, 3370.00 G; sweep width, 200 G; microwave power, 10 µW; microwave frequency, 9.43 MHz; modulation amplitude, 0.5 mT; modulation frequency, 100 kHz; time constant, 20.48 ms; conversion time, 25 ms; sweep time, 20 s; receiver gain, 43 dB.

### LC-MS analysis for EcPflD activity assay

A 200 µL reaction mixture containing 10 µM activated EcPflD, 0.1 mM titanium(III) citrate, and 10 mM 1,5-AM-6P was incubated for 1 hour at room temperature in a glove box. Negative controls omitted 1,5-AM-6P, SAM, or activated EcPflD. Protein was precipitated by adding 200 µL of acetonitrile, a resulting precipitate was removed by centrifugation, and the supernatant was filtered through a 0.22 µm PES membrane for LC-MS analysis.

LC-MS analysis was performed on an Agilent 6420 Triple Quadrupole LC/MS instrument (Agilent Technologies). The drying gas was maintained at a temperature of 300°C, with a flow rate of 9 L/min, and the nebulizer pressure was 15 psi. LC-MS analysis was performed using a ZIC-HILIC column (5 mm, 200 Å, 150×4.6 mm; Merck). HPLC conditions were as follows: mobile phase A = 90% 20 mM ammonium acetate + 10% acetonitrile, mobile phase B = acetonitrile; gradient elution from 90% B to 70% B over 10 minutes, then from 70% B to 50% B over 20 minutes. Flow rate was set to 0.5 mL/min. The mass spectrometer was operated in ESI negative ion mode.

### LC-MS analysis for SdPflD activity assay

The LC-MS analysis method for species SdPflD of *Streptococcus dysgalactiae subsp.Equisimilis* activity assay was the same as that for EcPflD.

### LC-MS analysis for EcFsaB-coupled EcPflD activity

A reaction mixture (200 µL) containing 10 µM activated EcPflD, 0.1 mM titanium(III) citrate, and 10 mM 1,5-AM-6P was incubated for 1 hour at room temperature in a glove box, enzymatic activity of EcFsaB-coupled EcPflD was measured. 10 µM EcFsaB was added to the above reaction mixture, and a resulting mixture was further incubated for another 1 hour in the glove box. Then, 50 µL of the reaction sample was mixed with 550 µL of 0.73 M sodium acetate (pH 5.0) and then with 400 µL of freshly prepared 2,4-dinitrophenylhydrazine (DNPH) solution (20 mg dissolved in 50 mL methanol), a resulting mixture was further incubated at 50°C for 1 hour. a resulting mixture was centrifuged at 13,000×g for 5 minutes and filtered before LC-MS analysis. 3-Phosphoglyceraldehyde (5 mM, Sigma-Aldrich) and hydroxyacetone (2 mM, TCI) standards were used as positive controls, which were similarly treated by sodium acetate (pH 5.0) and freshly prepared DNPH solution for 1 hour.

LC-MS analysis was performed using an Agilent ZORBAX SB-C18 reversed-phase column (4.6×250 mm) with 20 µL samples in ESI negative ion mode. The solvent system consisted of solvent C (deionized ultrapure water containing 0.1% formic acid) and D (chromatographic acetonitrile containing 0.1% formic acid). The LC analysis was performed with the following program: elution from 15% D to 100% D over 15 minutes at a flow rate of 1.0 mL/min, and the detection wavelength was set to 360 nm.

### Spectrophotometric determination of EcPflD, EcFsaB, and EcGldA coupling

A reaction mixture containing 20 mM Tris/HCl, (pH 7.5), 100 mM KCl, 10 mM 1,5-AM-6P, 40 nM activated EcPflD, 5 µM EcFsaB, 5 µM EcGldA, and 0.4 mM NADH was incubated in a 1 cm Eppendorf cuvette in a glove box at room temperature. Absorbance at 340 nm was monitored every 5 seconds using a cuvette mode of an ultra-micro UV-Vis spectrophotometer (Hangzhou Miu Instruments Co., Ltd.) inside the glove box. Negative controls were prepared by omitting 1,5-AM-6P, SAM, or EcPflD.

### LC-MS analysis for EcFsaB activity

EcFsaB activity for catalyzing the aldol condensation of hydroxyacetone and 3-phosphoglyceraldehyde was also assayed. A 200 µL reaction system containing 50 mM Tris/HCl, (pH 8.0), 100 mM KCl, 5 mM 3-phosphoglyceraldehyde, 10 mM hydroxyacetone, and 10 µM EcFsaB was incubated for 1 hour at room temperature. Negative controls omitted 3-phosphoglyceraldehyde, hydroxyacetone, or EcFsaB. LC-MS analysis was performed using the same sample preparation method and elution conditions as described for EcPflD activity assay, using a ZIC-HILIC column.

### Determination of Michaelis-Menten kinetic parameters of EcPflD

The reaction system containing 20 mM Tris/HCl, (pH 7.5), 100 mM KCl, 40 nM activated EcPflD and 1,5-AM-6P with various substrate concentrations, 2 µM EcFsaB, 2 µM EcGldA, and 0.4 mM NADH was measured using a cuvette mode of an ultra-micro UV-Vis spectrophotometer (Hangzhou Miu Instruments Co., Ltd.) in a 1 cm Eppendorf cuvette inside a glove box. The decrease in A₃₄₀ₙₘ was monitored at 5-second intervals.

### Construction of SdPflD expression plasmid for crystallography studies

An *E. coli* codon-optimized DNA fragment encoding PflD from *Streptococcus dysgalactiae subsp. equisimilis* (SdPflD, NCBI accession No. OCX05109.1) was synthesized by Beijing Tsingke Biotech Co., Ltd. and inserted into the SspI site of the HT vector.

### Expression and purification of SdPflD for crystallography studies

### Spectrophotometric determination of EcPflD, EcFsaB, and EcGldA coupling

For crystallographic studies of SdPflD, *E. coli* BL21(DE3) cells carrying the plasmid HT-SdPflD were cultured in 2 L of LB medium containing 50 µg/mL kanamycin. Cells were harvested and lysed using the same method as for EcYbiW. The supernatant of the cell lysate was purified using a 10 mL TALON Co²⁺ column. The eluate was pre-mixed with recombinant His₆-tagged TEV protease (with a molar ratio of TEV protease to SdPflD of approximately 1:10) and dialyzed overnight against 2 L of buffer A (20 mM Tris/HCl, pH 7.5, 200 mM KCl, and 5 mM BME) containing 5 mM BME. The dialyzed sample was loaded onto a 10 mL TALON Co²⁺ column to retain the TEV protease and His₆ tag. The flow-through was collected and dialyzed against 2 L of buffer B (20 mM Tris/HCl, pH 7.5, 5 mM BME), and then loaded onto a 10 mL DEAE column as described above. The main peak containing SdPflD was concentrated and purified using a Superdex 200 gel filtration column. The sample from the gel filtration column was concentrated to a final concentration of 9.9 mg/mL.

### Crystallization, data collection, and structure determination of SdPflD

Initial screening for SdPflD crystals was performed using the sitting-drop vapor diffusion method in a 96-well format with an automated liquid handling robot system (Gryphon, Art Robbins). Screening was carried out at 291 K using various crystal screening kits from Hampton Research and Molecular Dimensions. After further optimization using the hanging-drop vapor diffusion method in 24-well plates, crystals suitable for single-crystal X-ray diffraction studies were obtained. The optimal conditions for producing block-like crystals of SdPflD were 5.0 mg/mL SdPflD protein solution, 1.2 M sodium citrate, 0.1 M sodium HEPES, pH 8.0, and 10 mM 1,5-AM-6P. The block-like crystals were cryoprotected with 10% glycerol. Diffraction data were collected and processed at beamline BL10U2 of the Shanghai Synchrotron Radiation Facility (SSRF) to a resolution of 2.34 Å. A crystal structure model created with the website PHYRE2 was used for molecular replacement in the PHENIX software. The structure was manually built according to the electron density using Coot software, further refined in PHENIX, and then deposited in the RCSB Protein Data Bank (accession code: 8ID0). The appendix table contains data collection and final refinement statistics (Table 1). All structure drawings were generated using UCSF Chimera (Fig. 4B and Fig. 4D).

### Identification of the active center of 1,5-anhydromannitol-6-phosphate isomerase

The obtained PDB data of the SdPflD complex with 1,5-AM-6P (with a resolution of 2.34 Å) were displayed using UCSF Chimera software. Focusing on the substrate 1,5-AM-6P, amino acid residues within 10 Å of the substrate center were selected for display. Based on the hydrogen bond distances between the substrate and amino acid residues, key amino acid residues in the active center of 1,5-anhydromannitol-6-phosphate isomerase that bind to the substrate were identified. In combination with the catalytic mechanism of glycine radical 1,2-lyases described in the literature, the key amino acid residues of the active center of 1,5-anhydromannitol-6-phosphate isomerase were determined.

### Identification of isozymes of 1,5-anhydromannitol-6-phosphate isomerase

To identify isozymes of 1,5-anhydromannitol-6-phosphate isomerase in the glycine radical enzyme (GRE) family, a sequence similarity network (SSN) of 25,347 unique sequences from InterPro family IPR004184 was constructed using the web-based Enzyme Function Initiative Enzyme Similarity Tool (EFI-EST). The alignment score threshold was set to 250, the minimum sequence length was set to 650, and an 80% similarity representative node network (RepNode network) was displayed using Cytoscape v3.519. Under these settings, previously characterized GREs with different catalytic activities were separated into distinct clusters.

Sequences with 80% or higher similarity were represented by a single node, and sequences were folded to reduce the total number of nodes, making the less complex network easier to load in Cytoscape. For the cluster formed by PflD, one representative sequence was selected from each node based on different nodes. Using the crystal structure of the complex formed by SdPflD and the substrate, amino acid residues interacting with the substrate were used as conserved sites for multiple sequence alignment. Sequences that simultaneously contained these conserved sites were retained. Further analysis of neighboring genes of these sequences was performed, and sequences whose gene cluster contained an aldolase were retained. Finally, representative sequences were selected.

### Results, analysis, and discussion

A gene cluster involved in sugar metabolism is present in the *E. coli* MG1655 genome. Through bioinformatics analysis of this gene cluster, the inventors noted an uncharacterized GRE (PflD) involved therein. In addition to PflD and the S-adenosylmethionine radical enzyme family glycine radical enzyme activase (PflC), this gene cluster also contains a 1-deoxyfructose-6-phosphate aldolase (FsaB), a hydroxyacetone reductase (GldA), and a complete phosphoenolpyruvate-dependent phosphotransferase system (PTS), which includes PtsA, FrwB, FrwC, and FrwD. The function of the PTS is to transfer a phosphate group from phosphoenolpyruvate (PEP) to sugar coming in cells via a series of sequential steps involving different PTS components. The PTS consists of cytoplasmic components lacking sugar specificity and membrane-associated enzymes that are specific for up to a few sugars. This PTS transport complex may be involved in the uptake of hexoses and the phosphorylation of substrates. The aldolase FsaB may catalyze the formation of corresponding 2-keto-hexose-6-phosphates from hydroxyacetone, 1,3-dihydroxyacetone, and glyceraldehyde-3-phosphate. GldA catalyzes the dehydrogenation of glycerol or propylene glycol to generate 1,3-dihydroxyacetone or hydroxyacetone. Accordingly, the inventors speculated that this GRE is involved in sugar metabolism and that its substrate might be a hexose phosphate. Based on molecular docking results, the inventors hypothesized that the substrate of PflD is 1,5-anhydromannitol-6-phosphate (1,5-AM-6P) and proposed a novel radical-dependent glycolytic pathway. In this pathway, 1,5-AM-6P is catalyzed by PflD to undergo C-O bond cleavage, generating 1-deoxy-F6P. This product is further cleaved by the aldolase FsaB to produce hydroxyacetone and glyceraldehyde-3-phosphate. Hydroxyacetone is reduced by the hydroxyacetone reductase GldA to generate 1,2-propanediol (Fig. 1B).

To prove the inventors' hypothesis, the inventors selected the pflD, pflC, fsaB, and gldA genes from *E. coli* MG1655, subsequently heterologously expressed them in *E. coli* BL21(DE3) cells, and characterized the activity of these proteins.

The inventors reconstituted the [Fe-S]²⁺ cluster of the activase EcPflC and characterized it. Anaerobic reconstitution of the [4Fe-4S] cluster resulted in 2.15 ± 0.12 Fe and 2.30 ± 0.07 S per monomer (a radical SAM domain, theoretical maximum of 4Fe and 4S for a [4Fe-4S] cluster) and a typical UV-visible spectrum for a [4Fe-4S] protein, with a molar extinction coefficient ε₄₁₀ₙₘ of 9.45 mM⁻¹ cm⁻¹. Since the ε₄₁₀ₙₘ for each [4Fe-4S] cluster is about 15 mM⁻¹ cm⁻¹, the inventors estimated that each monomer contains approximately 0.63 [4Fe-4S], which is generally consistent with the measured Fe and S contents (Fig. 8A and Fig. 8B). LC-MS results showed that, like other radical SAM enzymes, EcPflC catalyzed the cleavage of SAM in the presence of the reducing agent titanium(III) citrate to form 5'-deoxyadenosine (Fig. 8C and Fig. 8D). EPR spectroscopy showed that incubation of EcPflD, EcPflC, SAM, and Ti(III) generated a radical, which was quantified as 0.060 Gly•/dimer (Fig. 3A).

To determine the activity of EcPflD, activated EcPflD was incubated with 1,5-AM-6P and analyzed by LC-MS. A new peak with m/z (-) = 243.0 at t_{R} = 18.40 min was observed in the complete reaction mixture (Fig. 3B to Fig. 3D). In the activity assay of EcFsaB catalyzing the aldol condensation of hydroxyacetone and glyceraldehyde-3-phosphate, a new peak (m/z (-) = 243.0, t_{R} = 18.40 min) was also generated in the complete reaction mixture (Fig. 9B to Fig. 9D). The Michaelis-Menten kinetic parameters of EcPflD were also determined (Fig. 11, k_{cat} = 33.47 ± 0.79 s⁻¹/EcPflD, K_{M} = 1.33 ± 0.12 mM). By coupling EcPflD with EcFsaB for the reaction, LC-MS analysis detected the production of the corresponding products, hydroxyacetone and glyceraldehyde-3-phosphate (Fig. 3E to Fig. 3H). Using the coupled spectrophotometric assay of EcPflD, EcFsaB, and EcGldA, a decrease in A₃₄₀ₙₘ was observed only in the complete reaction mixture, indicating that substrate 1,5-AM-6P undergoes C-O bond cleavage catalyzed by EcPflD to generate 1-deoxy-F6P, which is then cleaved by EcFsaB to produce hydroxyacetone and glyceraldehyde-3-phosphate; hydroxyacetone is reduced by EcGldA to generate 1,2-propanediol (Fig. 3I).

The pflD gene cluster in *Streptococcus dysgalactiae subsp. equisimilis* was shown in Fig. 19A. According to LC-MS analysis results, a new peak with m/z (-) = 243.0 at t_{R} = 18.40 min (Fig. 19B) was generated in the complete reaction mixture. The mass spectrum corresponding to this new peak was shown in Fig. 19C, and the mass spectrum corresponding to the substrate was shown in Fig. 19D. The LC-MS results indicated that SdPflD possesses 1,5-anhydromannitol-6-phosphate isomerase activity.

To further investigate the catalytic mechanism of PflD, the inventors determined the crystal structure of the SdPflD complex with 1,5-AM-6P (with a resolution of 2.34 Å). PflD contains one monomer per asymmetric unit. Each monomer exhibits the typical β/α barrel fold common to other GREs (Fig. 4B), as well as a radical-like conformation with Gly• and Cys• loops. From this crystal structure, it can be seen that the sulfur atom of the Cys• residue Cys431 is adjacent to the hydrogen atom at the C2 position of the substrate, with a distance of 3.74 Å, which is consistent with a catalytic mechanism involving "abstraction of the C2 hydrogen atom by Cys•". The 2-OH group of substrate 1,5-AM-6P forms a hydrogen bond with Glu433; the phosphate group coordinates with Gln162, Ser277, and Arg323 of PflD; and the 1-O atom forms hydrogen bonds with His169 and Glu445, and the 3-OH forms a hydrogen bond with Ser278 (Fig. 4D). Glu433 is involved in the deprotonation of the substrate 2-OH, consistent with the role of a base in the catalytic mechanism of GRE 1,2-elimination enzymes.

Based on the crystal structure of PflD and its similarity to other GRE 1,2-elimination enzymes, the inventors proposed the following catalytic mechanism for PflD. Cys431• abstracts the H atom from the C2 position of the substrate 1,5-AM-6P, generating a substrate radical at the C2 position. Then, Glu433 deprotonates the 2-OH of the substrate, and the substrate radical translocates to the C1 position, followed by C-O bond cleavage to form a product radical. The product radical then abstracts the H atom that Cys431 abstracted from the substrate, thereby generating the product 1-deoxy-F6P and regenerating Cys431• (Fig. 4F).

Based on the crystal structure of the complex formed by SdPflD and the substrate 1,5-anhydromannitol-6-phosphate, and referring to the hydrogen bond distances formed between the substrate and amino acid residues, the key amino acid residues in the active center of 1,5-anhydromannitol-6-phosphate isomerase that bind to the substrate were identified. In combination with the catalytic mechanism of glycine radical 1,2-lyases described in the literature, the key amino acid residues of the active center of 1,5-anhydromannitol-6-phosphate isomerase were determined. In SdPflD, these amino acid residues are Q162, H169, S277, S278, R323, F331, P335, C431, E433, D445, Y628, V630, and G752.

According to the SSN sequence analysis, the PflD cluster showed a total of 89 nodes and 953 sequences. the inventors selected one representative sequence from each node. Using the crystal structure of SdPflD (NCBI accession number OCX05109.1), the amino acid residues interacting with the substrate (Q162, H169, S277, S278, R323, F331, P335, C431, E433, D445, Y628, V630, and G752) were used as conserved sites for multiple sequence alignment. Sequences that simultaneously contained these conserved sites were retained, resulting in the screening of 77 isozyme sequences. Further analysis of the neighboring genes of these 77 sequences was performed, and sequences whose gene cluster contained the aldolase FsaB were retained. Finally, a total of 47 representative sequences were listed (Fig. 14 and Fig. 16).

### Example 3: Experiments on E. coli utilizing 1,5-anhydroglucitol and 1,5-anhydromannitol

### Gene knockout in E. coli MG1655

The CRISPR-assisted homologous recombination strategy was used to knock out ybiW and pflD in *E. coli* MG1655, respectively. The inventors designed primer pairs 7F/7R and 8F/8R to amplify the upstream and downstream sequences of ybiW, 500 bp at the 5' end and 504 bp at the 3' end, respectively, to generate fragments 1 and 2. Fragments 1 and 2 were assembled using PCR with primers 7F and 8R to generate fragment 3. Using pRed_Cas9_recA plasmid as template, fragments 4 and 5 were obtained by PCR amplification with primer pairs 9F/9R and 10F/10R. Fragments 3, 4, and 5 were Gibson assembled to generate the recombinant pRed_Cas9_recA_ΔybiW plasmid, which contains the guide RNA sequence "GCCTGCCAGAAAGTCTGCGG" (SEQ ID NO:187) designed to introduce Cas9-catalyzed cleavage near the homologous recombination site to increase the rate of desired homologous recombination.

The construction of the recombinant plasmid pRed_Cas9_recA_ΔpflD was similar to that of pRed_Cas9_recA_ΔybiW. Primer pairs 11F/11R and 12F/12R were used to amplify the upstream (500 bp) and downstream (505 bp) sequences of pflD to generate fragments 6 and 7. Similarly, fragment 8 was generated using fragments 6 and 7 as templates and 11F and 12R as primers. Fragments 9 and 10 were amplified by PCR using pRed_Cas9_recA as template with primer pairs 9F/13R and 13F/10R. pRed_Cas9_recA_ΔpflD containing the guide RNA sequence "AAATACCAGAACCCGCGCGG" (SEQ ID NO:188) was constructed using Gibson assembly with fragments 8, 9, and 10. DNA sequencing with primers 14F, 14R, and 15F confirmed the recombinant plasmids pRed_Cas9_recA_ΔybiW and pRed_Cas9_recA_ΔpflD.

To obtain *E. coli* MG1655_ΔybiW and E. coli MG1655_ΔpflD strains, pRed_Cas9_recA_ΔybiW and pRed_Cas9_recA_ΔpflD were transformed into competent cells of *E. coli* MG1655 strain by electroporation, respectively. Positive transformants were selected on LB agar plates containing 50 µg/mL kanamycin in a 30°C incubator. Single colonies were cultured in 5 mL LB medium containing 50 mg/L kanamycin and 2 g/L arabinose at 30°C for 24 hours. Cells were harvested and plated on LB agar plates containing 50 g/L kanamycin and 2 g/L arabinose at 30°C. Single colonies were picked and streaked on LB agar plates and cultured at 37°C to eliminate the recombinant plasmid. Colony PCR verification was performed using primers 16F/16R for ΔybiW and 17F/17R for ΔpflD, theoretically amplifying DNA fragments of approximately 1500 bp. Agarose gel electrophoresis showed successful genomic knockout, which was further confirmed by DNA sequencing using the same primer pairs.

### Anaerobic growth of E. coli strains using 1,5-AG/1,5-AM as the sole carbon source

Single colonies of *E. coli* MG1655 WT, ΔybiW, and ΔpflD strains freshly grown on LB agar plates were inoculated into 5 mL LB medium and cultured in a shaking incubator at 37°C for 4 hours. Cells from 100 µL of culture were pelleted by centrifugation, transferred to anaerobic bottles containing 5 mL anaerobic LB medium, and cultured at 37°C for 6 hours. Cells from 100 µL of culture were collected by centrifugation, transferred to anaerobic bottles containing 5 mL anaerobic M9 medium, and cultured at 37°C for 3 days. 1 mL of cells were harvested, washed three times with glucose-free anaerobic M9 medium, and resuspended in this medium. 100 µL of the cell suspension was transferred to anaerobic bottles, each containing 5 mL of glucose-free M9 medium or the same medium supplemented with 20 mg of sugar (glucose, 1,5-AG, or 1,5-AM). The anaerobic bottles were then placed in an incubator at 37°C for 7 days before photography, and cells were collected for SDS-PAGE gel analysis.

### SDS/PAGE and mass spectrometry for protein identification

Cells were harvested by centrifugation, lysed by boiling in Laemmli loading buffer, and analyzed on 10% SDS/PAGE gels. Prominent protein bands induced in cells cultured with glucose, 1,5-anhydroglucitol, or 1,5-anhydromannitol were excised manually. After in-gel digestion and extraction, the peptide mixture was analyzed by a Fusion Lumos mass spectrometer coupled to an Easy nLC 1200 system (Thermo Fisher Scientific). MS/MS spectra from each LC-MS/MS run were searched against the E. coli protein database (release April 1, 2021) containing 15,862 sequence entries from UniProt using an in-house Proteome Discoverer (version 2.2) search algorithm. Protein identification was performed based on Sequest HT.

### GC analysis of fermentation broth

Wild-type *E. coli* MG1655 was cultured at 37°C for 7 days in different carbon sources, and cells were removed by centrifugation. 200 µL of fermentation broth was aspirated, 800 µL of chromatographic grade absolute ethanol was added, mixed by vortexing, and centrifuged to remove insoluble precipitate. The resulting solution was filtered through a 0.45 µm organic filter membrane, and the filtrate was analyzed by gas chromatography. (R)-1,2-propanediol commercial standard was dissolved in chromatographic grade absolute ethanol.

Gas chromatography (GC) analysis was performed using an Agilent 6820 G1176A gas chromatograph (Agilent Technologies). The GC column used for analysis was an AT^{™}-Aquawax-DA (Alltech) gas chromatography column (30 m × 0.53 mm, 1.0 µm). GC conditions were as follows: carrier gas was high-purity nitrogen, column flow rate was constant at 1.0 mL/min; injector temperature 230°C, detector temperature 240°C; hydrogen flow rate 20 mL/min; air flow rate 200 mL/min. Temperature program was used: initial column temperature 60°C, hold for 2 minutes, ramp to 80°C at 20°C/min, hold for 3 minutes, ramp to 160°C at 20°C/min, hold for 2 minutes; then ramp to 220°C at 15°C/min, hold for 10 minutes.

### Results, analysis, and discussion

Using M9 medium with 1,5-AG as the sole carbon source, *E. coli* MG1655_wild-type and *E. coli* MG1655_ΔybiW strains were cultured anaerobically; glucose was used as the sole carbon source for positive control, and no carbon source was added for negative control. The *E. coli* MG1655_wild-type strain grew when using glucose or 1,5-AG as the carbon source, respectively; whereas the *E. coli* MG1655_ΔybiW strain could only grow using glucose as the sole carbon source (Fig. 5A). Further SDS-PAGE analysis results showed that when the *E. coli* MG1655_wild-type strain used 1,5-AG as the sole carbon source, the corresponding proteins in the YbiW-dependent pathway were induced. Combined with protein mass spectrometry analysis, the induced bands were identified respectively: two about 95 kDa bands contained PtsA and YbiW; an about 42 kDa band was identified as GldA; an about 27 kDa band contained FsaB and FsaA (Fig. 5B).

Similarly, using M9 medium with 1,5-AM as the sole carbon source, *E. coli* MG1655_wild-type and *E. coli* MG1655_ΔpflD strains were cultured anaerobically; glucose was used as the sole carbon source for positive control, and no carbon source was added for negative control. The *E. coli* MG1655_wild-type strain grew when using glucose or 1,5-AM as the carbon source, respectively; whereas the *E. coli* MG1655_ΔpflD strain could only grow using glucose as the sole carbon source (Fig. 5C). Further SDS-PAGE analysis results showed that when the *E. coli* MG1655_wild-type strain used 1,5-AM as the sole carbon source, the corresponding proteins in the PflD-dependent pathway were induced. Combined with protein mass spectrometry analysis, the induced bands were identified respectively: an about 95 kDa band contained PtsA; an about 90 kDa band contained PflD; an about 42 kDa band was identified as GldA; an about 27 kDa band contained FsaB (Fig. 5D). GC analysis results showed that when 1,5-AG or 1,5-AM was used as the sole carbon source, *E. coli* MG1655 produced 1,2-propanediol under anaerobic conditions, with a retention time of 8.85 min (Fig. 13).

The *E. coli* MG1655 genome contains the ybiW and pflD gene clusters (Fig. 5E). Combined with the growth results of strains on different carbon sources, SDS-PAGE analysis, and protein mass spectrometry analysis results, the inventors believe that 1,5-AG can induce the expression of the YbiW and PflD-related gene clusters in *E. coli* MG1655. 1,5-AG is transported into the cell using the PTS system in the PflD gene cluster, inducing high expression of FsaB and GldA in the PflD gene cluster, while also inducing high expression of YbiW and FsaA in the YbiW gene cluster. 1,5-AG is transported into the cell by the PTS system, and the C-6 position of the substrate is phosphorylated to generate 1,5-AG-6P; under the catalysis of YbiW, C-O bond cleavage occurs to generate 1-deoxy-F6P; this product is cleaved by FsaB and FsaA to produce hydroxyacetone and glyceraldehyde-3-phosphate; hydroxyacetone is reduced by EcGldA to generate 1,2-propanediol.

In contrast, 1,5-AM can only induce high expression of PflD-related pathway proteins. The substrate is transported into the cell by the PTS system in the gene cluster, and the C-6 position is phosphorylated to generate 1,5-AM-6P. Under the catalysis of PflD, C-O bond cleavage occurs to generate 1-deoxy-F6P; this product is cleaved by FsaB to produce hydroxyacetone and glyceraldehyde-3-phosphate; hydroxyacetone is reduced by GldA to generate 1,2-propanediol (Fig. 5F).

### Example 4: Experiments on production of 1,2-propanediol from starch via 1,5-AG and 1,5-AM, respectively Materials and Methods

### Experimental materials

Tryptone and yeast extract used to prepare LB medium were purchased from Oxoid Limited (Hampshire, UK). Ultrapure deionized water was purchased from Millipore Direct-Q. TALON resin was purchased from Clontech Laboratories Inc (California, USA). 1,5-Anhydroglucitol (1,5-AG) was purchased from Shanghai Yuanye Bio-Technology Co., Ltd., and 1,5-anhydromannitol (1,5-AM) was chemically synthesized by Tianjin Heowns Biochemical Technology Co., Ltd. Soluble starch was purchased from Tianjin Yuanli Chemical Co., Ltd. Maltose was purchased from Beijing Solarbio Science & Technology Co., Ltd. Direct-Q oligonucleotide primers were synthesized by Beijing Tsingke Biotech Co., Ltd. All protein purification chromatography experiments were performed on an ÄKTA pure FPLC system (GE Healthcare, USA). Anaerobic experiments were performed in a Lab2000 glove box (Etelux) protected by N₂ (with an oxygen concentration of less than 5 ppm).

### Gene synthesis and cloning

DNA fragments of the *E. coli* codon-optimized gene encoding α-1,4-glucan lyase from *Gracilariopsis lemaneiformis* (α-1,4-glucan lyase, Uniprot accession No. Q9STC1), the gene encoding 1,5-anhydro-D-fructose reductase from *Sus scrofa* (Gafr, Uniprot accession No. P82125), and the gene encoding 1,5-anhydro-D-fructose reductase from *Ensifer adhaerens* (Mafr, Uniport accession No. Q2I8V6) were synthesized by Beijing Tsingke Biotech Co., Ltd. and inserted into the SspI site of the HT plasmid (an optimized pET28 vector). To improve protein solubility, the α-1,4-glucan lyase gene was re-cloned using primer pairs 20F/20R and 21F/21R (Table 7) with the plasmid HT-α-1,4-glucan lyase as template, yielding two fragments, which were assembled by Gibson assembly to produce the recombinant plasmid α-1,4-glucan lyase-His₆, which contains a 6×His tag at the C-terminus. Mafr was re-cloned using primer pair 22F/22R (Table 7) and inserted into the SspI site of the HMT vector. In addition, an *E. coli* codon-optimized FDH gene fragment (FDH) from *Pseudomonas sp.* 101 was synthesized and cloned into the NcoI site of the expression vector pET28a.

The resulting HT plasmid contained a His₆ tag and a tobacco etch virus (TEV) protease cleavage site; HMT contained an N-terminal His6 tag, maltose-binding protein (MBP), and a TEV protease cleavage site; the pET28a plasmid contained an N-terminal His₆ tag, a thrombin site, followed by the gene of interest.

The gene fragment of sugar kinase TlGlkA (Uniprot number: Q7M537) was synthesized by Beijing Tsingke Biotech Co., Ltd. and inserted into the SspI site of the HT plasmid (an optimized pET28 vector) to express a protein with an N-terminal His₆ tag.

### Expression and purification of recombinant proteins

The plasmids α-1,4-glucan lyase-His₆, HT-Gafr, HMT-Mafr, and pET28a-FDH were transformed into *E. coli* BL21(DE3) cells for expression of the corresponding proteins. Positive clones were selected on LB agar plates containing 50 µg/mL kanamycin. Cells were cultured overnight in 4 mL LB medium, then transferred into fresh LB medium (typically 1 L LB medium in a 2.6 L flask) and grown at 37°C in a shaking incubator at 220 rpm. When OD₆₀₀ reached approximately 0.8, the temperature was reduced to 18°C, and isopropyl β-D-1-thiogalactopyranoside (IPTG) was added to a final concentration of 0.2 mM to induce the production of the target proteins. After 16-20 hours, cells were collected by centrifugation (8000×g, 10 minutes at 4°C). The collected cells were resuspended in 40 mL of lysis buffer (50 mM Tris/HCl, pH 8.0, 100 mM KCl, 1 mM phenylmethylsulfonyl fluoride (PMSF), 0.2 mg/mL lysozyme, 0.03% Triton X-100, and 0.02 mg/mL DNase I) and frozen in a refrigerator at -80°C.

The frozen cells were thawed and incubated at room temperature (RT, 25°C) for 20 minutes, during which cell lysis occurred. 5 mM β-mercaptoethanol (BME) was added, and nucleic acids were precipitated and removed with 1% streptomycin sulfate. Cell debris was removed by centrifugation at 10,000×g for 10 minutes at 4°C. The supernatant was filtered through a 0.22 µm filter and loaded onto a 5 mL TALON Co²⁺ column (Takara Bio USA, Inc.) pre-equilibrated with buffer A (20 mM Tris/HCl, pH 7.5, 200 mM KCl, and 5 mM BME). The column was washed with 10 times column volumes of buffer A to remove impurities, and proteins were eluted with 5 times column volumes of buffer A containing 150 mM imidazole. The eluted protein (about 20 mL) was dialyzed against 2 L of buffer B (20 mM PBS buffer, pH 7.2, 200 mM KCl, and 5 mM BME) for 3 hours at 4°C, concentrated, aliquoted, frozen in liquid nitrogen, and stored at -80°C.

Purified proteins were detected by SDS-PAGE using commercial gels (SurePAGE, Bis-Tris, 4-20%). Protein concentrations were calculated by measuring their absorbance at 280 nm using an Ultra-micro UV-Vis spectrophotometer (Hangzhou Miou Instruments Co., Ltd.). [α-1,4-glucan lyase (ε₂₈₀ = 218,420 M⁻¹ cm⁻¹), Gafr (ε₂₈₀ = 36,900 M⁻¹ cm⁻¹), MBP-Mafr (ε₂₈₀ = 95,800 M⁻¹ cm⁻¹), and FDH (ε₂₈₀ = 58,330 M⁻¹ cm⁻¹)].

The expression and purification methods, SDS-PAGE detection method, and concentration determination method for sugar kinase TlGlkA were the same as those for LpYbiW and LpYbiY. [TlGlkA (ε₂₈₀ = 51,800 M⁻¹ cm⁻¹)]. The results were shown in FIG. 26.

### LC-MS analysis for α-1,4-glucan lyase activity assay

A 200 µL reaction mixture containing 50 mM PBS buffer (pH 7.2), 100 mM KCl, 10 mM maltose/10 mg/mL soluble starch, and 5 µM α-1,4-glucan lyase was incubated at RT for 2 hours. Nno substrate (maltose/soluble starch) or no α-1,4-glucan lyase was contained in negative controls. 100 µL of acetonitrile was added to the reaction system to precipitate proteins, the precipitate was removed by centrifugation, and the supernatant was filtered through a 0.22 µm PES membrane for LC-MS analysis.

LC-MS analysis was performed on an Agilent 6420 Triple Quadrupole LC/MS instrument (Agilent Technologies). The drying gas was maintained at a temperature of 300°C, with a flow rate of 9 L/min, nebulizer pressure of 15 psi. LC-MS analysis was performed using a ZIC-HILIC column (5 mm, 200 Å, 150 × 4.6 mm; Merck). HPLC conditions were as follows: mobile phase A: 90% 20 mM ammonium acetate and 10% acetonitrile, mobile phase B: acetonitrile; gradient elution from 90% B to 70% B over 10 minutes, and from 70% B to 50% B over 20 minutes. The flow rate was set to 0.5 mL/min. The mass spectrometer was operated in ESI positive ion mode.

### LC-MS analysis for Gafr, Mafr, and FDH activity assays

For Gafr activity assay, a 200 µL reaction mixture containing 50 mM PBS buffer (pH 7.2), 100 mM KCl, 10 mM maltose/10 mg/mL soluble starch, 5 µM α-1,4-glucan lyase, 5 µM Gafr, 1 mM NADPH, 5 µM FDH, and 20 mM sodium formate was incubated at RT for 2 hours. No substrate (maltose/soluble starch), no α-1,4-glucan lyase, or no FDH was contained in negative controls. The product standard used was 1,5-anhydroglucitol (5 mM). Proteins were precipitated with 100 µL acetonitrile and removed by centrifugation. The supernatant was filtered through a 0.22 µm nylon membrane filter before LC-MS analysis.

For Mafr activity assay, a 200 µL reaction mixture containing 50 mM PBS buffer (pH 7.2), 100 mM KCl, 10 mM maltose/10 mg/mL soluble starch, 5 µM α-1,4-glucan lyase, 5 µM Mafr, 1 mM NADPH, 5 µM FDH, and 20 mM sodium formate was incubated at RT for 2 hours. No substrate (maltose/soluble starch), no α-1,4-glucan lyase, or no FDH was contained in egative controls. The product standard used was 1,5-anhydromannitol (5 mM). Proteins were precipitated with 100 µL acetonitrile and removed by centrifugation. The supernatant was filtered through a 0.22 µm nylon membrane filter before LC-MS analysis. The LC-MS detection method was identical to that described for α-1,4-glucan lyase activity assay.

### LC-MS analysis for TlGlkA activity assay

A 200 µL reaction mixture containing 50 mM Tris (pH=7.5), 100 mM KCl, 2 mM MgCl2, 5 mM ADP, 20 mM 1,5-AG, and 10 µM TlGlkA was incubated at RT for 1 h. No 1,5-AG or no TlGlkA was contained in negative controls. 200 µL of acetonitrile was added to the reaction system to precipitate proteins, the precipitate was removed by centrifugation, and the supernatant was filtered through a 0.22 µm PES membrane for LC-MS analysis. The LC-MS analysis method for TlGlkA activity assay was the same as that for LpYbiW activity assay, and the results are shown in FIG. 26.

### Activity assays of α-1,4-glucan lyase, Gafr, Mafr, and FDH in M9 medium

The purified α-1,4-glucan lyase, Gafr, Mafr, and FDH protein solutions were degassed with argon to remove oxygen and placed in a glove box. 1 g of soluble starch was dissolved in 100 mL of oxygen-free M9 medium without glucose to obtain "starch M9 medium". A first 200 µL reaction mixture contained 100 µL of "starch M9 medium" and 5 µM α-1,4-glucan lyase. A second 200 µL reaction mixture contained 100 µL of "starch M9 medium", 5 µM α-1,4-glucan lyase, 5 µM Gafr, and 1 mM NADPH. A third reaction mixture was composed of the second reaction mixture plus 5 µM FDH and 20 mM sodium formate. A fourth (fifth) reaction mixture was similar to the second (third) but contained 5 µM Mafr instead of Gafr. These five reaction mixtures were incubated at RT for 2 hours before LC-MS analysis. The LC-MS determination method was exactly the same as that described for α-1,4-glucan lyase activity assay. "Starch M9 medium" was also treated in the same manner and subjected to LC-MS analysis.

### Anaerobic growth of E. coli MG1655_WT using soluble starch as the sole carbon source

A single colony of freshly grown *E. coli* MG1655 WT strain on an LB agar plate was inoculated into 5 mL of LB medium and cultured at 37°C in a shaking incubator for 4 hours. Cells from 100 µL of the culture were transferred to an anaerobic bottle containing 5 mL of oxygen-free LB medium and cultured at 37°C for 6 hours. 5 mL of cells were harvested, washed three times with oxygen-free M9 medium without glucose, and resuspended in 2 mL of oxygen-free M9 medium without glucose.

Anaerobic bottles 1 and 2 contained 5 mL of oxygen-free "starch M9 medium", bottle 3 contained 5 mL of M9 medium without glucose, bottle 4 contained 5 mL of M9 medium supplemented with 20 mg glucose. A final concentration of 0.3 µM of degassed α-1,4-glucan lyase protein solution was added to 5 mL of oxygen-free "starch M9 medium" to obtain bottle 5. Degassed α-1,4-glucan lyase, Gafr, and FDH protein solutions were added to 5 mL of oxygen-free starch M9 medium at final enzyme concentrations of 0.3 µM, 1.8 µM, and 0.6 µM, respectively. In addition, NADPH and sodium formate were added at final concentrations of 0.2 mM and 2 mM, respectively, to obtain bottle 6. Bottle 7 was prepared identically to bottle 6 except that it contained 1.8 µM MBP instead of Gafr. After incubation at RT for 4 hours in a glove box, 100 µL of the cell suspension was transferred to these anaerobic bottles. The anaerobic bottles were then placed in a 37°C incubator for 3-7 days, then photographed, and cells were collected for SDS-PAGE gel analysis, and the supernatant was extracted with ethanol for GC analysis.

### Identification of proteins by SDS/PAGE and mass spectrometry

Cells were harvested by centrifugation, lysed by boiling in Laemmli loading buffer, and analyzed on a 10% SDS/PAGE gel. Prominent protein bands induced in cells co-cultured with α-1,4-glucan lyase, Gafr, FDH, and E. coli MG1655_WT, and in cells co-cultured with α-1,4-glucan lyase, Mafr, FDH, and E. coli MG1655_WT were excised manually. After in-gel digestion and extraction, the peptide mixtures were analyzed by a Fusion Lumos mass spectrometer coupled to an Easy nLC 1200 system (Thermo Fisher Scientific). MS/MS spectra from each LC-MS/MS run were searched against the E. coli protein database (released April 1, 2021) containing 15,862 sequence entries from UniProt using an in-house Proteome Discoverer (version 2.2) search algorithm. Protein identification was performed based on Sequest HT.

### GC analysis of fermentation broth

The seven groups of anaerobic bottles were cultured at 37°C for 7 days, and cells were removed by centrifugation. 200 µL of fermentation broth was taken, 800 µL of chromatographically pure absolute ethanol was added, vortexed to mix, centrifuged to remove insoluble precipitate, and then a supernate was filtered through a 0.45 µm organic membrane. The resulting filtrate was analyzed by gas chromatography. Commercial (R)-1,2-propanediol standard was dissolved in chromatographically pure absolute ethanol.

Gas chromatography (GC) analysis was performed using an Agilent 6820 model G1176A gas chromatograph (Agilent Technologies). The GC column used for GC analysis was an AT^{™}-Aquawax-DA (Alltech) gas chromatography column (30 m × 0.53 mm, 1.0 µm). GC conditions were as follows: carrier gas was high-purity nitrogen, column flow rate was constant at 1.0 mL/min; inlet temperature 230°C, detector temperature 240°C; hydrogen flow rate 20 mL/min; air flow rate 200 mL/min. A programmed temperature ramp was used: initial column temperature 60°C, hold for 2 minutes, ramp to 80°C at 20°C/min, hold for 3 minutes, ramp to 160°C at 20°C/min, hold for 2 minutes; then ramp to 220°C at 15°C/min, hold for 10 minutes.

### Results, analysis, and discussion

The inventors discovered two novel glycine radical enzyme (GRE)-involved glycolytic pathways for producing 1,2-propanediol from 1,5-AG and from 1,5-AM. In this example, the inventors studied the complete pathway for producing 1,2-propanediol from starch via 1,5-AG or 1,5-AM. The inventors selected α-1,4-glucan lyase from *Gracilariopsis lemaneiformis* (α-1,4-glucan lyase, Uniprot accession No. Q9STC1), the gene for 1,5-anhydro-D-fructose reductase from *Sus scrofa* (Gafr, Uniprot accession No. P82125), 1,5-anhydro-D-fructose reductase from *Ensifer adhaerens* (Mafr, Uniport accession No. Q2I8V6), and formate dehydrogenase (FDH) from *Pseudomonas* 101. These genes were codon-optimized for *E. coli* and synthesized. Soluble proteins were obtained by heterologous expression using the *E. coli* system (FIG. 20).

Through in vitro enzyme activity experiments, the inventors determined the activity of the purified proteins. By LC-MS analysis, using soluble starch and maltose as substrates, α-1,4-glucan lyase catalyzed the production of 1,5-anhydrofructose (1,5-AF) (Fig. 21A, Fig. 21B; Fig. 22A, Fig. 22B); adding Gafr and NADPH to this reaction system resulted in the production of 1,5-anhydroglucitol (1,5-AG); further adding FDH and sodium formate significantly increased the yield of 1,5-AG (Fig. 21C, Fig. 21D; Fig. 22C). These experiments demonstrated the in vitro activity of these purified enzymes, and through catalysis by these enzymes, the production of 1,5-AG from starch could be achieved.

Using soluble starch and maltose as substrates, adding Mafr and NADPH to the α-1,4-glucan lyase reaction system, the inventors found that 1,5-anhydromannitol (1,5-AM) was produced; further adding FDH and sodium formate significantly increased the yield of 1,5-AM (Fig. 21E, Fig. 21F; Fig. 22D). These experiments demonstrated the in vitro activity of these purified enzymes, and through catalysis by these enzymes, the production of 1,5-AM from starch could be achieved.

The inventors aimed to achieve the production of 1,2-propanediol from starch through co-culture of in vitro enzymes and bacteria. Using M9 medium with soluble starch as the sole carbon source, wild-type *E. coli* MG1655 was cultured anaerobically with the addition of different enzymes. First, the activity of the enzymes in the medium was tested. When α-1,4-glucan lyase, Gafr, and NADPH were added to the "starch M9 medium", the production of 1,5-AG was detected. When FDH and sodium formate were also added to the medium, the yield of 1,5-AG in the medium was further increased (Fig. 23A, Fig. 23C). When α-1,4-glucan lyase, Mafr, and NADPH were added to the "starch M9 medium", the production of 1,5-AM was detected. When FDH and sodium formate were further added to the medium, the yield of 1,5-AM in the medium was further increased (Fig. 23B, Fig. 23D).

Through co-culture experiments of enzymes and cells, it was observed that *E. coli* MG1655 could not use starch as the sole carbon source for growth; when α-1,4-glucan lyase was added to the "starch M9 medium", no significant growth of *E. coli* MG1655 was observed, indicating that it could not utilize 1,5-AF for growth. However, when α-1,4-glucan lyase, Gafr, FDH, NADPH, and sodium formate were added to the "starch M9 medium", *E. coli* MG1655 showed significant growth; when α-1,4-glucan lyase, MBP-Mafr, FDH, NADPH, and sodium formate were added to the "starch M9 medium", *E. coli* MG1655 also showed significant growth (Fig. 24A).

Cells from different experimental groups were collected and analyzed by SDS-PAGE. The inventors observed that when α-1,4-glucan lyase, Gafr, FDH, NADPH, and sodium formate were added to the medium, *E. coli* MG1655 cells were induced to produce bands of ~95 kDa, ~42 kDa, and ~27 kDa, which were consistent with the results when 1,5-AG was used as the sole carbon source. When α-1,4-glucan lyase, MBP-Mafr, FDH, NADPH, and sodium formate were added to the M9 medium, *E. coli* cells were induced to produce bands of ~42 kDa and ~27 kDa, which were consistent with the results when 1,5-AM was used as the sole carbon source; however, due to the presence of a large amount of MBP-Mafr in the medium, a distinct ~95 kDa band was not observed (Fig. 24B).

By GC analysis of the fermentation broth, the inventors found that co-culture of α-1,4-glucan lyase, Gafr, FDH with cells could produce 1,2-propanediol; similarly, co-culture of α-1,4-glucan lyase, MBP-Mafr, and FDH with cells could also produce 1,2-propanediol (Fig. 24C). The genome of *E. coli* MG1655 contained the YbiW and PflD gene clusters (Fig. 25A). Based on the growth results of *E. coli* MG1655 strain in "starch M9 medium" co-cultured with different enzymes, combined with SDS-PAGE analysis and GC results of the fermentation broth, the inventors found that through the in vitro conversion by α-1,4-glucan lyase, Gafr, and FDH, E. coli MG1655 could produce 1,2-propanediol from starch via 1,5-AG (Fig. 25B). Similarly, through the in vitro conversion by α-1,4-glucan lyase, MBP-Mafr, and FDH, E. coli MG1655 could also produce 1,2-propanediol from starch via 1,5-AM (Fig. 25C).

**Table 1. Data collection and refinement statistics for EcYbiW and SdPflD crystals**

| | **EcYbiW** | **SdPflD** |
|---|---|---|
| Data collection | | |
| Space group | F222 | P 62 |

| **Cell size (Å)** | | |
|---|---|---|
| a, b, c (Å) | 116.48Å 207.17Å 208.05Å | 183.91Å 183.91Å 60.02Å |
| α, β, γ (°) | 90.00, 90.00, 90.00 | 90.00, 90.00, 120.00 |
| Resolution (Å) | 46.48 - 2.65 (2.745 - 2.65) | 53.09 - 2.34 (2.424 - 2.34) |
| Rmerge | 0.19 | 0.20 |
| I/σ (I) | 1.55 | 5.34 |
| Completeness (%) | 97.44 (92.74) | 98.79(96.34) |

| **Refinement** | | |
|---|---|---|
| Resolution (Å) | 46.48 - 2.65 (2.745 - 2.65) | 53.09 - 2.34 (2.424 - 2.34) |
| Reflection number | 35659 (3322) | 48712 (4715) |
| Rwork/Rfree | 0.223/0.284 | 0.157/0.193 |

| Atomic number | | |
|---|---|---|
| Protein | 6264 | 6111 |
| Ligand/Ion | 15 | 15 |
| Water | 165 | 564 |

| **Ramachandran plot (%)** | | |
|---|---|---|
| Optimal | 89.21 | 96.62 |
| Additionally permitted | 9.18 | 3.12 |

| **R.M.S. Deviation** | | |
|---|---|---|
| RMSD Length (Å) | 0.009 | 0.007 |
| RMSD Angle (°) | 1.28 | 1.18 |

| | | |
|---|---|---|
| Note: The statistics shown in the parentheses represent the highest resolution enclosure information. | | |

**Table 2. Accession numbers, strain sources, and SEQ ID NOs for 1,5-anhydroglucitol-6-phosphate isomerase isozymes**

| **Accession number** | **Strain source** | **SEQ ID NO:** |
|---|---|---|
| A0A807DR53 | *Lactiplantibacillus plantarum* | 2 |
| A0A0R2FVZ1 | *Lactobacillus selangorensis* | 3 |
| A0A0A6S4M4 | *Streptococcus uberis* | 4 |
| A0A0K1F3T2 | *Olsenella* sp. oral taxon 807 | 5 |
| A0A134AK54 | *Leptotrichia wadei* | 6 |
| A0A134A1S1 | *Senella* sp. DNF00959 | 7 |
| A0A0E2UQR7 | *Streptococcus parauberis* | 8 |
| A0A173RRH3 | *Anaerostipes hadrus* | 9 |
| A0A1E8VV95 | *Senella* sp. HMSC062G07 | 10 |
| A0A1I3HGA4 | *Selenomonas ruminantium* | 11 |
| A0A285HLM7 | *Orenia metallireducens* | 12 |
| A0A2M9H9C8 | *Bifidobacterium primatium* | 13 |
| C9N0T4 | *Leptotrichia hofstadii*F0254 | 14 |
| A0A0R2AF79 | *Ligilactobacillus agilis*DSM 20509 | 15 |
| U2TM27 | *Senella profusa*F0195 | 16 |
| A0A0A6PW80 | *Clostridium butyricum* | 17 |
| A0A1C0A9L3 | *Orenia metallireducens* | 18 |
| A0A0B8PMP8 | *Vibrio ishigakensis* | 19 |
| A0A425WQY7 | *Coriobacteriaceae* | 20 |
| F2N714 | *Coriobacterium glomerans* strain ATCC 49209/DSM 20642/JCM 10262/PW2 | 21 |
| M2NEK5 | *Eggertshia catenaformis*OT 569 | 22 |
| A0A0A7FYZ2 | *Clostridium baratiistr. Sullivan* | 23 |
| A0A143X8V0 | *Clostridiales* bacteria CHKCI006 | 24 |
| A0A2N2BBI5 | *Firmicutes* bacteria HGW-Firmicutes-5 | 25 |
| A0A2T0BJ14 | *Clostridium vincintii* | 26 |
| A0A380JDF6 | *Streptococcus downei* MFe28 | 27 |
| A0A847CI73 | *Erysipelotrichaceae* | 28 |
| A0A8J6Z417 | Quinella sp.3Q1 | 29 |
| D1AFQ7 | *Sebaldella termitidis* strain ATCC 33386/NCTC 11300 | 30 |
| A0A062WZ81 | *Ligilactobacillus animalis* | 31 |
| A0A0R1V613 | *Liquorilactobacillus satsumensis* DSM 16230 | 32 |
| A0A0M0A869 | *Clostridium botulinum* | 33 |
| A0A0V8QAB4 | *Acetivibrio ethanolgignens* | 34 |
| A0A2N3J357 | *Aeromonas sobria* | 35 |
| A0A1I2MD83 | *Clostridium cadaveris* | 36 |
| A0A1L8MKJ9 | *Streptococcus bovimastitidis* | 37 |
| A0A4R1NC63 | *Sodalis ligni* | 38 |
| A0A5R9CBU2 | Lactococcus raffinolactis | 39 |
| C2ELS4 | *Lactobacillus ultunensis* DSM 16047 | 40 |
| A0A3P1W4E8 | *Leptotrichia sp.* OH3620 | 41 |
| A0A498R759 | *Lucifera butyrica* | 42 |
| A0A4V6Q2Z2 | *Fonticella tunisiensis* | 43 |
| A7MME9 | *Cronobacter sakazakii* strain ATCC BAA-894 | 44 |
| A0A0R1Q920 | *Liquorilactobacillus uvarum* DSM 19971 | 45 |
| A0A1Y4F189 | *Anaeromassilibacillus sp.* An250 | 46 |
| A0A498D0T9 | *Anaerotruncus sp.* 22A2-44 | 47 |
| A0A510JEA5 | *Leptotrichia hofstadii* | 48 |
| A0A0A2VZ96 | *Beauveria bassiana* D1-5 | 49 |
| A0A447V187 | *Cedecea lapagei* | 50 |
| B2ISH7 | *Streptococcus pneumoniae* strain CGSP14 | 51 |
| A0A075SG00 | *Streptococcus suis* 6407 | 52 |
| A0A0R2GM47 | *Lactiplantibacillus plantarum* | 53 |
| A0A1Q8F4Y7 | *Aeromonas veronii* | 54 |
| A0A290PWH6 | *Lactococcus raffinolactis* | 55 |
| A0A447Y009 | *Escherichia coli* | 56 |
| A0A0B7GIL0 | *Streptococcus sanguinis* | 57 |
| A0A1L7RNK7 | *Actinomyces succiniciruminis* | 58 |
| A0A376TJI4 | *Bacillus coli* | 59 |
| A0A3R9YDI0 | *Vagococcus humatus* | 60 |
| A0A064C019 | *Streptococcus pneumoniae* | 61 |
| A0A0P7FGJ3 | *Vibrio alginolyticus* | 62 |
| A0A0R2KGV0 | *Ligilactobacillus acidipiscis* | 63 |
| A0A1T4P4Y5 | *Pilibacter termitis* | 64 |
| A0A1E3KU28 | *Lactiplantibacillus plantarum* | 65 |
| A0A349BXL4 | *Lachnospiraceae* | 66 |
| A0A3T0ZV83 | *Aeromonas hydrophila* | 67 |
| A0A1L8WPN6 | *Enterococcus ratti* | 68 |
| A0A0R2H5D7 | *Kandleria vitulina* | 69 |
| A0A0H3J4P2 | *Clostridium pasteurianum* DSM 525 | 70 |
| A0A0T6U3W3 | *Aeromonas allosaccharophila* | 71 |
| K8CC45 | *Enterobacter sakazakii* | 72 |
| A0A1W6B1J9 | *Pantoea alhagi* | 73 |
| B6FW14 | *Peptacetobacter hiranonis* strain DSM 13275/JCM 10541/KCTC 15199/TO-931 | 74 |
| A0A034T1M9 | *Edwardsiella piscicida* | 75 |
| A0A089PXZ6 | *Cedecea neteri* | 76 |
| A0A0A3AQL5 | *Chelonobacter oris* | 77 |
| A0A0D0QVG1 | *Aeromonas sp.* L_1B5_3 | 78 |
| A0A0F4VRP2 | *Clostridium sp.* IBUN125C | 79 |
| A0A1M5EXF5 | *Vibrio gazogenes* DSM 21264 | 80 |
| A0A2Z5Y0I5 | *Melissococcus plutonius* | 81 |
| A0A5C7QZI1 | *Tolumonas sp.* | 82 |
| A0A3D0GQ55 | *Erysipelotrichaceae* | 83 |
| A0A0R1F6B2 | *Loigolactobacillus coryniformissub sp. Coryniformis* KCTC 3167 | 84 |
| A0A4P9VTG8 | *Zooshikella ganghwensis* | 85 |
| A0A829T2C0 | *Vibrio sp.* | 86 |
| A0A2T3N2G3 | *Photobacterium lipolyticum* | 87 |
| A0A8S7XWX0 | *Bacillus coli* | 88 |
| X0PS46 | *Agrilactobacillus composti* DSM 18527 | 89 |
| A0A061Q2Y5 | *Vibrio sp.* JCM 19052 | 90 |
| A0A174H662 | *Clostridium symbiosum* | 91 |
| F3Y874 | *Melissococcus plutonius* strain ATCC 35311/CIP 104052/LMG 20360/NCIMB 702443 | 92 |
| A0A1S1BRH2 | *Aerococcus sp.* HMSC035B07 | 93 |
| A0A1Y4BCL8 | *Senella sp.* An293 | 94 |
| A0A7Z0VH52 | -- | 95 |
| A0A0I9W1Z4 | -- | 96 |

**Table 3. Primers for plasmid construction.**

| **SEQ ID NO:** | **Name** | **Target** | **Sequence (5' to 3')** | **Application** |
|---|---|---|---|---|
| 154 | 1F | genome DNA* | cctgtacttccaatccatgacgaatcgtatctc | Construction of HT-EcPflD |
| 155 | 1R | genome DNA* | cgttatccacttccttacagctgatgcgtctg | Construction of HT-EcPflD |
| 156 | 2F | genome DNA* | cctgtacttccaatccatgacatcatccgcc | Construction of HMT-EcPflC |
| 157 | 2R | genome DNA* | ccgttatccacttccttaacctcccacggtaac | Construction of HMT-EcPflC |
| 158 | 3F | genome DNA* | cctgtacttccaatccatggaactgtatctgg | Construction of HT-EcFsaB |
| 159 | 3R | genome DNA* | ccgttatccacttccaatttagagatgagtagtgcc | Construction of HT-EcFsaB |
| 160 | 4F | genome DNA* | cctgtacttccaatccatggaccgcattattc | Construction of HT-EcGldA |
| 161 | 4R | genome DNA* | ccgttatccacttccaatttattcccactctgc | Construction of HT-EcGldA |
| 162 | 5F | genome DNA* | ccagagcggatcaggaatgaccacactgaa | Construction of pACYC-EcYbiW |
| 163 | 5R | genome DNA* | gagaatctgcccatatgttacagcatatgttc | Construction of pACYC-EcYbiW |
| 164 | 6F | pACYC-EcYbiW | ggctttgcggtgagcgcagcgaacgcacgcgtctgcacatg | Construction of pACYC-EcYbiW(E114A , E115A and K117A) |
| 165 | 6R | pACYC-EcYbiW | cgctcaccgcaaagccagcacccgggacg | Construction of pACYC-EcYbiW(E114A , E115A and K117A) |

| | | | | |
|---|---|---|---|---|
| Note: * represents the genome of *E coli* MG165. | | | | |

**Table 4. Accession numbers, strain sources, and SEQ ID NOs of 47 1,5-anhydromannitol-6-phosphate isomerase isozymes**

| **Accession number** | **Strain source** | **SEQ ID NO:** |
|---|---|---|
| P32674 | *Bacillus coli* | 98 |
| A0A7C9H654 | *Firmicutes* | 99 |
| A0A510J954 | *Pseudoleptotrichia goodfellowii* | 100 |
| D4F6M7 | *Edwardsiella tarda* | 101 |
| D4E0C4 | *Serratia odorifera* DSM 4582 | 102 |
| A0A2V4E297 | *Gilliamella apicola* | 103 |
| A0A084T2T3 | *Vibrio* ER1A | 104 |
| A0A0R2FPV6 | *Lactobacillus selangorensis* | 105 |
| A0A0N8NM72 | *Caloranaerobacter sp.* | 106 |
| A0A1T4K0K6 | *Pilibacter termitis* | 107 |
| A0A096BHK0 | *Caloranaerobacter azorensis* H53214 | 108 |
| A0A725B0Z8 | *Salmonella enteritidis* | 109 |
| A0A0B8Q7B9 | *Vibrio* JCM 19236 | 110 |
| A0A037YM28 | *Bacillus coli* | 111 |
| C4LFY8 | *Tolumonas auensis* strain DSM 9187/TA4 | 112 |
| A0A6N7XPT4 | *Senella porci* | 113 |
| A0A4S2F5T8 | *Mycobacteriaceae* | 114 |
| A0A4R7KVA0 | *Fonticella tunisiensis* | 115 |
| A0A437UWZ5 | *Coriobacterialesbacteria* OH1046 | 116 |
| A0A3R9P0U8 | *Bacillus sp*. HMF5848 | 117 |
| A0A380TM32 | *Actinobacillus rossii* | 118 |
| C2ELS8 | *Lactobacillus ultunensis* DSM 16047 | 119 |
| A0A2N2B0Z5 | *Firmicutes bacteria* HGW-Firmicutes-7 | 120 |
| A0A1J0GF99 | *Clostridium estertheticum Esterification subspecies* | 121 |
| A0A1D8GPW2 | *Geosporobacter ferrireducens* | 122 |
| A0A0B8PE23 | *Vibrio ishigakensis* | 123 |
| A0A239SQG5 | *Streptococcus merionis* | 124 |
| A0A823B0Q1 | *Shigella boydii* | 125 |
| X0PU04 | *AgriLactobacillus composti* DSM 18527 | 126 |
| A0A0R2FH98 | *Lactobacillus selangorensis* | 127 |
| A0A430AQ99 | *Vagococcus elongatus* | 128 |
| A0A1W1WZ75 | *Clostridium acidisoli* DSM 12555 | 129 |
| A0A376L3P4 | *Bacillus coli* | 130 |
| A0A1T4WMT8 | *Caloramator quimbayensis* | 131 |
| A0A11H9K1 | *Clostridium uliginosum* | 132 |
| A0A143I1W1 | *Enterobacter asburiae* | 133 |
| N2BYX9 | *Atopobium minutum* | 134 |
| A0A4S2EVE2 | *Coriobacteriaceae* | 135 |
| A0A7S7M803 | *Thermophilibacter immobilis* | 136 |
| A0A2T3N2G1 | *Lactobacillus solubilis* | 137 |
| A0A381GG45 | *Citrobacter amalonaticus* | 138 |
| A0A0A0F8F5 | *Bacillus coli* G3 | 139 |
| A0A0J0GCD9 | *Escherichia albertii* | 140 |
| A0A0H3J2B1 | *Pasteurella pestis bacillus* | 141 |
| D1AJA5 | *Termite Saccharomonospora* strain ATCC 33386/NCTC 11300 | 142 |
| A0A8S0W6S0 | *Acididesulfobacillus acetoxydans* | 143 |
| A0A7W2G1V0 | *clostridium* | 144 |

**Table 5. Primers for construction of E. coli ΔybiW and ΔpflD strains**

| **SEQ ID NO:** | **name** | **Target** | **Sequence (5' to 3')** | **Application** |
|---|---|---|---|---|
| 166 | 7F | genome DNA† | ccagtagaaacagacgtctaggggcattttcgctgacata | Construction of pRed_Cas9_recA_ΔybiW |
| 167 | 7R | genome DNA† | ccttacgaggataacaacctctcctcggcccggcgg | Construction of pRed_Cas9_recA_ΔybiW |
| 168 | 8F | genome DNA† | ggtgttatcctcgtaaggtcgcggttaac | Construction of pRed_Cas9_recA_ΔybiW |
| 169 | 8R | genome DNA† | glgagaatccaagcttctagccttlatgcagccggaaatg | Construction of pRed_Cas9_recA_ΔybiW |
| 170 | 9F | pRed_Cas9_recA | ctagaagcttggattctaccaataaaaaacgc | Construction of pRed_Cas9_recA_ΔybiW and pRed_Cas9_recA_ΔpflD |
| 171 | 9R | pRed_Cas9_recA | cccgcagactttctggcaggccgctagatctgactccataac | Construction of pRed_Cas9_recA_ΔybiW |
| 172 | 10F | pRed_Cas9_recA | gcggctgccgagaaagtctgcgggtttagagctagaaatagc | Construction of pRed_Cas9_recA_ΔybiW |
| 173 | 10R | pRed_Cas9_recA | ctagacgtctgtttctactggtattggcacaaaacctg | Construction of pRed_Cas9_recA_ΔybiW and pRed_Cas9_recA_ΔpflD |
| 174 | 11F | genome DNA† | ccagtagaaacagacgtctagcggagcagtggtgcggctg | Construction of pRed_Cas9_recA_ΔpflD |
| 175 | 11R | genome DNA† | gtccgcgcgatgatgcatgaggaacctcgtgggaagc | Construction of pRed_Cas9_recA_ΔpflD |
| 176 | 12F | genome DNA† | atgacatcatccgccggacagcgcatcagctg | Construction of pRed_Cas9_recA_ΔpflD |
| 177 | 12R | genome DNA† | gagaatccaagcttctagcaatttcgccagcggtaa | Construction of pRed_Cas9_recA_ΔpflD |
| 178 | 13F | pRed_Cas9_recA | gcgaaataccagaacccgcgcgggttttagagctagaaatagcaa | Construction of pRed_Cas9_recA_ΔpflD |
| 179 | 13R | pRed_Cas9_recA | ccgcgcgggttctggtattccgctagatctgactccca | Construction of pRed_Cas9_recA_ΔpflD |
| 180 | 14F | pRed_Cas9_recA_Δ ybiW and pRed_Cas9_recA_Δ pflD | gctgtatgcacaaagcatc | Sequencing |
| 181 | 14R | pRed_Cas9_recA_Δ ybiW and pRed_Cas9_recA_Δ pflD | cgagagctcgctggacctcctg | Sequencing |
| 182 | 15F | pRed_Cas9_recA_Δ ybiW and pRed_Cas9_recA_Δ pflD | gctgctgagcaacccgaactc | Sequencing |
| 183 | 16F | genome DNA# | ccgcgaaataccgtttgccttatgtagacc | Sequencing |
| 184 | 16R | genome DNA# | ggtgtcagaacccggaaagcc | Sequencing |
| 185 | 17F | genome DNA* | gcggcactgtgatgggctgtg | Sequencing |
| 186 | 17R | genome DNA* | gccacatcagctgacgacggcgc | Sequencing |

| | | | | |
|---|---|---|---|---|
| Note: † represents the genome of *E coli* MG1655WT; # represents the genome of ΔybiW; * represents the genome of ΔPflD. | | | | |

**Table 6. Primers for plasmid construction**

| **SEQ ID NO:** | **Name** | **Target** | **Sequence (5' to 3')** | **Application** |
|---|---|---|---|---|
| 189 | 18F | genome DNA* | cctgtacttcccaatccatgaccacactgaaactg | Construction of HT-EcYbiW |
| 190 | 18R | genome DNA* | gatccgttatccacttccttacagcatatgttcag | Construction of HT-EcYbiW |
| 191 | 19F | genome DNA* | cctgtattttcagggcgcccatatgccaacgacatcacaac | Construction of pACYC-MBP-EcYbiY |
| 192 | 19R | genome DNA* | ccaattgagatctgcccatatgttaccacccattcacccg | Construction of pACYC-MBP-EcYbiY |

| | | | | |
|---|---|---|---|---|
| Note: * represents the genome of *E coli* MG165 | | | | |

**Table 7: Primers used for plasmid construction**

| **SEQ ID NO:** | **primer** | **Target** | **Sequence (5' to 3')** | **Application** |
|---|---|---|---|---|
| 193 | 20F | HT-1,4-glucan lyase | caccaccaccaccaccactgagatcc | Construction of 1,4-glucan lyase-His₆ |
| 194 | 20R | HT-1,4-glucan lyase | ggtatatctccttcttaagttaaacaaaattattt | Construction of 1,4-glucan lyase-His₆ |
| 195 | 21F | HT-1,4-glucan lyase | aagaaggagatataccatgtttagcaccctggcc | Construction of 1,4-glucan lyase-His₆ |
| 196 | 21R | HT-1,4-glucan lyase | ctcagtggtggtggtggtggtggggtaatggtaataactgc | Construction of 1,4-glucan lyase-His₆ |
| 197 | 22F | HMT-Mafr | cctgtacttccaatccatgaaccgttggggtc | Construction of HMT-Mafr |
| 198 | 22R | HMT-Mafr | cggatccgttatccacttccttacagacctgtttc | Construction of HMT-Mafr |

The exemplary examples of the various embodiments of the present disclosure are described above. However, without departing from the spirit and scope of the present disclosure, those skilled in the art may modify or improve the exemplary examples described herein. Variations or equivalent solutions resulting therefrom also fall within the scope of the present disclosure.

## Claims

1. A method for producing 1,2-propanediol, comprising:
a) reacting a mixture I comprising an α-1,4-glucan lyase, a 1,5-anhydro-D-fructose reductase, and reduced nicotinamide adenine dinucleotide (NADH) or reduced nicotinamide adenine dinucleotide phosphate (NADPH) with a substrate containing α-1,4-glycosidic bonds; or reacting a culture broth of cells capable of expressing and secreting an α-1,4-glucan lyase and/or a 1,5-anhydro-D-fructose reductase with NADH or NADPH and a substrate containing α-1,4-glycosidic bonds;
b) reacting a reaction product obtained in step a) with a mixture II comprising a sugar kinase, a 1,5-anhydroalditol-6-phosphate isomerase, a radical enzyme activator, an aldolase, and a hydroxyacetone reductase, or co-culturing the reaction product obtained in step a) with cells capable of expressing a 1,5-anhydroalditol-6-phosphate isomerase, a radical enzyme activator, an aldolase, a hydroxyacetone reductase, and a transport complex, to obtain 1,2-propanediol, wherein the transport complex is preferably a phosphoenolpyruvate-phosphotransferase system.

2. A method for producing 1,2-propanediol, comprising:
reacting 1,5-anhydroalditol with a mixture II comprising a sugar kinase, a 1,5-anhydroalditol-6-phosphate isomerase, a radical enzyme activator, an aldolase, and a hydroxyacetone reductase, or co-culturing 1,5-anhydroalditol with cells capable of expressing a 1,5-anhydroalditol-6-phosphate isomerase, a radical enzyme activator, an aldolase, a hydroxyacetone reductase, and a transport complex, to obtain 1,2-propanediol, preferably the 1,5-anhydroalditol is 1,5-anhydroglucitol or 1,5-anhydromannitol.

3. The method of claim 1, wherein the mixture I further comprises other enzymes that regenerate NAD(P)⁺ to NAD(P)H, such as formate dehydrogenase (FDH); the cells capable of expressing and secreting the α-1,4-glucan lyase and/or the 1,5-anhydro-D-fructose reductase are also capable of expressing other enzymes that regenerate NAD(P)⁺ to NAD(P)H, such as FDH, and secreting expressed enzymes into an extracellular space.

4. The method of claim 1 or 3, wherein the 1,5-anhydro-D-fructose reductase is a 1,5-anhydro-D-fructose reductase from *Sus scrofa* (Gafr) or a 1,5-anhydro-D-fructose reductase from *Ensifer adhaerens* (Mafr).

5. The method of claim 1 or 3, wherein the α-1,4-glucan lyase is derived from *Gracilariopsis lemaneiformis*, and/or the FDH is derived from *Pseudomonas sp*..

6. The method of claim 1 or 3, wherein the reaction product obtained in step a) is 1,5-anhydroalditol; preferably, the 1,5-anhydroalditol is 1,5-anhydroglucitol or 1,5-anhydromannitol.

7. The method of claim 1 or 3, wherein the substrate containing α-1,4-glycosidic bonds is selected from starch, maltose, or glycogen; preferably, the reaction in step a) is carried out in a solution containing starch, for example, a culture medium.

8. The method of claim 1 or 2, wherein the cell is a eukaryotic cell or a prokaryotic cell;
preferably, the eukaryotic cell is a yeast cell; and/or
preferably, the prokaryotic cell is selected from: *Escherichia spp., Klebsiella spp., Streptococcus spp., Lactobacillus spp., Bifidobacterium spp., Bacteroidetes*, and *Firmicutes*.

9. The method of claim 8, wherein the cell is an *E. coli* cell, preferably an *E. coli* cell that is induced to highly express YbiW and/or PflD gene clusters by 1,5-AG and/or 1,5-AM, or is genetically modified to highly express YbiW and/or PflD gene clusters under a strong promoter.

10. The method of claim 1 or 2, wherein the 1,5-anhydroalditol-6-phosphate isomerase comprises an amino acid sequence shown in SEQ ID NO:1 or 97 or a functional variant thereof, wherein the functional variant possesses 1,5-anhydroalditol-6-phosphate isomerase activity.

11. The method of claim 10, wherein the 1,5-anhydroalditol-6-phosphate isomerase comprises the amino acid sequence shown in SEQ ID NO:1 or a functional variant thereof;
preferably, the 1,5-anhydroalditol-6-phosphate isomerase has an active site in its three-dimensional conformation defined such that, with reference to SEQ ID NO: 1, the active site includes amino acid residues H165, H282, S283, H334, C441, E443, R453, T455, L562, S662, I664, and G786, which are spatially close to one another.

12. The method of claim 10, wherein the 1,5-anhydroalditol-6-phosphate isomerase comprises an amino acid sequence shown in SEQ ID NO:97 or a functional variant thereof;
preferably, the 1,5-anhydroalditol-6-phosphate isomerase has an active site in its three-dimensional conformation defined such that, with reference to SEQ ID NO: 97, the active site includes amino acid residues Q162, H169, S277, S278, R323, F331, P335, C431, E433, D445, Y628, V630, and G752, which are spatially close to one another.

13. The method of claim 1 or 2, wherein the sugar kinase or the transport complex phosphorylates the reaction product obtained in step a), preferably phosphorylates 1,5-anhydroalditol to 1,5-anhydroalditol-6-phosphate.

14. The method of claim 13, wherein the transport complex comprises at least one of SEQ ID NOs:150-153 or a functional variant thereof, and the transport complex possesses phosphoenolpyruvate-dependent phosphotransferase system transport activity.

15. The method of claim 1 or 2, wherein the radical enzyme activator is an S-adenosylmethionine radical enzyme family glycine radical enzyme activating enzyme, and/or the aldolase is 1-deoxyfructose-6-phosphate aldolase.

16. Use of a composition comprising an α-1,4-glucan lyase, a 1,5-anhydro-D-fructose reductase, and reduced nicotinamide adenine dinucleotide (NADH) or reduced nicotinamide adenine dinucleotide phosphate (NADPH) in catalyzing the formation of 1,5-anhydroalditol from a substrate containing α-1,4-glycosidic bonds, preferably the composition further comprises other enzymes that regenerate NAD(P)⁺ to NAD(P)H, such as formate dehydrogenase (FDH), and preferably the substrate containing α-1,4-glycosidic bonds is selected from starch, maltose, or glycogen.

17. Use of a composition comprising a sugar kinase, a 1,5-anhydroalditol-6-phosphate isomerase, a radical enzyme activator, an aldolase, and a hydroxyacetone reductase in catalyzing the formation of 1,2-propanediol from 1,5-anhydroalditol.

18. The use of claim 16 or 17, wherein the 1,5-anhydroalditol is 1,5-anhydroglucitol or 1,5-anhydromannitol.
